(19) Europäisches Patentamt / European Patent Office / Office européen des brevets

(11) **EP 3 817 763 B1**

(12) **EUROPEAN PATENT SPECIFICATION**

(45) Date of publication and mention of the grant of the patent:
**03.09.2025 Bulletin 2025/36**

(21) Application number: **19831325.6**

(22) Date of filing: **02.07.2019**

(51) International Patent Classification (IPC):
*A61K 38/16* (2006.01)   *A61K 38/17* (2006.01)
*A61K 35/17* (2025.01)   *C12N 5/0783* (2010.01)
*C12N 5/0786* (2010.01)   *C12N 5/078* (2010.01)
*A61P 35/02* (2006.01)   *A61P 35/04* (2006.01)
*A61K 38/00* (2006.01)   *C07K 14/705* (2006.01)
*C07K 14/725* (2006.01)

(52) Cooperative Patent Classification (CPC):
**C07K 14/705; A61K 40/11; A61K 40/31;
A61K 40/4261; A61P 35/02; A61P 35/04;
C07K 14/7051; C07K 14/70517; C07K 14/70521;
C07K 14/70578; C12N 5/0636; A61K 38/00;
A61K 2239/31; A61K 2239/38; A61K 2239/53;**
(Cont.)

(86) International application number:
**PCT/US2019/040346**

(87) International publication number:
**WO 2020/010110 (09.01.2020 Gazette 2020/02)**

(54) **CHIMERIC RECEPTORS IN COMBINATION WITH TRANS METABOLISM MOLECULES ENHANCING GLUCOSE IMPORT AND THERAPEUTIC USES THEREOF**

CHIMÄRE REZEPTOREN IN KOMBINATION MIT TRANSMETABOLISMUSMOLEKÜLEN ZUR VERSTÄRKUNG DES GLUKOSEIMPORTS UND THERAPEUTISCHE VERWENDUNGEN DAVON

RÉCEPTEURS CHIMÉRIQUES EN ASSOCIATION AVEC DES MOLÉCULES DE MÉTABOLISME TRANS AMÉLIORANT L'IMPORTATION DE GLUCOSE ET LEURS UTILISATIONS THÉRAPEUTIQUES

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **03.07.2018   US 201862693677 P
03.07.2018   US 201862693668 P
01.08.2018   US 201862713369 P
07.11.2018   US 201862756664 P
07.11.2018   US 201862756698 P**

(43) Date of publication of application:
**12.05.2021 Bulletin 2021/19**

(73) Proprietor: **SOTIO Biotech Inc.
Boston MA 02114 (US)**

(72) Inventors:
• **MCGINNESS, Kathleen**
**Boston, MA 02114 (US)**
• **ETTENBERG, Seth**
**Boston, MA 02114 (US)**
• **BARRON, Luke**
**Boston, MA 02114 (US)**
• **FRAY, Michael**
**Boston, MA 02114 (US)**
• **WILSON, Charles**
**Boston, MA 02114 (US)**
• **MOTZ, Gregory**
**Boston, MA 02114 (US)**

(74) Representative: **Maiwald GmbH
Elisenhof
Elisenstraße 3
80335 München (DE)**

**(Cont. next page)**

(56) References cited:
WO-A2-2017/180989

• MELITA IRVING ET AL: "Engineering Chimeric Antigen Receptor T-Cells for Racing in Solid Tumors: Don&#8217;t Forget the Fuel", FRONTIERS IN IMMUNOLOGY, vol. 8, 3 April 2017 (2017-04-03), Lausanne, CH, XP055513766, ISSN: 1664-3224, DOI: 10.3389/fimmu.2017.00267

• KAWALEKAR OMKAR U ET AL: "Distinct Signaling of Coreceptors Regulates Specific Metabolism Pathways and Impacts Memory Development in CAR T Cells", IMMUNITY, CELL PRESS, AMSTERDAM, NL, vol. 44, no. 2, 16 February 2016 (2016-02-16), pages 380 - 390, XP029428484, ISSN: 1074-7613, DOI: 10.1016/J.IMMUNI.2016.01.021

• CLOVIS S. PALMER ET AL: "Glucose Metabolism Regulates T Cell Activation, Differentiation, and Functions", FRONTIERS IN IMMUNOLOGY, vol. 6, 22 January 2015 (2015-01-22), XP055559629, DOI: 10.3389/fimmu.2015.00001

• CRETENET GASPARD ET AL: "Cell surface Glut1 levels distinguish human CD4 and CD8 T lymphocyte subsets with distinct effector functions", SCIENTIFIC REPORTS, vol. 6, no. 1, 12 April 2016 (2016-04-12), XP055788666, Retrieved from the Internet <URL:http://www.nature.com/articles/srep24129.pdf> DOI: 10.1038/srep24129

• KISHTON RIGEL J ET AL: "Metabolic Regulation of T Cell Longevity and Function in Tumor Immunotherapy", CELL METABOLISM, CELL PRESS, UNITED STATES, vol. 26, no. 1, 5 July 2017 (2017-07-05), pages 94 - 109, XP085115795, ISSN: 1550-4131, DOI: 10.1016/J.CMET.2017.06.016

• FREEMERMAN, A ET AL.: "Glucose Transporter 1 (GLUT1 )-Mediated Glucose Metabolism Drives A Proinflammatory Phenotype", THE JOURNAL OF BIOLOGICAL CHEMISTRY, vol. 289, no. 11, 14 March 2014 (2014-03-14), pages 7884 - 7896, XP055544688, DOI: 10.1074/jbc.M113.522037

• MACINTYRE, A ET AL.: "The Glucose Transporter Glutl Is Selectively Essential For CD 4 T Cell Activation And Effector Function", CELL METABOLISM, vol. 20, no. 1, 1 July 2014 (2014-07-01), pages 61 - 72, XP055673185

• JACOBS, S ET AL.: "Glucose Uptake Is Limiting In T Cell Activation And Requires CD 28-Mediated AKT-Depending And Independent Pathways", THE JOURNAL OF IMMUNOLOGY, vol. 180, no. 7, 1 April 2008 (2008-04-01), pages 4476 - 4486, XP055349349, DOI: 10.4049/jimmunol.180.7.4476

(52) Cooperative Patent Classification (CPC): (Cont.)
C07K 2319/00; C07K 2319/02; C07K 2319/03; C07K 2319/30; C07K 2319/33; C12N 2510/00

**Description**

BACKGROUND OF DISCLOSURE

Cancer immunotherapy, including cell-based therapy, is used to provoke immune responses attacking tumor cells while sparing normal tissues. It is a promising option for treating various types of cancer because of its potential to evade genetic and cellular mechanisms of drug resistance, and to target tumor cells while sparing normal tissues.

Cell-based therapy may involve cytotoxic T cells having reactivity skewed toward cancer cells. Eshhar et al., Proc. Natl. Acad. Sci. U. S. A.; 1993; 90(2):720-724; Geiger et al., J Immunol. 1999; 162(10):5931-5939; Brentjens et al., Nat. Med. 2003; 9(3):279-286; Cooper et al., Blood. 2003; 101(4):1637-1644; and Imai et al., Leukemia. 2004; 18:676-684. One approach is to express a chimeric antigen receptor (CAR) having an antigen-binding domain fused to one or more T cell activation signaling domains. Binding of a cancer antigen via the antigen-binding domain results in T cell activation and triggers cytotoxicity. Efficacy of chimeric antigen receptor-expressing autologous T lymphocytes in treating B-cell precursor acute lymphoblastic leukemia (ALL) has been demonstrated in clinical trials. Pule et al., Nat. Med. 2008;14(11):1264-1270; Porter et al., N Engl J Med; 2011; 25;365(8):725-733; Brentjens et al., Blood. 2011;118(18):4817-4828; Till et al., Blood. 2012;119(17):3940-3950; Kochenderfer et al., Blood. 2012;119(12):2709-2720; and Brentjens et al., Sci Transl Med. 2013;5(177):177ra138.

Irving et al. report engineering Chimeric Antigen Receptor T-cells for solid tumors (Frontiers in Immunology 8(3), 2017). Kawalekar et al. show that the choice of signaling domain can metabolically reprogram T cells to alter mitochondrial biogenesis and persistence (Immunity 44(2):380-390, 2016). Palmer et al. report that glucose metabolism regulates T cell activation, differentiation and functions (Frontiers in Immunology 6(22), 2015).

Cretenet et al. report that cell surface Glut1 levels distinguish human CD4 and CD8 T lymphocyte subsets with distinct effector functions (Scientific Reports 6(1), 2016).

Kishton et al. report metabolic regulation of T cell longevity and function in tumor immunotherapy (Cell Metabolism 26(1):94-109, 2017).

Another approach is to express an antibody-coupled T cell Receptor (ACTR) protein in an immune cell, such as an NK cell or a T cell, the ACTR protein containing an extracellular Fc-binding domain. When the ACTR-expressing T cells (also called "ACTR T cells") are administered to a subject together with an anti-cancer antibody, they may enhance toxicity against cancer cells targeted by the antibody *via* their binding to the Fc domain of the antibody. Kudo et al., Cancer Research (2014) 74:93-103.

Cell-based immune therapies, while promising, have faced challenges caused by specific characteristics of the tumor microenvironment (TME), which is cellular environment created via the interaction between malignant tumor cells and non-transformed cells. It is therefore of great importance to develop strategies to improve efficacy of cell-based immune therapies in light of the TME.

SUMMARY OF DISCLOSURE

The present disclosure is based on the development of strategies to increase glucose uptake by immune cells, including those that express a chimeric receptor polypeptide, such as an antibody-coupled T-cell receptor (ACTR) polypeptide or a chimeric antigen receptor (CAR), for use in cell-based immune therapy. Increased glucose uptake may be achieved by expressing (*e.g.,* over-expressing) in immune cells (*e.g.,* T cells or natural killer cells) one or more glucose importation polypeptides such as those described herein. Such genetically engineered immune cells are expected to have an enhanced glucose uptake, for example, in a low glucose environment (*e.g.,* in a tumor microenvironment). As such, immune cells that co-express one or more glucose importation polypeptides and a chimeric receptor polypeptide would exhibit superior bioactivities (*e.g.,* under tumor microenvironment such as low glucose conditions, optionally in the presence of a therapeutic antibody), for example, cell proliferation, activation (*e.g.,* increased cytokine production, *e.g.,* IL-2 or IFNy production), cytotoxicity, and/or *in vivo* anti-tumor activity.

Accordingly, provided herein is a genetically engineered immune cell, wherein the immune cell has an improved glucose uptake activity as relative to a wild-type immune cell of the same type, wherein the immune cell is genetically engineered to express or overly express:

> (i) a glucose importation polypeptide, and
> (ii) a chimeric receptor polypeptide; wherein the chimeric receptor polypeptide comprises:

>> (a) an extracellular target binding domain;
>> (b) a transmembrane domain; and
>> (c) a cytoplasmic signaling domain;

wherein the nucleic acid encoding the glucose importation polypeptide is located extrachromosomally or is integrated into a chromosome of the immune cell at a site that is different from the native loci of the endogenous gene, wherein the glucose importation polypeptide is a glucose transporter (GLUT) or a sodium-glucose cotransporter (SGLT), preferably wherein the glucose importation polypeptide is selected from the group consisting of: GLUT1, GLUT3, GLUT1 S226D, SGLT1, SGLT2, GLUT8, GLUT8 L12A L13A, GLUT11, GLUT7, and GLUT4.

In some embodiments, the chimeric receptor polypeptide is an antibody-coupled T cell receptor (ACTR), which comprises an extracellular Fc-binding domain (a). In other embodiments, the chimeric receptor is a chimeric antigen receptor (CAR), which comprises an extracellular antigen binding domain (a). In some examples, (c) is located at the C-terminus of the chimeric receptor polypeptide. In some instances, the chimeric polypeptide may further comprise at least one co-stimulatory signaling domain. In other instances, the chimeric receptor polypeptide may be free of co-stimulatory signaling domains.

Any of the chimeric receptor polypeptides described herein (*e.g.,* an ACTR polypeptide or a CAR polypeptide) may further comprise a hinge domain, which is located at the C-terminus of (a) and the N-terminus of (b). In other examples, the chimeric receptor polypeptide may be free of any hinge domain. When the chimeric receptor is an ACTR polypeptide, it may be free of a hinge domain from any non-CD16A receptor. Alternatively or in addition, the chimeric receptor polypeptide further comprises a signal peptide at its N-terminus.

In some embodiments, the chimeric receptor polypeptide disclosed herein may be an ACTR polypeptide comprising an Fc binding domain (a). In some examples, the Fc binding domain of (a) can be an extracellular ligand-binding domain of an Fc-receptor, for example, an extracellular ligand-binding domain of an Fc-gamma receptor, an Fc-alpha receptor, or an Fc-epsilon receptor. In particular examples, the Fc binding domain is an extracellular ligand-binding domain of CD16A (*e.g.,* F158 CD16A or V158 CD16A), CD32A, or CD64A. In other examples, the Fc binding domain of (a) can be an antibody fragment that binds the Fc portion of an immunoglobulin. For example, the antibody fragment can be a single chain variable fragment (ScFv), a single domain antibody, or a nanobody. Additionally, the Fc binding domain of (a) can be a naturally-occurring protein that binds the Fc portion of an immunoglobulin or an Fc-binding fragment thereof. For example, the Fc binding domain can be Protein A or Protein G, or an Fc-binding fragment thereof. In further examples, the Fc binding domain of (a) can be a synthetic polypeptide that binds the Fc portion of an immunoglobulin. Examples include, but are not limited to, a Kunitz peptide, a SMIP, an avimer, an affibody, a DARPin, or an anticalin.

In some embodiments, the chimeric receptor polypeptide disclosed herein can be a CAR polypeptide comprising an extracellular antigen binding domain (a). In some examples, the extracellular antigen binding domain of (a) is a single chain antibody fragment that binds to a tumor antigen, a pathogenic antigen, or an immune cell specific to an autoantigen. In certain examples, the tumor antigen is associated with a hematologic tumor. Examples include, but are not limited to, CD19, CD20, CD22, Kappa-chain, CD30, CD123, CD33, LeY, CD138, CD5, BCMA, CD7, CD40, and IL-1RAP. In certain examples, the tumor antigen is associated with a solid tumor. Examples include, but are not limited to, GD2, GPC3, FOLR (*e.g.,* FOLR1 or FOLR2), HER2, EphA2, EFGRVIII, IL13RA2, VEGFR2, ROR1, NKG2D, EpCAM, CEA, Mesothelin, MUC1, CLDN18.2, CD171, CD133, PSCA, cMET, EGFR, PSMA, FAP, CD70, MUC16, L1-CAM, B7H3, and CAIX. In certain examples, the pathogenic antigen is a bacterial antigen, a viral antigen, or a fungal antigen, for example, those described herein.

In some embodiments, the transmembrane domain of (b) in any of the chimeric receptor polypeptide (*e.g.,* ACTR or CAR polypeptide) can be of a single-pass membrane protein, *e.g.,* CD8α, CD8β, 4-1BB, CD28, CD34, CD4, FcεRIγ, CD16A, OX40, CD3ζ, CD3ε, CD3y, CD3δ, TCRα, CD32, CD64, VEGFR2, FAS, and FGFR2B. Alternatively, the transmembrane domain of (b) can be a non-naturally occurring hydrophobic protein segment.

In some embodiments, the at least one co-stimulatory signaling domain of the chimeric receptor polypeptides described herein (*e.g.,* ACTR or CAR polypeptides), if applicable, can be of a co-stimulatory molecule, which can be 4-1BB, CD28, CD28$_{LL \to GG}$ variant, OX40, ICOS, CD27, GITR, HVEM, TIM1, LFA1, and CD2. In some examples, the at least one co-stimulatory signaling domains is a CD28 co-stimulatory signaling domain or a 4-1BB co-stimulatory signaling domain. In some instances, the ACTR polypeptide may comprise two co-stimulatory signaling domains. In some instances, one of the co-stimulatory signaling domains is a CD28 co-stimulatory signaling domain; and the other co-stimulatory domain can be a 4-1BB co-stimulatory signaling domain, an OX40 co-stimulatory signaling domain, a CD27 co-stimulatory signaling domain, or an ICOS co-stimulatory signaling domain. Specific examples include, but are not limited to, CD28 and 4-1BB; or CD28$_{LL \to GG}$ variant and 4-1BB.

In some embodiments, the cytoplasmic signaling domain of (c) in any of the chimeric receptor polypeptides described herein (*e.g.,* ACTR or CAR polypeptides) can be a cytoplasmic domain of CD3ζ or FcεR1γ.

In some embodiments, the hinge domain of any of the chimeric polypeptides described herein (*e.g.,* ACTR or CAR polypeptides), when applicable, can be of CD28, CD16A, CD8α, or IgG. In other examples, the hinge domain is a non-naturally occurring peptide. For example, the non-naturally occurring peptide may be an extended recombinant polypeptide (XTEN) or a (Gly$_4$Ser)$_n$ polypeptide, in which n is an integer of 3-12, inclusive. In some examples, the hinge domain is a short segment, which may contain up to 60 amino acid residues.

In specific examples, an ACTR polypeptide as described herein may comprise (i) a CD28 co-stimulatory domain; and (ii) a CD28 transmembrane domain, a CD28 hinge domain, or a combination thereof. For example, the ACTR polypeptide comprises components (a)-(e) as shown in Table 3. In particular examples, the ACTR polypeptide comprises the amino acid sequence selected from SEQ ID NOs: 1-80.

In specific examples, a CAR polypeptide described herein may comprise (i) a CD28 co-stimulatory domain or a 4-1BB co-stimulatory domain; and (ii) a CD28 transmembrane domain, a CD28 hinge domain, or a combination thereof. In further specific examples, a CAR polypeptide described herein may comprise (i) a CD28 co-stimulatory domain or a 4-1BB co-stimulatory domain, (ii) a CD8 transmembrane domain, a CD8 hinge domain, or a combination thereof. For example, the CAR polypeptide may comprise an amino acid sequence selected from SEQ ID NOs: 104 and 105.

The immune cells described herein, expressing the glucose importation polypeptide and optionally the ACTR polypeptide, may be a natural killer cell, macrophage, neutrophil, eosinophil, or T cell. The immune cells can be derived from peripheral blood mononuclear cells (PBMC), hematopoietic stem cells (HSCs), or induced pluripotent stem cells (iPSCs). In some examples, the immune cell is a T cell, in which the expression of an endogenous T cell receptor, an endogenous major histocompatibility complex, an endogenous beta-2-microglobulin, or a combination thereof has been inhibited or eliminated.

Any of the immune cells described herein may comprise a nucleic acid or a nucleic acid set, which collectively comprises: (a) a first nucleotide sequence encoding the glucose importation polypeptide; and optionally (b) a second nucleotide sequence encoding the chimeric receptor polypeptide (*e.g.,* ACTR or CAR polypeptide). The nucleic acid or the nucleic acid set is an RNA molecule or a set of RNA molecules. In some instances, the immune cell comprises the nucleic acid, which comprises both the first nucleotide sequence and the second nucleotide sequence. Such a nucleic acid may further comprise a third nucleotide sequence located between the first nucleotide sequence and the second nucleotide sequence, wherein the third nucleotide sequence encodes a ribosomal skipping site (*e.g.,* a P2A peptide), an internal ribosome entry site (IRES), or a second promoter.

In some examples, the nucleic acid or the nucleic acid set is comprised within a vector or a set of vectors, which can be an expression vector or a set of expression vectors (*e.g.,* viral vectors such as retroviral vectors, *e.g.,* lentiviral vectors or gamma retroviral vectors). A nucleic acid set or a vector set refers to a group of two or more nucleic acid molecules or two or more vectors, each encoding one of the polypeptides of interest (*i.e.,* the glucose importation polypeptide and the chimeric receptor polypeptide). Any of the nucleic acids described herein is also within the scope of the present disclosure.

In another aspect, the present disclosure provides a pharmaceutical composition, comprising any of the immune cells described herein, a pharmaceutically acceptable carrier. When the immune cells express an ACTR polypeptide, the pharmaceutical composition may further comprise an Fc-containing therapeutic agent, which may be a therapeutic antibody or an Fc fusion protein. The Fc-containing therapeutic agent may bind to a target antigen, which can be a tumor antigen, a pathogenic antigen, or an immune cell specific for an autoantigen. The pathogenic antigen can be a bacterial antigen, a viral antigen, or a fungal antigen.

In some examples, the Fc-containing therapeutic agent can be a therapeutic antibody, including, but not limited to, Adalimumab, Ado-Trastuzumab emtansine, Alemtuzumab, Basiliximab, Bevacizumab, Belimumab, Brentuximab, Canakinumab, Cetuximab, Certolizumab, Daclizumab, Denosumab, Dinutuximab, Eculizumab, Efalizumab, Epratuzumab, Gemtuzumab, Golimumab, hu14.18K322A, Ibritumomab, Infliximab, Ipilimumab, Labetuzumab, Muromonab, Natalizumab, Obinutuzumab, Ofatumumab, Omalizumab, Palivizumab, Panitumumab, Pertuzumab, Ramucirumab, Ranibizumab, Rituximab, Tocilizumab, Trastuzumab, Tositumomab, Ustekinumab, Mogamulizumab and Vedolizumab.

Further, the present disclosure provides a kit, comprising (i) a first pharmaceutical composition that comprises any of the immune cells described herein, which express both the glucose importation polypeptide and the ACTR polypeptide, and a pharmaceutically acceptable carrier; and (ii) an Fc-containing therapeutic agent as described herein and a pharmaceutically acceptable carrier.

Moreover, provided herein is a population of the genetically engineered immune cell described herein for use in inhibiting cells expressing a target antigen (*e.g.,* reducing the number of such cells, blocking cell proliferation, and/or suppressing cell activity) in a subject. When the chimeric receptor polypeptide is an ACTR polypeptide, the subject (*e.g.,* a human patient such as a human patient suffering from a cancer) may have been treated or is being treating with an Fc-containing therapeutic agent specific to the target antigen, *e.g.,* a tumor antigen or a pathogenic antigen (for example, a bacterial antigen, a viral antigen, or a fungal antigen), or an immune cell specific for an autoantigen. In some examples, at least some of the cells expressing the target antigen are located in a low-glucose environment.

In some examples, the immune cells are autologous. In other examples, the immune cells are allogeneic. In any of the methods described herein, the immune cells may be activated, expanded, or both *ex vivo,* if needed. In some instances, the immune cells comprise T cells, which are activated in the presence of one or more of anti-CD3 antibody, anti-CD28 antibody, IL-2, phytohemagglutinin, and an engineered artificial stimulatory cell or particle. In other instances, the immune cells comprise natural killer cells, which are activated in the presence of one or more of 4-1BB ligand, anti-4-1BB antibody, IL-15, anti-IL-15 receptor antibody, IL-2, IL-12, IL-21, K562 cells, and an engineered artificial stimulatory cell or particle.

In some examples, the subject to be treated by the methods described herein may be a human patient suffering from a cancer, for example, carcinoma, lymphoma, sarcoma, blastoma, and leukemia. Additional exemplary target cancer includes, but are not limited to, a cancer of B-cell origin, breast cancer, gastric cancer, neuroblastoma, osteosarcoma, lung cancer, skin cancer, prostate cancer, colon cancer, renal cell carcinoma, ovarian cancer, rhabdomyosarcoma, leukemia, mesothelioma, pancreatic cancer, head and neck cancer, retinoblastoma, glioma, glioblastoma, liver cancer, and thyroid cancer. Exemplary cancers of B-cell origin are selected from the group consisting of B-lineage acute lymphoblastic leukemia, B-cell chronic lymphocytic leukemia, and B-cell non-Hodgkin's lymphoma.

Also within the scope of the present disclosure are the genetically engineered immune cells described herein, which co-express a glucose importation polypeptide and a chimeric receptor polypeptide, for use in treating a target disease or disorder such as cancer or an infectious disorder. When the immune cells express an ACTR polypeptide, it can be co-used with any of the Fc-containing therapeutic agents also disclosed herein, such as a therapeutic antibody.

The details of one or more embodiments of the disclosure are set forth in the description below. Other features or advantages of the present disclosure will be apparent from the detailed description of several embodiments and also from the appended claims.

## BRIEF DESCRIPTION OF THE DRAWINGS

The following drawings form part of the present specification and are included to further demonstrate certain aspects of the present disclosure, which can be better understood by reference to one or more of these drawings in combination with the detailed description of specific embodiments presented herein.

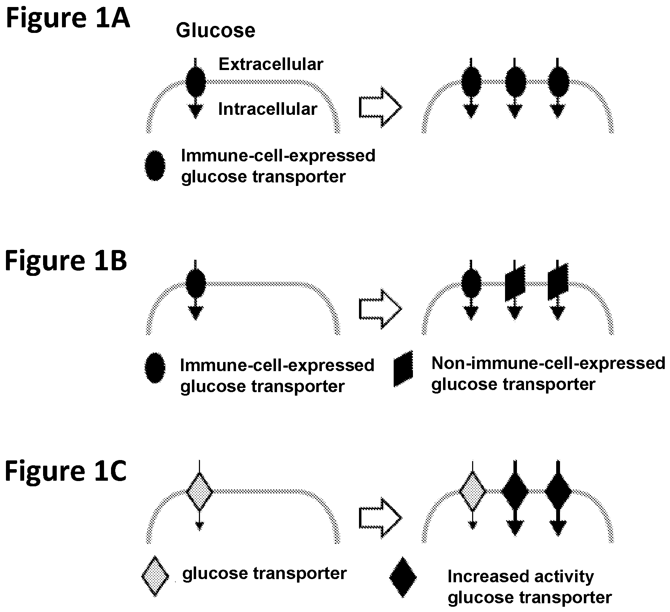

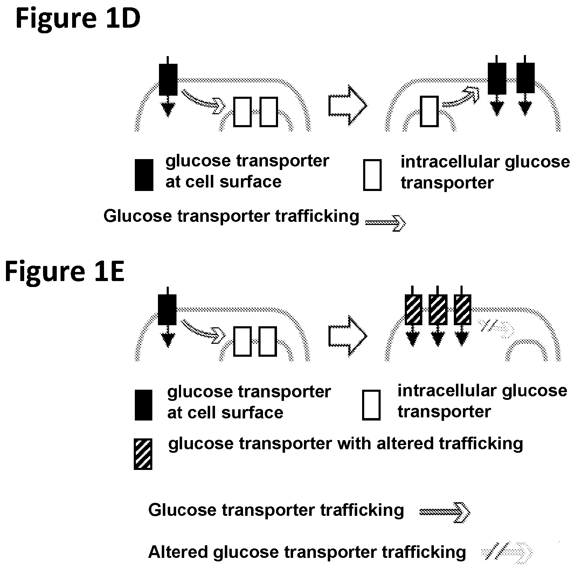

*Figure 1* is a schematic illustration of exemplary strategies for modulating glucose uptake by a cell, including overexpressing an immune-cell expressed transporter (A), expressing a non-immune-cell-expressed transporter (B), expressing a mutant transporter with increased activity (C), modulating trafficking of an intracellular transporter to the cell surface (D), or expressing a mutant transporter that has increased trafficking to the cell surface (E).

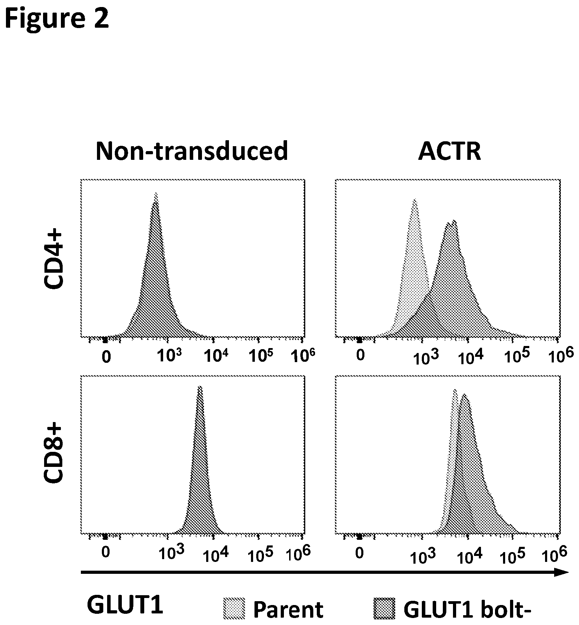

*Figure 2* is a panel of flow-cytometry histograms showing increased expression of GLUT1 in $CD4^+$ and $CD8^+$ T cells expressing ACTR alone (SEQ ID NO: 57) or ACTR in combination with GLUT1 (SEQ ID NO: 81). ACTR-transduced cells are $CD16^+$ and non-transduced cells are $CD16^-$.

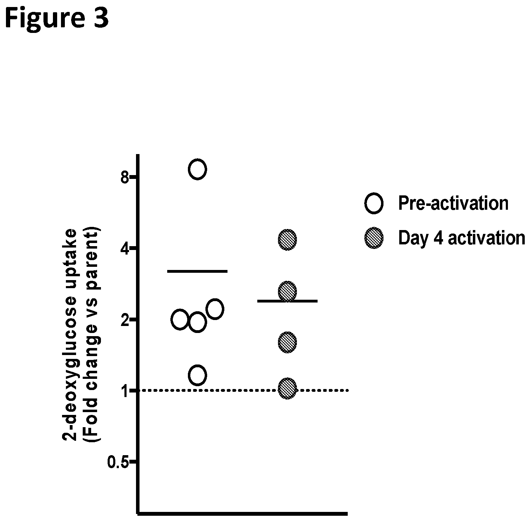

*Figure 3* is a graph showing 2-deoxy-glucose (2DG) uptake of T cells co-expressing ACTR (SEQ ID NO:57) and GLUT1 (SEQ ID NO: 81) relative to T cells expressing ACTR alone (parent; SEQ ID NO: 57) prior to activation (pre-activation) or after 4 days of activation with fixed JHH7 target cells and anti-GPC3 antibody, fixed HepG2 target cells and anti-GPC3 antibody, or fixed IGROV-1 target cells and anti-FOLRα antibody.

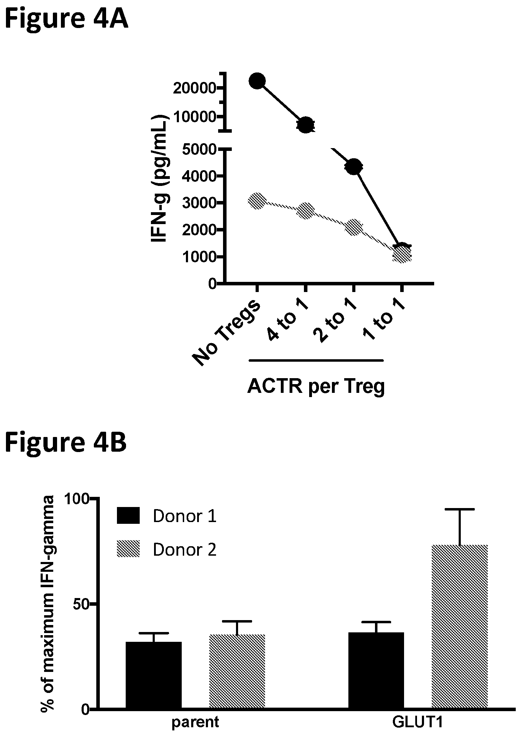

*Figure 4* is a panel of graphs showing the inhibitory effect of regulatory T cells on IFN-gamma production from T cells expressing ACTR alone (parent, SEQ ID NO: 57) in the presence of different ratios of ACTR T cells and regulatory T cells (panel A) and the enhanced effect of ACTR T cells in combination with GLUT1 relative to ACTR T cells alone in the presence of suppressive regulatory T cells as indicated by IFN-gamma production (panel B).

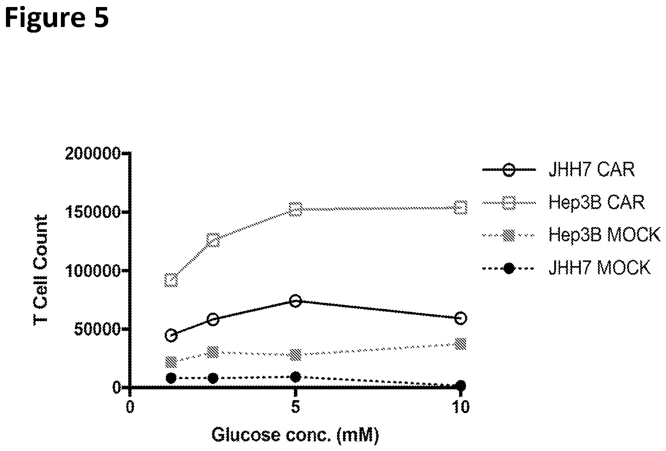

*Figure 5* is a graph demonstrating the number of T cells expressing a GPC3-targeting CAR polypeptide (SEQ ID NO: 104) or mock, untransduced T cells that are present after six days of co-culture in the presence of GPC3-expressing JHH7 or Hep3B target cells and varying concentrations of glucose.

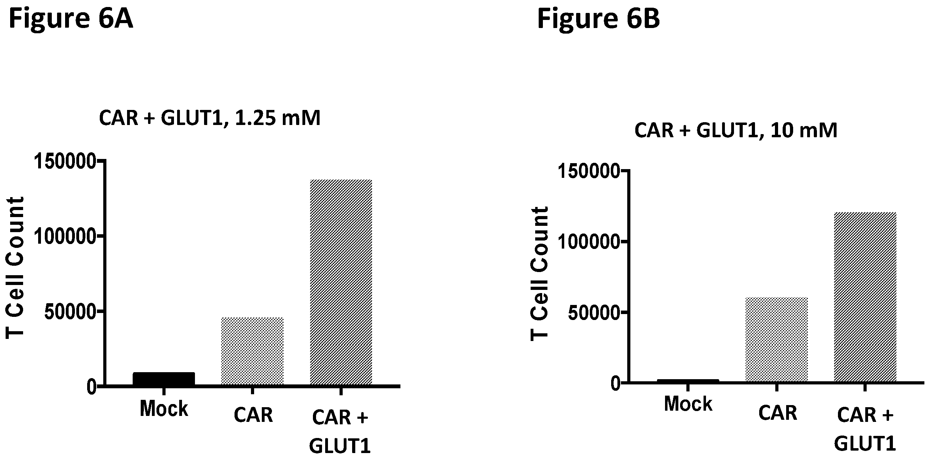

*Figure 6* is a set of graphs (panels A and B) demonstrating the number of T cells expressing a GPC3-targeting CAR polypeptide (SEQ ID NO: 104) or T cells co-expressing a GPC3-targeting CAR polypeptide and a GLUT1 polypeptide (SEQ ID NO: 81) that are present after six days of co-culture in the presence of GPC3-expressing JHH7 target cells and either 1.25 mM or 10 mM glucose.

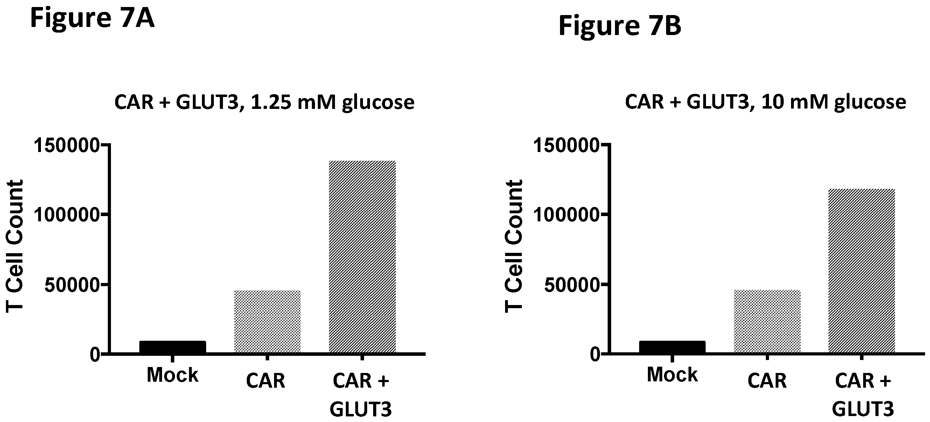

*Figure 7* is a set of graphs (panels A and B) demonstrating the number of T cells expressing a GPC3-targeting CAR polypeptide (SEQ ID NO: 103) or T cells co-expressing the GPC3-targeting CAR polypeptide and a GLUT3 polypeptide (SEQ ID NO: 83) that are present after six days of co-culture in the presence of GPC3-expressing JHH7 target cells and either 1.25 mM or 10 mM glucose.

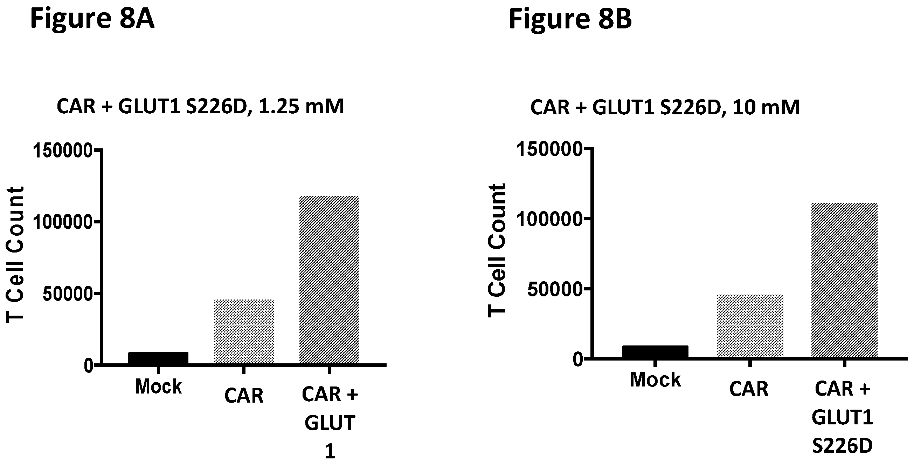

*Figure 8* is a set of graphs (panels A and B) demonstrating the number of T cells expressing a GPC3-targeting CAR polypeptide (SEQ ID NO: 104) or T cells co-expressing the GPC3-targeting CAR polypeptide and a GLUT1 S226D mutant (SEQ ID NO: 82) that are present after six days of co-culture in the presence of GPC3-expressing JHH7 target cells and either 1.25 mM or 10 mM glucose.

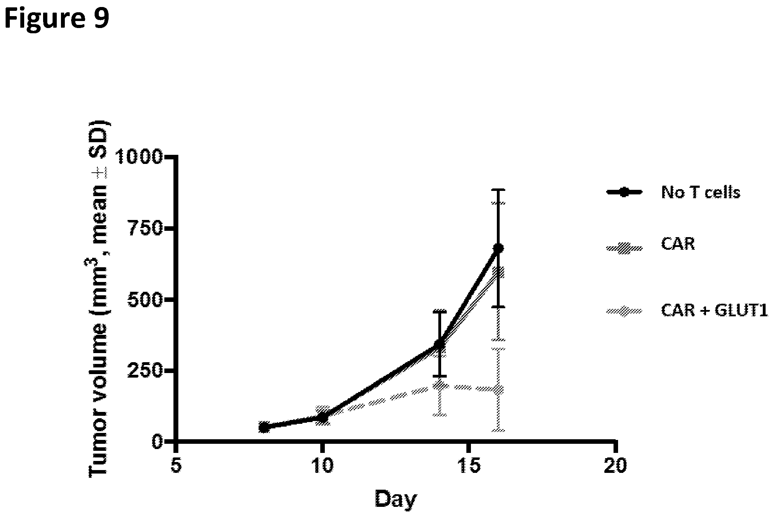

*Figure 9* is a graph showing tumor growth curve data for an *in vivo* mouse JHH7 xenograft model. Results are shown for no treatment, mice treated with a T cell expressing a GPC3-targeting CAR (SEQ ID NO: 104), and mice treated with a T cell co-expressing the GPC3-targeting CAR and GLUT1 (SEQ ID NO: 81).

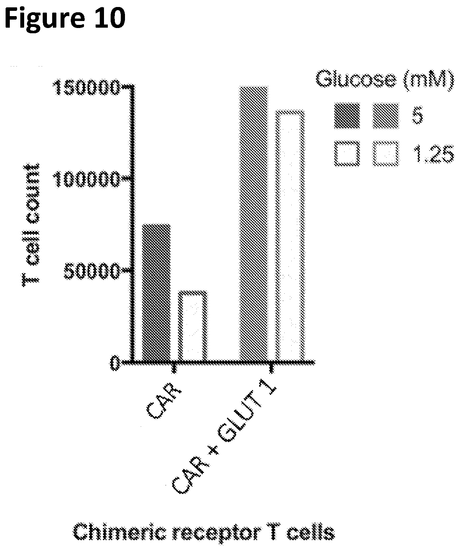

*Figure 10* is a graph showing T cell proliferation for an *in vitro* solid tumor cell line. Results are shown for a T cell expressing a GPC3-targeting CAR (SEQ ID NO: 104), and T cells treated with a T cell co-expressing the GPC3-targeting CAR and GLUT1 (SEQ ID NO: 81).

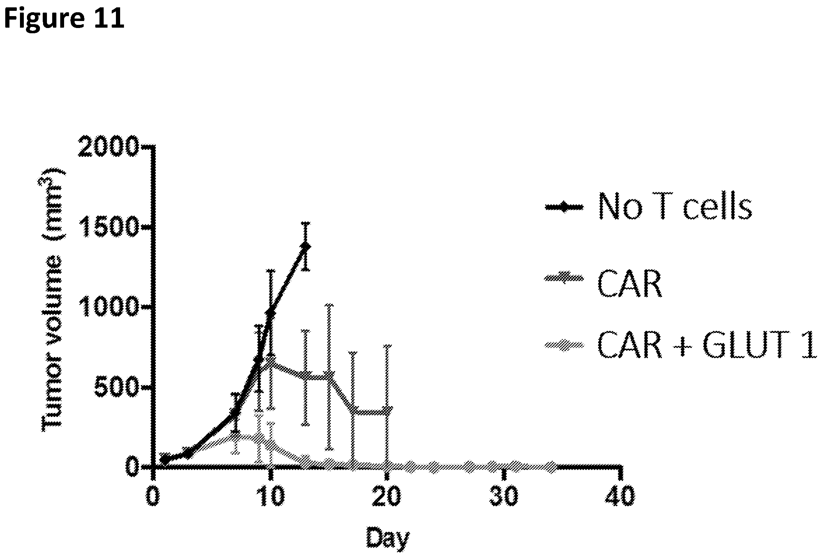

*Figure 11* is a graph showing tumor growth curve data for an *in vivo* mouse JHH7 xenograft model (hepatocellular carcinoma). Results are shown for no treatment, mice treated with a T cell expressing a GPC3-targeting CAR (SEQ ID NO: 104), and mice treated with a T cell co-expressing the GPC3-targeting CAR and GLUT1 (SEQ ID NO: 81).

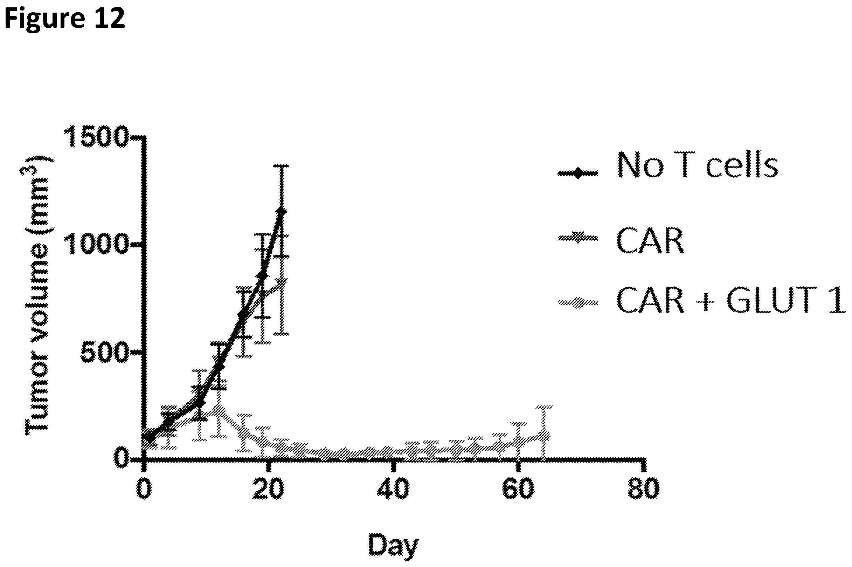

*Figure 12* is a graph showing tumor growth curve data for an *in vivo* mouse Hep3B xenograft model (hepatocellular carcinoma). Results are shown for no treatment, mice treated with a T cell expressing a GPC3-targeting CAR (SEQ ID NO: 104), and mice treated with a T cell co-expressing the GPC3-targeting CAR and GLUT1 (SEQ ID NO: 81).

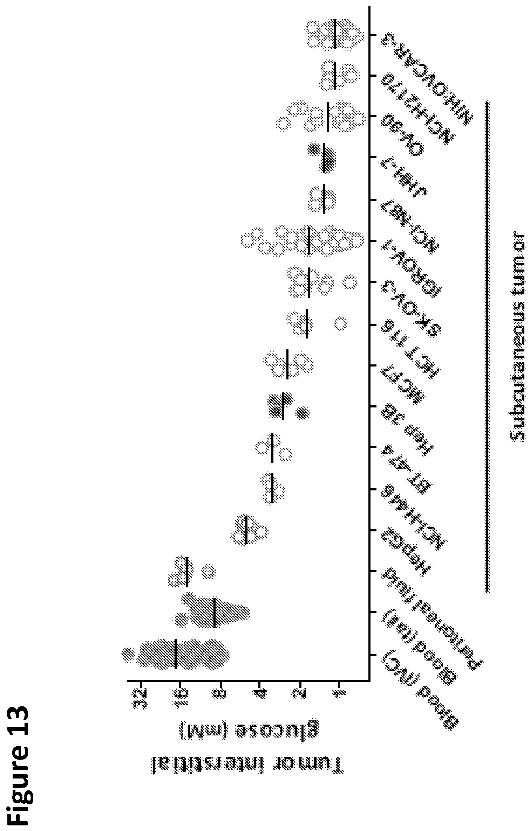

*Figure 13* is a graph showing low glucose levels within interstitial tumor fluid compared to systemic glucose in the blood for *in vivo* xenograft mouse models.

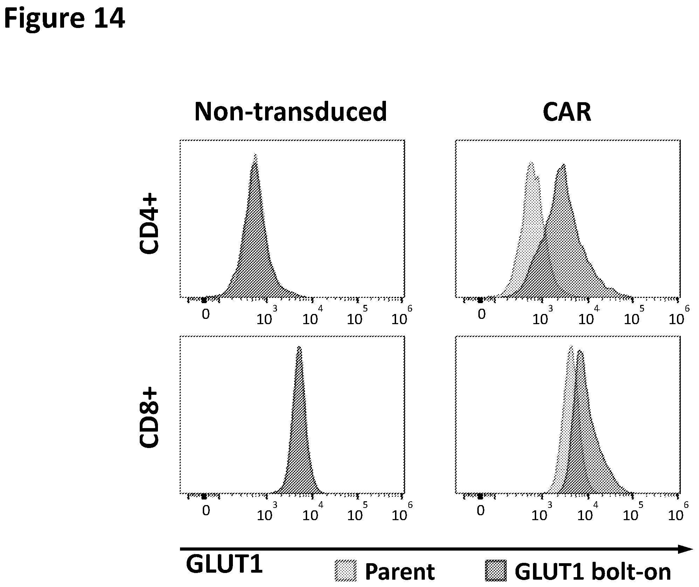

*Figure 14* is a panel of flow-cytometry histograms showing expression of GLUT1 in CD4$^+$ and CD8$^+$ T cells expressing anti-GPC3 CAR alone (SEQ ID NO: 104) or the anti-GPC3 CAR in combination with GLUT1 (SEQ ID NO: 81).

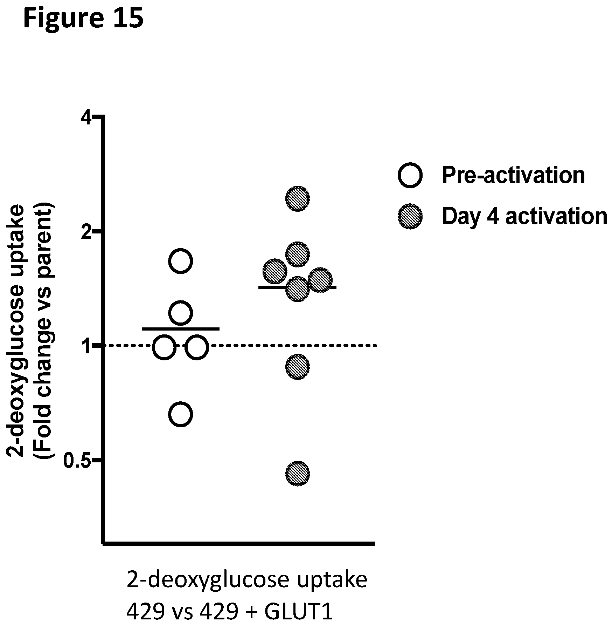

*Figure 15* is a graph showing 2-deoxy-glucose (2DG) uptake of T cells co-expressing anti-GPC3 CAR and GLUT1 (SEQ ID NO: 81) relative to T cells expressing anti-GPC3 CAR alone (parent; SEQ ID NO: 104) prior to activation (pre-activation) or after 4 days of activation with fixed JHH7 or fixed HepG2 target cells.

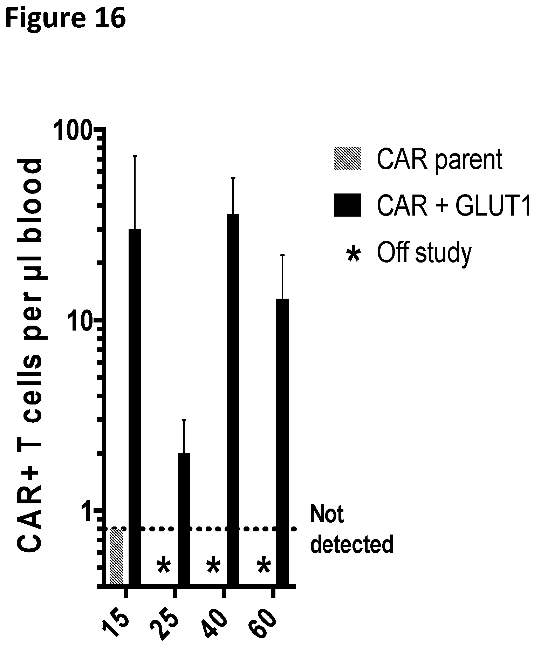

*Figure 16* is a graph showing the number of CAR T cells per μL of blood in blood samples taken from Hep3B tumor-bearing NSG mice treated with T cells expressing anti-GPC3 CAR alone (CAR parent; SEQ ID NO: 104) and T cells co-expressing anti-GPC3 CAR and GLUT1 (SEQ ID NO: 81).

## DETAILED DESCRIPTION OF DISCLOSURE

Tumor microenvironments have specific characteristics, such as low glucose levels, some of which may constrain the activity of effector immune cells such as effector T cells. The present disclosure is based, at least in part, on the development of strategies for enhancing effector immune cell activities in tumor microenvironments. In particular, the present disclosure features methods for enhancing glucose uptake by the effector immune cells, thereby enhancing their growth and bioactivity. Glucose import can be modulated by various factors, including the expression level of glucose transporters, the activation status of such transporters, and/or trafficking of the transporters. The present disclosure provides various approaches to enhance immune cell glucose uptake. Some examples are illustrated in Figure 1, including: overexpressing an immune-cell expressed transporter (A), expressing a non-immune-cell-expressed transporter (B), expressing a mutant transporter with increased activity (C), modulating trafficking of an intracellular transporter to the cell surface (D), or expressing a mutant transporter that has increased trafficking to the cell surface (E). Alternatively, the glucose uptake capacity of immune cells can be improved by regulating expression of endogenous genes coding for proteins involved in glucose importation and/or regulating the cellular trafficking or activity of such proteins.

Enhanced glucose uptake was observed in ACTR-T cells co-expressing GLUT1. Further, activity of ACTR-T cells co-expressing GLUT1 was improved in the presence of suppressive regulatory ($T_{reg}$) T cells. These results indicate that co-expression of GLUT1 in ACTR-T cells would be expected to enhance ACTR-T cell activity, for example, under low glucose conditions. Similarly, co-expression of an anti-GPC3 CAR polypeptide and a GLUT1 polypeptide in T cells enhanced T cell proliferation, particularly under low glucose conditions, enhanced glucose uptake in the T cells, a higher survival level of CAR-T cells and enhanced anti-tumor activity as observed in a mouse model relative to T cells expressing the CAR alone. Since tumor microenvironment typically has a low glucose level, the combination of ACTR-T cells and molecules that enhances glucose import (*e.g.,* GLUT1) would be expected to show enhanced anti-tumor activity. Accordingly, the present disclosure provides a modified (*e.g.,* genetically engineered) immune cell, wherein the immune cell has an improved glucose uptake activity as relative to a wild-type immune cell of the same type, wherein the immune cell is genetically engineered to express or overly express:

(i) a glucose importation polypeptide, and
(ii) a chimeric receptor polypeptide; wherein the chimeric receptor polypeptide comprises:

(a) an extracellular target binding domain;
(b) a transmembrane domain; and
(c) a cytoplasmic signaling domain;

wherein the nucleic acid encoding the glucose importation polypeptide is located extrachromosomally or is integrated into a chromosome of the immune cell at a site that is different from the native loci of the endogenous gene, wherein the glucose importation polypeptide is a glucose transporter (GLUT) or a sodium-glucose cotransporter (SGLT), preferably wherein the glucose importation polypeptide is selected from the group consisting of: GLUT1, GLUT3, GLUT1 S226D, SGLT1, SGLT2, GLUT8, GLUT8 L12A L13A, GLUT11, GLUT7, and GLUT4.

Also provided herein are the genetically engineered immune cells, optionally in combination with an Fc-containing agent when needed (*e.g.,* when the immune cells express an ACTR polypeptide), for use in improving immune

EP 3 817 763 B1

cell proliferation, and/or for use in inhibiting or decreasing in target cells (*e.g.,* target cancer cells) in a subject (*e.g.,* a human cancer patient), *e.g.*, via ADCC. The present disclosure also provides pharmaceutical compositions and kits comprising the described genetically engineered immune cells.

The genetically engineered immune cells described herein, expressing (*e.g.,* over-expressing) a glucose importation polypeptide, may confer at least the following advantages. The expression of the glucose importation polypeptide would enhance the glucose uptake capacity of the immune cells expressing such. As such, the genetically engineered immune cells may proliferate better, produce more cytokines, exhibit greater anti-tumor cytotoxicity, and/or exhibit greater T cell survival in a low-glucose environment (*e.g.,* a low glucose tumor microenvironment) relative to immune cells that do not express (or do not over-express) the glucose importation polypeptide, leading to enhanced cytokine production, survival rate, cytotoxicity, and/or anti-tumor activity.

## I. Glucose Importation Polypeptides

As used herein, a glucose importation polypeptide refers to a polypeptide that mediates glucose uptake (*i.e.,* increases glucose import) across the plasma membrane of cells. Such a glucose importation polypeptide may increase glucose uptake *via* any mechanism. As exemplified in Figure 1, a glucose importation polypeptide may be a glucose transporter, which is a cell membrane protein that facilitates the transport of glucose across the cell membrane. Glucose transporters may be divided into three separate classes: class I (GLUT1-GLUT4), class II (GLUT5, GLUT7, GLUT9, and GLUT11), and class III (GLUT6, GLUT8, GLUT10, GLUT12, and GLUT13) transporters. Any such transporter, which may be of any suitable species (*e.g.,* a mammal such as a human) may be contemplated for use with the compositions and methods described herein.

Alternatively, the glucose importation polypeptide may be a molecule that is mutated to mimic an activated glucose importation polypeptide (*e.g.,* a phosphorylation mimic) or mutated to impact its intracellular trafficking (e.g. traffic to the cell surface) such that glucose importation polypeptide activity is increased. Alternatively, expression of an endogenous glucose importation polypeptide may modulated, for example, by expressing a transcription factor or a microRNA, or by modulating the polypeptide's stability or degradation, for example, by modulating factors that mediate its degradation, for example an E3 ligase that is part of the ubiquitin/proteasome pathway. Additionally, the trafficking of an endogenous glucose importation polypeptide may be modulated, for example, by expressing a polypeptide that increases its trafficking to the cell surface.

Exemplary glucose importation polypeptides may include, but are not limited to, glucose transporters such as GLUT1 *(*e.g., GLUT1 S226D), GLUT3, GLUT8 (*e.g.,* GLUT8 L12A L13A), GLUT11, GLUT7, and GLUT4, and sodium/-glucose co-transporters (e.g., SGLT1 and SGLT2). Amino acid sequences of the representative glucose importation polypeptides are provided below.

### *GLUT1* (SEQ ID NO:81)

```
MEPSSKKLTGRLMLAVGGAVLGSLQFGYNTGVINAPQKVIEEFYNQTWVHRYGESILPTTLTTLWSLS

VAIFSVGGMIGSFSVGLFVNRFGRRNSMLMMNLLAFVSAVLMGFSKLGKSFEMLILGRFIIGVYCGLT
TGFVPMYVGEVSPTALRGALGTLHQLGIVVGILIAQVFGLDSIMGNKDLWPLLLSIIFIPALLQCIVL
PFCPESPRFLLINRNEENRAKSVLKKLRGTADVTHDLQEMKEESRQMMREKKVTILELFRSPAYRQPI
LIAVVLQLSQQLSGINAVFYYSTSIFEKAGVQQPVYATIGSGIVNTAFTVVSLFVVERAGRRTLHLIG
LAGMAGCAILMTIALALLEQLPWMSYLSIVAIFGFVAFFEVGPGPIPWFIVAELFSQGPRPAAIAVAG
FSNWTSNFIVGMCFQYVEQLCGPYVFIIFTVLLVLFFIFTYFKVPETKGRTFDEIASGFRQGGASQSD
KTPEELFHPLGADSQV
```

### *GLUT1 S226D* (SEQ ID NO:82; S226D in boldface)

```
MEPSSKKLTGRLMLAVGGAVLGSLQFGYNTGVINAPQKVIEEFYNQTWVHRYGESILPTTLTTLWSLS

VAIFSVGGMIGSFSVGLFVNRFGRRNSMLMMNLLAFVSAVLMGFSKLGKSFEMLILGRFIIGVYCGLT
TGFVPMYVGEVSPTALRGALGTLHQLGIVVGILIAQVFGLDSIMGNKDLWPLLLSIIFIPALLQCIVL
PFCPESPRFLLINRNEENRAK**D**VLKKLRGTADVTHDLQEMKEESRQMMREKKVTILELFRSPAYRQPI
LIAVVLQLSQQLSGINAVFYYSTSIFEKAGVQQPVYATIGSGIVNTAFTVVSLFVVERAGRRTLHLIG
LAGMAGCAILMTIALALLEQLPWMSYLSIVAIFGFVAFFEVGPGPIPWFIVAELFSQGPRPAAIAVAG
FSNWTSNFIVGMCFQYVEQLCGPYVFIIFTVLLVLFFIFTYFKVPETKGRTFDEIASGFRQGGASQSD
KTPEELFHPLGADSQV
```

### *GLUT3* (SEQ ID NO:83)

8

MGTQKVTPALIFAITVATIGSFQFGYNTGVINAPEKIIKEFINKTLTDKGNAPPSEVLLTSLWSLSVA
IFSVGGMIGSFSVGLFVNRFGRRNSMLIVNLLAVTGGCFMGLCKVAKSVEMLILGRLVTGLFCGLCTG
FVPMYIGEISPTALRGAFGTLNQLGIVVGILVAQIFGLEFILGSEELWPLLLGFTILPAILQSAALPF
CPESPRFLLINRKEEENAKQILQRLWGTQDVSQDIQEMKDESARMSQEKQVTVLELFRVSSYRQPIII
SIVLQLSQQLSGINAVFYYSTGIFKDAGVQEPIYATIGAGVVNTIFTVVSLFLVERAGRRTLHMIGLG
GMAFCSTLMTVSLLLKDNYNGMSFVCIGAILVFVAFFEIGPGPIPWFIVAELFSQGPRPAAMAVAGCS
NWTSNFLVGLLFPSAAHYLGAYVFIIFTGFLITFLAFTFFKVPETRGRTFEDITRAFEGQAHGADRSG
KDGVMEMNSIEPAKETTTNV

**GLUT4 (SEQ ID NO:84)**

MPSGFQQIGSEDGEPPQQRVTGTLVLAVFSAVLGSLQFGYNIGVINAPQKVIEQSYNETWLGRQGPEG
PSSIPPGTLTTLWALSVAIFSVGGMISSFLIGIISQWLGRKRAMLVNNVLAVLGGSLMGLANAAASYE
MLILGRFLIGAYSGLTSGLVPMYVGEIAPTHLRGALGTLNQLAIVIGILIAQVLGLESLLGTASLWPL
LLGLTVLPALLQLVLLPFCPESPRYLYIIQNLEGPARKSLKRLTGWADVSGVLAELKDEKRKLERERP
LSLLQLLGSRTHRQPLIIAVVLQLSQQLSGINAVFYYSTSIFETAGVGQPAYATIGAGVVNTVFTLVS
VLLVERAGRRTLHLLGLAGMCGCAILMTVALLLLERVPAMSYVSIVAIFGFVAFFEIGPGPIPWFIVA
ELFSQGPRPAAMAVAGFSNWTSNFIIGMGFQYVAEAMGPYVFLLFAVLLLGFFIFTFLRVPETRGRTF
DQISAAFHRTPSLLEQEVKPSTELEYLGPDEND

**GLUT7 (SEQ ID NO:85)**

MENKEAGTPPPIPSREGRLQPTLLLATLSAAFGSAFQYGYNLSVVNTPHKVFKSFYNETYFERHATFM
DGKLMLLLWSCTVSMFPLGGLLGSLLVGLLVDSCGRKGTLLINNIFAIIPAILMGVSKVAKAFELIVF
SRVVLGVCAGISYSALPMYLGELAPKNLRGMVGTMTEVFVIVGVFLAQIFSLQAILGNPAGWPVLLAL
TGVPALLQLLTLPFFPESPRYSLIQKGDEATARQALRRLRGHTDMEAELEDMRAEARAERAEGHLSVL
HLCALRSLRWQLLSIIVLMAGQQLSGINAINYYADTIYTSAGVEAAHSQYVTVGSGVVNIVMTITSAV
LVERLGRRHLLLAGYGICGSACLVLTVVLLFQNRVPELSYLGIICVFAYIAGHSIGPSPVPSVVRTEI
FLQSSRRAAFMVDGAVHWLTNFIIGFLFPSIQEAIGAYSFIIFAGICLLTAIYIYVVIPETKGKTFVE
INRIFAKRNRVKLPEEKEETIDAGPPTASPAKETSF

**GLUT8 (SEQ ID NO:86)**

MTPEDPEETQPLLGPPGGSAPRGRRVFLAAFAAALGPLSFGFALGYSSPAIPSLQRAAPPAPRLDDAA
ASWFGAVVTLGAAAGGVLGGWLVDRAGRKLSLLLCSVPFVAGFAVITAAQDVWMLLGGRLLTGLACGV
ASLVAPVYISEIAYPAVRGLLGSCVQLMVVVGILLAYLAGWVLEWRWLAVLGCVPPSLMLLLMCFMPE
TPRFLLTQHRRQEAMAALRFLWGSEQGWEDPPIGAEQSFHLALLRQPGIYKPFIIGVSLMAFQQLSGV
NAVMFYAETIFEEAKFKDSSLASVVVGVIQVLFTAVAALIMDRAGRRLLLVLSGVVMVFSTSAFGAYF
KLTQGGPGNSSHVAISAPVSAQPVDASVGLAWLAVGSMCLFIAGFAVGWGPIPWLLMSEIFPLHVKGV
ATGICVLTNWLMAFLVTKEFSSLMEVLRPYGAFWLASAFCIFSVLFTLFCVPETKGKTLEQITAHFEG
R

**GLUT8 L12A L13A (SEQ ID NO:87, mutations in boldface)**

MTPEDPEETQP**AA**GPPGGSAPRGRRVFLAAFAAALGPLSFGFALGYSSPAIPSLQRAAPPAPRLDDAA
ASWFGAVVTLGAAAGGVLGGWLVDRAGRKLSLLLCSVPFVAGFAVITAAQDVWMLLGGRLLTGLACGV
ASLVAPVYISEIAYPAVRGLLGSCVQLMVVVGILLAYLAGWVLEWRWLAVLGCVPPSLMLLLMCFMPE
TPRFLLTQHRRQEAMAALRFLWGSEQGWEDPPIGAEQSFHLALLRQPGIYKPFIIGVSLMAFQQLSGV
NAVMFYAETIFEEAKFKDSSLASVVVGVIQVLFTAVAALIMDRAGRRLLLVLSGVVMVFSTSAFGAYF
KLTQGGPGNSSHVAISAPVSAQPVDASVGLAWLAVGSMCLFIAGFAVGWGPIPWLLMSEIFPLHVKGV
ATGICVLTNWLMAFLVTKEFSSLMEVLRPYGAFWLASAFCIFSVLFTLFCVPETKGKTLEQITAHFEG
R

**GLUT11 (SEQ ID NO:88)**

MRALRRLIQGRILLLTICAAGIGGTFQFGYNLSIINAPTLHIQEFTNETWQARTGEPLPDHLVLLMWS
LIVSLYPLGGLFGALLAGPLAITLGRKKSLLVNNIFVVSAAILFGFSRKAGSFEMIMLGRLLVGVNAG
VSMNIQPMYLGESAPKELRGAVAMSSAIFTALGIVMGQVVGLRELLGGPQAWPLLLASCLVPGALQLA
SLPLLPESPRYLLIDCGDTEACLAALRRLRGSGDLAGELEELEEERAACQGCRARRPWELFQHRALRR
QVTSLVVLGSAMELCGNDSVYAYASSVFRKAGVPEAKIQYAIIGTGSCELLTAVVSCVVIERVGRRVL
LIGGYSLMTCWGSIFTVALCLQSSFPWTLYLAMACIFAFILSFGIGPAGVTGILATELFDQMARPAAC
MVCGALMWIMLILVGLGFPFIMEALSHFLYVPFLGVCVCGAIYTGLFLPETKGKTFQEISKELHRLNF
PRRAQGPTWRSLEVIQSTEL

### SGLT1 (SEQ ID NO:89)

MDSSTWSPKTTAVTRPVETHELIRNAADISIIVIYFVVVMAVGLWAMFSTNRGTVGGFFLAGRSMVWW
PIGASLFASNIGSGHFVGLAGTGAASGIAIGGFEWNALVLVVVLGWLFVPIYIKAGVVTMPEYLRKRF
GGQRIQVYLSLLSLLLYIFTKISADIFSGAIFINLALGLNLYLAIFLLLAITALYTITGGLAAVIYTD
TLQTVIMLVGSLILTGFAFHEVGGYDAFMEKYMKAIPTIVSDGNTTFQEKCYTPRADSFHIFRDPLTG
DLPWPGFIFGMSILTLWYWCTDQVIVQRCLSAKNMSHVKGGCILCGYLKLMPMFIMVMPGMISRILYT
EKIACVVPSECEKYCGTKVGCTNIAYPTLVVELMPNGLRGLMLSVMLASLMSSLTSIFNSASTLFTMD
IYAKVRKRASEKELMIAGRLFILVLIGISIAWVPIVQSAQSGQLFDYIQSITSYLGPPIAAVFLLAIF
WKRVNEPGAFWGLILGLLIGISRMITEFAYGTGSCMEPSNCPTIICGVHYLYFAIILFAISFITIVVI

SLLTKPIPDVHLYRLCWSLRNSKEERIDLDAEEENIQEGPKETIEIETQVPEKKKGIFRRAYDLFCGL
EQHGAPKMTEEEKAMKMKMTDTSEKPLWRTVLNVNGIILVTVAVFCHAYFA

### SGLT2 (SEQ ID NO:90)

MEEHTEAGSAPEMGAQKALIDNPADILVIAAYFLLVIGVGLWSMCRTNRGTVGGYFLAGRSMVWWPVG
ASLFASNIGSGHFVGLAGTGAASGLAVAGFEWNALFVVLLLGWLFAPVYLTAGVITMPQYLRKRFGGR
RIRLYLSVLSLFLYIFTKISVDMFSGAVFIQQALGWNIYASVIALLGITMIYTVTGGLAALMYTDTVQ
TFVILGGACILMGYAFHEVGGYSGLFDKYLGAATSLTVSEDPAVGNISSFCYRPRPDSYHLLRHPVTG
DLPWPALLLGLTIVSGWYWCSDQVIVQRCLAGKSLTHIKAGCILCGYLKLTPMFLMVMPGMISRILYP
DEVACVVPEVCRRVCGTEVGCSNIAYPRLVVKLMPNGLRGLMLAVMLAALMSSLASIFNSSSTLFTMD
IYTRLRPRAGDRELLLVGRLWVVFIVVVSVAWLPVVQAAQGGQLFDYIQAVSSYLAPPVSAVFVLALF
VPRVNEQGAFWGLIGGLLMGLARLIPEFSFGSGSCVQPSACPAFLCGVHYLYFAIVLFFCSGLLTLTV
SLCTAPIPRKHLHRLVFSLRHSKEEREDLDADEQQGSSLPVQNGCPESAMEMNEPQAPAPSLFRQCLL
WFCGMSRGGVGSPPPLTQEEAAAAARRLEDISEDPSWARVVNLNALLMMAVAVFLWGFYA

The glucose importation polypeptide may be a naturally-occurring polypeptide from a suitable species, for example, a mammalian glucose importation polypeptide such as those derived from human or a non-human primate. Such naturally-occurring polypeptides are known in the art and can be obtained, for example, using any of the above-noted amino acid sequences as a query to search a publicly available gene database, for example GenBank. The glucose importation polypeptide for use in the instant disclosure may share a sequence identity of at least 85% (*e.g.,* 90%, 95%, 97%, 98%, 99%, or above) as any of the exemplary proteins noted above.

The "percent identity" of two amino acid sequences is determined using the algorithm of Karlin and Altschul Proc. Natl. Acad. Sci. USA 87:2264-68, 1990, modified as in Karlin and Altschul Proc. Natl. Acad. Sci. USA 90:5873-77, 1993. Such an algorithm is incorporated into the NBLAST and XBLAST programs (version 2.0) of Altschul, et al. J. Mol. Biol. 215:403-10, 1990. BLAST protein searches can be performed with the XBLAST program, score=50, wordlength=3 to obtain amino acid sequences homologous to the protein molecules of the invention. Where gaps exist between two sequences, Gapped BLAST can be utilized as described in Altschul et al., Nucleic Acids Res. 25(17):3389-3402, 1997. When utilizing BLAST and Gapped BLAST programs, the default parameters of the respective programs (*e.g.,* XBLAST and NBLAST) can be used.

Alternatively, the glucose importation polypeptide may be a functional variant of a native counterpart. Such a functional variant may contain one or more mutations outside the functional domain(s) of the native counterpart. Functional domains of a native glucose importation polypeptide may be known in the art or can be predicted based on its amino acid sequence. Mutations outside the functional domain(s) would not be expected to substantially affect the biological activity of the protein. In some instances, the functional variant may exhibit an increased activity in glucose uptake as relative to the native counterpart. Alternatively, the functional variant may exhibit a decreased activity in glucose uptake as relative to the native counterpart. Additionally, the functional variant may have increased trafficking to the cell surface. Alternatively, the functional variant may have decreased trafficking to the cell surface.

Alternatively or in addition, the functional variant may contain a conservative mutation(s) at one or more positions in the native counterpart (*e.g.,* up to 20 positions, up to 15 positions, up to 10 positions, up to 5, 4, 3, 2, 1 position(s)). As used herein, a "conservative amino acid substitution" refers to an amino acid substitution that does not alter the relative charge or size characteristics of the protein in which the amino acid substitution is made. Variants can be prepared according to methods for altering polypeptide sequence known to one of ordinary skill in the art such as are found in references which compile such methods, *e.g.,* Molecular Cloning: A Laboratory Manual, J. Sambrook, et al., eds., Second Edition, Cold Spring Harbor Laboratory Press, Cold Spring Harbor, New York, 1989, or Current Protocols in Molecular Biology, F.M. Ausubel, et al., eds., John Wiley & Sons, Inc., New York. Conservative substitutions of amino acids include substitutions made amongst amino acids within the following groups: (a) M, I, L, V; (b) F, Y, W; (c) K, R, H; (d) A, G; (e) S, T; (f) Q, N; and (g) E, D.

## II. Chimeric Receptor Polypeptides

As used herein, a chimeric receptor polypeptide refers to a non-naturally occurring molecule that can be expressed on the surface of a host cell. A chimeric receptor polypeptide comprises an extracellular target binding domain that can target an antigen of interest (e.g., an antigen associated with a disease such as cancer or an antigen associated with a pathogen; see discussions herein). An extracellular target binding domain may bind to an antigen of interest directly (*e.g.,* an extracellular antigen binding domain in a CAR polypeptide as disclosed herein). Alternatively, an extracellular target binding domain may bind to the antigen of interest *via* an intermediate, for example, an Fc-containing agent such as an antibody. A chimeric receptor polypeptide may further comprise a transmembrane domain, a hinge domain, a cytoplasmic signaling domain, one or more co-stimulatory domains, a cytoplasmic signaling domain, or a combination thereof. In some instances, the chimeric receptor polypeptide may be free of co-stimulatory domains. The chimeric receptor polypeptides are configured such that, when expressed on a host cell, the extracellular target binding domain is located extracellularly for binding to a target antigen, directly or indirectly. The optional co-stimulatory signaling domain may be located in the cytoplasm for triggering activation and/or effector signaling.

In some embodiments, chimeric receptor polypeptides described herein may further comprise a hinge domain, which may be located at the C-terminus of the extracellular target binding domain and the N-terminus of the transmembrane domain. The hinge may be of any suitable length. In other embodiments, the chimeric receptor polypeptide described herein may have no hinge domain at all. In yet other embodiments, the chimeric receptor polypeptide described herein may have a shortened hinge domain (*e.g.,* including up to 25 amino acid residues).

In some embodiments, a chimeric receptor polypeptide as described herein may comprise, from N-terminus to C-terminus, the extracellular target binding domain, the transmembrane domain, and the cytoplasmic signaling domain. In some embodiments, a chimeric receptor polypeptide as described herein comprises, from N-terminus to C-terminus, the extracellular target binding domain, the transmembrane domain, at least one co-stimulatory signaling domain, and the cytoplasmic signaling domain. In other embodiments, a chimeric receptor polypeptide as described herein comprises, from N-terminus to C-terminus, the extracellular target binding domain, the transmembrane domain, the cytoplasmic signaling domains, and at least one co-stimulatory signaling domain.

In some embodiments, the chimeric receptor polypeptide can be an antibody-coupled T cell receptor (ACTR) polypeptide. As used herein, an ACTR polypeptide (*a.k.a.,* an ACTR construct) refers to a non-naturally occurring molecule that can be expressed on the surface of a host cell and comprises an extracellular domain with binding affinity and specificity for the Fc portion of an immunoglobulin ("Fc binder" or "Fc binding domain"), a transmembrane domain, and a cytoplasmic signaling domain. In some embodiments, the ACTR polypeptides described herein may further include at least one co-stimulatory signaling domain.

In other embodiments, the chimeric receptor polypeptide disclosed herein may be a chimeric antigen receptor (CAR) polypeptide. As used herein, a CAR polypeptide (*a.k.a., a* CAR construct) refers to a non-naturally occurring molecule that can be expressed on the surface of a host cell and comprises an extracellular antigen binding domain, a transmembrane domain, and a cytoplasmic signaling domain. The CAR polypeptides described herein may further include at least one co-stimulatory signaling domain.

The extracellular antigen binding domain may be any peptide or polypeptide that specifically binds to a target antigen, including naturally occurring antigens that are associated with a medical condition (*e.g.,* a disease), or an antigenic moiety conjugated to a therapeutic agent that targets a disease-associated antigen.

In some embodiments, the CAR polypeptides described herein may further include at least one co-stimulatory signaling domain. The CAR polypeptides are configured such that, when expressed on a host cell, the extracellular antigen-binding domain is located extracellularly for binding to a target molecule and the cytoplasmic signaling domain. The optional co-stimulatory signaling domain may be located in the cytoplasm for triggering activation and/or effector signaling.

As used herein, the phrase "a protein X transmembrane domain" (*e.g.,* a CD8 transmembrane domain) refers to any portion of a given protein, *i.e.,* transmembrane-spanning protein X, that is thermodynamically stable in a membrane.

As used herein, the phrase "a protein X cytoplasmic signaling domain," for example, a CD3$\zeta$ cytoplasmic signaling domain, refers to any portion of a protein (protein X) that interacts with the interior of a cell or organelle and is capable of relaying a primary signal as known in the art, which lead to immune cell proliferation and/or activation. The cytoplasmic signaling domain as described herein differs from a co-stimulatory signaling domain, which relays a secondary signal for fully activating immune cells.

As used herein, the phrase "a protein X co-stimulatory signaling domain," *e.g.,* a CD28 co-stimulatory signaling domain, refers to the portion of a given co-stimulatory protein (protein X, such as CD28, 4-1BB, OX40, CD27, or ICOS) that can transduce co-stimulatory signals (secondary signals) into immune cells (such as T cells), leading to fully activation of the immune cells.

### A. *Extracellular Target Binding Domain*

The chimeric receptor polypeptides disclosed herein comprise an extracellular domain that targets an antigen of interest (*e.g.,* those described herein) *via* either direct binding or indirectly binding (through an intermediate such as an antibody). The chimeric receptor polypeptides may be ACTR polypeptides that comprise an Fc binding domain. Alternatively, the chimeric receptor polypeptides may be CAR polypeptides that comprise an extracellular antigen binding domain.

Fc binding domains

The ACTR polypeptides described herein comprise an extracellular domain that is an Fc binding domain, *i.e.,* capable of binding to the Fc portion of an immunoglobulin (*e.g.,* IgG, IgA, IgM, or IgE) of a suitable mammal (*e.g.,* human, mouse, rat, goat, sheep, or monkey). Suitable Fc binding domains may be derived from naturally occurring proteins such as mammalian Fc receptors or certain bacterial proteins (*e.g.,* protein A, protein G). Additionally, Fc binding domains may be synthetic polypeptides engineered specifically to bind the Fc portion of any of the antibodies described herein with high affinity and specificity. For example, such an Fc binding domain can be an antibody or an antigen-binding fragment thereof that specifically binds the Fc portion of an immunoglobulin. Examples include, but are not limited to, a single-chain variable fragment (scFv), a domain antibody, or a nanobody. Alternatively, an Fc binding domain can be a synthetic peptide that specifically binds the Fc portion, such as a Kunitz domain, a small modular immunopharmaceutical (SMIP), an adnectin, an avimer, an affibody, a DARPin, or an anticalin, which may be identified by screening a peptide combinatory library for binding activities to Fc.

In some embodiments, the Fc binding domain is an extracellular ligand-binding domain of a mammalian Fc receptor. As used herein, an "Fc receptor" is a cell surface bound receptor that is expressed on the surface of many immune cells (including B cells, dendritic cells, natural killer (NK) cells, macrophages, neutrophils, mast cells, and eosinophils) and exhibits binding specificity to the Fc domain of an antibody. Fc receptors are typically comprised of at least two immunoglobulin (Ig)-like domains with binding specificity to an Fc (fragment crystallizable) portion of an antibody. In some instances, binding of an Fc receptor to an Fc portion of the antibody may trigger antibody dependent cell-mediated cytotoxicity (ADCC) effects. The Fc receptor used for constructing an ACTR polypeptide as described herein may be a naturally-occurring polymorphism variant (*e.g.,* the CD16 V158 variant), which may have increased or decreased affinity to Fc as compared to a wild-type counterpart. Alternatively, the Fc receptor may be a functional variant of a wild-type counterpart, which carry one or more mutations (*e.g.,* up to 10 amino acid residue substitutions including 1, 2, 3, 4, 5, 6, 7, 8, 9, or 10 mutations) that alter the binding affinity to the Fc portion of an Ig molecule. In some instances, the mutation may alter the glycosylation pattern of the Fc receptor and thus the binding affinity to Fc.

The table below lists a number of exemplary polymorphisms in Fc receptor extracellular domains (see, *e.g.,* Kim et al., J. Mol. Evol. 53:1-9, 2001) which may be used in any of the methods or constructs described herein:

**Table 1. Exemplary Polymorphisms in Fc Receptors**

| Amino Acid Number | 19 | 48 | 65 | 89 | 105 | 130 | 134 | 141 | 142 | 158 |
|---|---|---|---|---|---|---|---|---|---|---|
| FCR10 | R | S | D | I | D | G | F | Y | T | V |
| P08637 | R | S | D | I | D | G | F | Y | I | F |
| S76824 | R | S | D | I | D | G | F | Y | I | V |
| J04162 | R | N | D | V | D | D | F | H | I | V |
| M31936 | S | S | N | I | D | D | F | H | I | V |

(continued)

| Amino Acid Number | 19 | 48 | 65 | 89 | 105 | 130 | 134 | 141 | 142 | 158 |
|---|---|---|---|---|---|---|---|---|---|---|
| M24854 | S | S | N | I | E | D | S | H | I | V |
| X07934 | R | S | N | I | D | D | F | H | I | V |
| X14356 (FcγRII) | N | N | N | S | E | S | S | S | I | I |
| M31932 (FcγRI) | S | T | N | R | E | A | F | T | I | G |
| X06948 (Fcα$^{χεI}$)I) | R | S | E | S | Q | S | E | S | I | V |

Fc receptors are classified based on the isotype of the antibody to which it is able to bind. For example, Fc-gamma receptors (FcγR) generally bind to IgG antibodies, such as one or more subtype thereof (*i.e.,* IgG1, IgG2, IgG3, IgG4); Fc-alpha receptors (FcαR) generally bind to IgA antibodies; and Fc-epsilon receptors (FcεR) generally bind to IgE antibodies. In some embodiments, the Fc receptor is an Fc-gamma receptor, an Fc-alpha receptor, or an Fc-epsilon receptor. Examples of Fc-gamma receptors include, without limitation, CD64A, CD64B, CD64C, CD32A, CD32B, CD16A, and CD16B. An example of an Fc-alpha receptor is FcαR1/CD89. Examples of Fc-epsilon receptors include, without limitation, FcεRI and FcεRII/CD23. The table below lists exemplary Fc receptors for use in constructing the ACTR polypeptides described herein and their binding activity to corresponding Fc domains:

**Table 2. Exemplary Fc Receptors**

| Receptor name | Principal antibody ligand | Affinity for ligand |
|---|---|---|
| FcγRI (CD64) | IgG1 and IgG3 | High (Kd ~ $10^{-9}$ M) |
| FcγRIIA (CD32) | IgG | Low (Kd > $10^{-7}$ M) |
| FcγRIIB1 (CD32) | IgG | Low (Kd > $10^{-7}$ M) |
| FcγRIIB2 (CD32) | IgG | Low (Kd > $10^{-7}$ M) |
| FcγRIIIA (CD16a) | IgG | Low (Kd > $10^{-6}$ M) |
| FcγRIIIB (CD16b) | IgG | Low (Kd > $10^{-6}$ M) |
| FcεRI | IgE | High (Kd ~ $10^{-10}$ M) |
| FcεRII (CD23) | IgE | Low (Kd > $10^{-7}$ M) |
| FcαRI (CD89) | IgA | Low (Kd > $10^{-6}$ M) |
| Fcα/μR | IgA and IgM | High for IgM, Mid for IgA |
| FcRn | IgG | |

Selection of the ligand binding domain of an Fc receptor for use in the ACTR polypeptides described herein will be apparent to one of skill in the art. For example, it may depend on factors such as the isotype of the antibody to which binding of the Fc receptor is desired and the desired affinity of the binding interaction.

In some examples, the Fc binding domain is the extracellular ligand-binding domain of CD16, which may incorporate a naturally occurring polymorphism that may modulate affinity for Fc. In some examples, the Fc binding domain is the extracellular ligand-binding domain of CD16 incorporating a polymorphism at position 158 (*e.g.,* valine or phenylalanine). In some embodiments, the Fc binding domain is produced under conditions that alter its glycosylation state and its affinity for Fc.

The amino acid sequences of human CD16A F158 and CD16A V158 variants are provided below with the F158 and V158 residue highlighted in bold/face and underlined (signal peptide italicized):

### CD16A F158 (SEQ ID NO:91):

*MWQLLLLPTALLLLVSA*GMRTEDLPKAVVFLEPQWYRVLEKDSVTLKCQGAYSPEDNSTQWFHNESLISSQASSYF
IDAATVDDSGEYRCQTNLSTLSDPVQLEVHIGWLLLQAPRWVFKEEDPIHLRCHSWKNTALHKVTYLQNGKGRKY
FHHNSDFYIPKATLKDSGSYFCRGL**F**GSKNVSSETVNITITQGLAVSTISSFFPPGYQ

***CD16A V158 (SEQ ID NO:92):***

*MWQLLLPTALLLLVSA*GMRTEDLPKAVVFLEPQWYRVLEKDSVTLKCQGAYSPEDNSTQWFHNESLISSQAS
SYFIDAATVDDSGEYRCQTNLSTLSDPVQLEVHIGWLLLQAPRWVFKEEDPIHLRCHSWKNTALHKVTYLQN
GKGRKYFHHNSDFYIPKATLKDSGSYFCRGL**V**GSKNVSSETVNITITQGLAVSTISSFFPPGYQVSFCLVMV
LLFAVDTGLYFSVKTNIRSSTRDWKDHKFKWRKDPQDK

In some embodiments, the Fc binding domain is the extracellular ligand-binding domain of CD16 incorporating modifications that render the ACTR polypeptide specific for a subset of IgG antibodies. For example, mutations that increase or decrease the affinity for an IgG subtype (*e.g.*, IgG1) may be incorporated.

Any of the Fc binding domains described herein may have a suitable binding affinity for the Fc portion of a therapeutic antibody. As used herein, "binding affinity" refers to the apparent association constant or $K_A$. The $K_A$ is the reciprocal of the dissociation constant, $K_D$. The extracellular ligand-binding domain of an Fc receptor domain of the ACTR polypeptides described herein may have a binding affinity $K_d$ of at least $10^{-5}$, $10^{-6}$, $10^{-7}$, $10^{-8}$, $10^{-9}$, $10^{-10}$ M or lower for the Fc portion of antibody. In some embodiments, the Fc binding domain has a high binding affinity for an antibody, isotype(s) of antibodies, or subtype(s) thereof, as compared to the binding affinity of the Fc binding domain to another antibody, isotype(s) of antibodies, or subtypes(s) thereof. In some embodiments, the extracellular ligand-binding domain of an Fc receptor has specificity for an antibody, isotype(s) of antibodies, or subtype(s) thereof, as compared to binding of the extracellular ligand-binding domain of an Fc receptor to another antibody, isotype(s) of antibodies, or subtypes(s) thereof.

Other Fc binding domains as known in the art may also be used in the ACTR constructs described herein including, for example, those described in WO2015058018A1 and PCT Application No.: PCT/US2018/015999.

Extracellular antigen binding domains

The CAR polypeptides described herein comprise an extracellular antigen binding domain, which re-directs the specificity of immune cells expressing the CAR polypeptide. As used herein, "an extracellular antigen binding domain" refers to a peptide or polypeptide having binding specificity to a target antigen of interest, which can be a naturally occurring antigen associated with a medical condition (*e.g.*, a disease), or an antigenic moiety conjugated to a therapeutic agent that targets a disease-associated antigen. The extracellular antigen binding domain as described herein does not comprise an extracellular domain of an Fc receptor, and may not bind to the Fc portion of an immunoglobulin. An extracellular domain that does not bind to an Fc fragment means that the binding activity between the two is not detectable using a conventional assay or only background or biologically insignificant binding activity is detected using the conventional assay.

In some instances, the extracellular antigen binding domain of any CAR polypeptides described herein is a peptide or polypeptide capable of binding to a cell surface antigen (*e.g.*, a tumor antigen), or an antigen (or a fragment thereof) that is complex with a major histocompatibility complex and be presented on the cell surface of an antigen-presenting cell. Such an extracellular antigen binding domain may be a single-chain antibody fragment (scFv), which may be derived from an antibody that binds the target cell surface antigen with a high binding affinity. Table 3 below lists exemplary cell-surface target antigens and exemplary antibodies binding to such.

**Table 3. Exemplary Cell Surface Target Antigen and Exemplary Antibodies Binding to Such**

| Exemplary Target Antigens | Exemplary Antibodies | | Exemplary Target Antigens | Exemplary Antibodies and Fc-fusion Agents |
|---|---|---|---|---|
| CD137 (4-1BB) | utomilumab | | CD74 | milatuzumab |
| Trophoblast glycoprotein (5T4) | naptumomab estafenatox | | HLA-DR | IMMU-114 |
| Adenosine A2a receptor (A2aR) | anti-A2aR mAbs | | Hsp70 | mi-TUMEXtx |
| Alk-1 protein kinase (ACVRL1) | ascrinvacumab | | Hsp90 | ZSG-102 |
| ADAM-10 (ADAM 10) | 8C7 | | ICAM-1 | BI-505 |
| TACE (ADAM17) | MEDI-3622 | | Inducible T-cell co-stimulator (ICOS) | GSK-3359609 |
| ADAM-28 (ADAM28) | GFC-201 | | Immunoglobulin kappa (Ig kappa) | KappaMab |

(continued)

| Exemplary Target Antigens | Exemplary Antibodies | | Exemplary Target Antigens | Exemplary Antibodies and Fc-fusion Agents |
|---|---|---|---|---|
| CD156; Immunoglobulin G1; Immunoglobulin G2 (ADAM8) | MAB-1031 | | Immunoglobulin antigen (Ig lambda) | LambdaMab |
| ADAM-9 (ADAM9) | AEX-6003 | | IL-6 receptor (IL-6R) | tocilizumab |
| Anterior gradient protein 2 homolog (AGR2) | agtuzumab | | IL-7 receptor (IL-7R) | anti-IL7R mAbs |
| Anaplastic lymphoma kinase (ALK) | KTN-0125 | | IL-13 receptor alpha 1 subunit (IL13RA1) | ASLAN-004 |
| Angiopoietin ligand-2 (Ang-2); Vascular endothelial growth factor-A (VEGF-A) | vanucizumab | | IL-13 receptor alpha 2 subunit (IL13RA2) | anti-IL13RA2 mAbs |
| Lactadherin (Anti-idiotype) | TriAb (11D10) | | IL-1 receptor accessory protein (IL1RAP) | CAN-04 |
| Tumor necrosis factor ligand 13 (APRIL) | BION-1301 | | IL-2 receptor beta (IL2R beta) | Mikbeta1 |
| Aspartate beta-hydroxylase (ASPH) | PAN-622 | | Immunoglobulin like domain receptor 2 (ILDR2) | BAY-1905254 |
| Axl tyrosine kinase (AXL) | BA-3011 | | Integrin alpha-X/beta-1 (Integrin a10b1) | anti-Integrin a10b1 mAbs |
| CD276 antigen (B7-H3) | BVD m276; hu8H9 | | Integrin alpha-3/beta-1 (Integrin a3b1) | BCMab-1 |
| V-set domain-containing T-cell activation inhibitor 1 (VTCN1; also B7-H4) | FPA-150 | | Integrin alpha-6/beta-4 (Integrin a6b4) | 90Y-ITGA6B4 |
| B-cell activating factor; (BAFF; also TNFSF13B and CD257) | blisibimod | | Integrin alpha-9 (Integrin a9) | GND-001 |
| B-cell activating factor receptor; (BAFF-R; also TNFSF13C and CD268) | VAY736 | | CD49b (Integrin alpha 2) | Vatelizumab |
| BAG molecular chaperone regulator 3 (BAG3) | anti-BAG3 mAbs | | CD49c (Integrin alpha 3) | anti-CD49c mAbs |
| Basigin (BSG; CD147) | cHAb 18 | | CD49d; (Integrin alpha 4) | anti-CD49d mAbs |
| B-cell maturation antigen (BCMA; also TNFRSF17) | SEA-BCMA | | CD51 | abituzumab |
| ADP ribosyl cyclase-2 (BST1) | OX-001 | | CD29 (integrin beta 1) | OS-2966 |
| B and T lymphocyte attenuator (BTLA) | 40E4 | | CD61 (Integrin beta 3) | anti-CD61 mAbs |
| Complement C5a receptor (C5aR) | neutrazumab | | Jagged-1 | anti-Jagged-1 mAbs |
| CACNA2D1 calcium channel subunit (CACNA2D1) | anti-CACNA2D 1 mAbs | | Kidney-associated antigen 1 (KAAG1) | AB-3A4 |
| Carbonic anhydrase-IX (CAIX) | G250 | | Potassium channel subfamily K member 9 (KCNK9) | Y-4 |
| Calreticulin (CALR) | Anti-CALR mAbs | | KIR2DL1/2L3 | lirilumab |
| Caveolin 1 (CAV1) | anti-CAV1 mAbs | | tyrosine-protein kinase kit (KIT) | CDX-0158 |

(continued)

| Exemplary Target Antigens | Exemplary Antibodies | | Exemplary Target Antigens | Exemplary Antibodies and Fc-fusion Agents |
|---|---|---|---|---|
| Carbonic anhydrase-XII (CAXII) | 177Lu-6A10-Fab; anti-CAXII mAbs | | L1CAM | anti-L 1 CAM mAbs |
| CCR2 chemokine receptor (CCR2) | plozalizumab | | Death receptor 5 (DR5) | APOMAB |
| CCR3 chemokine receptor (CCR3) | anti-CCR3 mAbs | | CD223 (LAG3) | relatlimab |
| CCR4 chemokine receptor (CCR4) | mogamulizumab | | Lewis Y | hu3S193; MB311 |
| CCR5 chemokine receptor (CCR5) | PRO 140; CCR5mAb004 | | Zinc transporter SLC39A6 (LIV1) | SGN-LIV1 |
| CCR7 chemokine receptor (CCR7) | anti-CCR7 mAbs | | Lysyl oxidase-like protein 2 (LOXL2) | AB-0023 |
| CCR9 chemokine receptor (CCR9) | anti-CCR9 mAbs | | Leucine rich repeat-containing protein 15 (LRRC15) | ABBV-085 |
| Interleukin-3 receptor alpha (IL3RA; CD123) | CSL362; KHK2823 | | Leucine rich repeat-containing protein 32 (LRRC32) | ARGX-115 |
| Aminopeptidase N (CD13) | MI-130110 | | Lymphocyte antigen 75 (LY75) | MEN-1309 |
| Prominin 1 (CD133) | anti-CD133 mAbs | | Ly6/PLAUR domain-containing protein 3 (LYPD3) | BAY-1129980 |
| Syndecan-1 (CD138) | indatuximab ravtansine | | Melanoma associated antigen (MAGE peptide presented in MHC) | LxC-002 |
| CD160 | ELB-021 | | Matriptase (ST14) | anti-ST14 mAbs |
| Activated leukocyte cell adhesion molecule (CD166) | CX-2009 | | MICA/B | IPH4301 |
| B-lymphocyte antigen CD19 | MOR208 | | MIF/HLA-A2 (MIF peptide presented in MHC) | RL21A |
| B-lymphocyte antigen CD20 | rituximab; obinituzumab; ocaratuzumab | | Anti-mullerian hormone II (MHR2) | GM-102 |
| Membrane glycoprotein OX2 CD200 | samalizumab | | MMP1/HLA (MMP1 peptide presented in MHC1) | Anti-MMP1/HLA mAbs |
| CD22 | epratuzumab | | Metalloprotease-9 (MMP9) | andecaliximab |
| Immunoglobulin epsilon Fc receptor II (CD23) | lumiliximab | | Mesothelin (MSLN) | MORAb-009 |
| Signal transducer CD24 | anti-CD24 mAbs | | Mucin 1 (MUC1) | PankoMab-GEX |
| IL-2 receptor alpha subunit CD25 | 90Y-daclizumab | | Mucin 13 (MUC13) | anti-MUC13 mAbs |
| CD27 | varilumab | | Endomucin (MUC14) | anti-MUC14 mAbs |
| CD28 | theralizumab | | Mucin 16 (MUC16) | sofituzumab |
| CD3 | Muromonab-CD3 (OKT3) | | Cell surface glycoprotein MUC18 (CD146) | AA98 |

(continued)

| Exemplary Target Antigens | Exemplary Antibodies | | Exemplary Target Antigens | Exemplary Antibodies and Fc-fusion Agents |
|---|---|---|---|---|
| CD30 | brentuximab ve-dotin | | Mucin 5AC (MUC5AC) | ensituximab |
| Immunoglobulin gamma Fc receptor IIB (CD32B) | BI-1206 | | N-glycolyl GM3 (NeuGcGM3) | 99mTc-labeled 14F7 |
| CD33 | lintuzumab | | Sodium-dependent phosphate transport protein 2B (SLC34A2) | XMT-1536 |
| CD37 | otlertuzumab | | Nucleolin (NCL) | anti-nucleolin mAbs |
| ADP ribosyl cyclase-1 (CD38) | daratumumab | | Nectin-4 | enfortumab vedotin |
| CD39 | OREG-103 | | Neurofibromin (NF1) | anti-neurofibromin mAbs |
| CD4 | IT-1208 | | NGcGM3 ganglioside | racotumomab |
| CD40 | lucatumumab | | NKG2A | monalizumab |
| CD43 | leukotuximab | | non-POU domain-containing octamer-binding protein (NONO) | PAT-LM1 |
| CD44 | RG7356 | | Notch-1 | brontictuzumab |
| CD45 | 131I-BC8 | | CD73 | oleclumab |
| Membrane cofactor protein (CD46) | AugmAb | | Netrin-1 (NTN1) | NP-137 |
| CD47 | Hu5F9-G4 | | OX-40 | PF-04518600 |
| CD52 | alemtuzumab | | P2X purinoceptor 7 (P2RX7) | BIL-010t |
| CD55 | PAT-SC1 | | FGF receptor (pan FGFR) | MM-161 |
| Neural cell adhesion molecule 1; (CD56) | IMGN-901 | | Integrin (Pan integrin) | NOD201 |
| T-cell differentiation antigen CD6 | itolizumab | | P-cadherin, also cadherin-3 (CDH3) | PCA-062 |
| CD70 | SGN-70 | | Programmed cell death protein 1 (PD-1) | pembrolizumab |
| CD79b | polatuzumab vedotin | | Programmed cell death ligand 1 (PD-L1) | avelumab; Euchloe H12 |
| CD8 | anti-CD8 mAbs | | Programmed cell death ligand 2 (PD-L2) | rHIgM12B7 |
| CD80 | galiximab | | PDGF receptor alpha (PDGFRA) | olaratumumab |
| CD98 | IGN-523 | | Placenta specific protein 1 (PLAC1) | anti-PLAC1 mAbs |
| CD99 | NV-103 | | PR1/HLA (PR1 peptide in MHC) | anti-PR1/HLA mAbs |
| Cadherin-1 (CDH1) | anti-CDH1 mAbs | | Prolactin receptor PRLR | ABBV-176 |
| Cadherin-17 (CDH17) | anti-CDH17 mAbs | | Phosphatidylserine | anti-phosphatidylserine mAbs |
| Cadherin 19 (CDH19) | anti-CDH19 mAbs | | Prostate stem cell antigen (PSCA) | anti-PSCA mAbs |

(continued)

| Exemplary Target Antigens | Exemplary Antibodies | | Exemplary Target Antigens | Exemplary Antibodies and Fc-fusion Agents |
|---|---|---|---|---|
| Cadherin-6 (CDH6) | HKT-288 | | Glutamate carboxypeptidase II (PSMA) | ATL-101 |
| CD66a (CEACAM1) | CM-24 | | Parathyroid hormone-related protein (PTH-rP) | CAL |
| CD66e (CEACAM5) | IMMU-130 | | Tyrosine-protein kinase-like 7 (PTK7) | cofetuzumab pelidotin |
| CD66c; CD66e (CEACAM5/6) | NEO-201 | | Protein tyrosine phosphatase IVA3 (PTP4A3) | PRL3-zumab |
| Claudin 18 (Claudin 18.2) | IMAB362 | | Poliovirus receptor related immunoglobulin domain containing (PVRIG) | COM-701 |
| Claudin 6 | IMAB027 | | Receptor activator of nuclear factor kappa-B ligand (RANKL) | denosumab |
| SLAM family member 7 (CS1) | elotuzumab | | Recepteur d'origine nantais (RON) | anti-RON mAbs |
| colony stimulating factor-1 receptor (CSF1R) | cabiralizumab | | Tyrosine-protein kinase transmembrane receptor ROR1 (ROR1); also NTRKR1 | cirmtuzumab |
| Cytotoxic T-lymphocyte protein-4 (CTLA4) | ipilumumab | | Tyrosine-protein kinase transmembrane receptor ROR2 (ROR2); also NTRKR2 | BA-3021 |
| Coxsackievirus and adenovirus receptor (CXADR) | anti-CXADR mAbs | | R-spondin-3 (RSPO3) | rosmantuzumab |
| CXCR2 chemokine receptor | anti-CXCR2 mAbs | | Sphingosine-1-phosphate receptor 3 (S1PR3) | EDD7H9 |
| CXCR3 chemokine receptor | anti-CXCR3 mAbs | | Surface Antigen In Leukemia (SAIL) | IGN-786 |
| CXCR4 chemokine receptor | ulocuplumab | | Semaphorin-4D (SEMA4D) | VX-15 |
| CXCR5 chemokine receptor | STI-B030X | | carbohydrate antigen 19-9 (CA 19-9) | MVT-1075 |
| CXCR7 chemokine receptor | anti-CXCR7 mAbs | | Sialyl Thomsen nouveau antigen (STn) | anti-STn mAbs |
| DCLK1 | anti-DCLK1 mAbs | | Sialic acid-binding Ig-like lectin 8 (Siglec-8) | AK-002 |
| Dickkopf-related protein 1 (DKK1) | BHQ-880 | | Sialic acid-binding Ig-like lectin 9 (Siglec-9) | anti-Siglec-9 mAbs |
| DLK1 | ADCT-701 | | Signal Regulatory Protein Alpha (SIRPA) | OSE-172 |
| Delta-like protein ligand 3 (DLL3) | SC16LD6.5 | | CD48; also SLAM family member 2 (SLAMF2) | SGN-CD48A |
| Delta-like protein ligand 4 (DLL4); VEGF (VEGF) | navicixizumab | | CD352; SLAM family member 6 (SLAMF6) | SGN-CD352A |
| Dipeptidyl peptidase-4 (DPP4), (also CD26) | YSCMA | | Neutral amino acid transporter B0 (SLC1A5) | KM-8094 |

18

(continued)

| Exemplary Target Antigens | Exemplary Antibodies | | Exemplary Target Antigens | Exemplary Antibodies and Fc-fusion Agents |
|---|---|---|---|---|
| Death receptor-3 (DR3) | PTX-35 | | Somatostatin 2 receptor (SSTR2) | XmAb-18087 |
| TRAIL-1 receptor (DR4) | HuYON007 MultYbody | | Stabilin 1 (STAB1) | FP-1305 |
| TRAIL-1 receptor; TRAIL-2 receptor (DR4/DR5) | DR4/DR5 Surrobody | | Metalloreductase (STEAP1) | 89Zr-DFO-MSTP2109A |
| TRAIL-2 receptor (DR5) | DS-8273 | | Survivin | anti-survivin mAbs |
| EGF-like protein 6 (EGFL6) | anti-EGFL6 mAbs | | TAG-72 | 90Y-IDEC-159 |
| Epidermal growth factor receptor (EGFR) | cetuximab; Sym004; nimotuzumab | | T cell receptor (TCR) | anti-TCR mAbs |
| Epidermal growth factor receptor vIII (EGFRvIII) | ABT-806 | | Endosialin (TEM1) | ontuxizumab |
| Epithelial membrane protein 2 (EMP2) | ONCR-201 | | Anthrax toxin receptor 1 (ANTXR1); also TEM8 | anti-TEM8 mAbs |
| Endoglin | carotuximab | | Tissue factor (TF) | MORAb-066 |
| Ectonucleotide pyrophosphatase/pho sphodiesterase family member 3 (ENPP3) | AGS-16C3F | | Transforming growth factor, beta receptor II TGF-beta type II (TGFBR2) | anti-TGFBR2 mAbs |
| Prostaglandin $E_2$ receptor 2 (PTGER2) | anti-PTGER2 mAbs | | Thomsen-Friedenreich Antigen | JAA-F11 |
| Prostaglandin $E_2$ receptor 4 (PTGER4) | anti-PTGER4 mAbs | | T cell immunoreceptor with Ig and ITIM domains (TIGIT) | BMS-986207 |
| EpCAM | oportuzumab monatox | | Hepatitis A virus cellular receptor 1 (HAVCR1); also TIM-1 | CDX-014 |
| Ephrin type-A receptor 2 (EphA2) | MEDI-547 | | Hepatitis A virus cellular receptor 2 (HAVCR2); also TIM-3 | MBG453 |
| Ephrin type-A receptor 3 (EphA3) | KB004 | | Toll-like receptor 2 (TLR-2) | OPN-305 |
| Fibroblast activation protein (FAP) | F19 | | Toll-like receptor 4 (TLR-4) | anti-TLR4 mAbs |
| CD95 (FAS) | asunercept | | Transmembrane 4 L6 family member 1 (TM4SF 1) | anti-TM4SF1 mAbs |
| Fc receptor like protein 5 (FCRL5) | RG-6160 | | Tumor necrosis factor receptor 2 (TNFR2) | anti-TNFR2 mAbs |
| FGF receptor 1 (FGFR1) | FP-1039 | | CD71 | anti-CD71 mAbs |
| FGF receptor 2b (FGFR2b) | FPA-144 | | Triggering receptor expressed on myeloid cells 1 (TREM1) | anti-TREM1 mAbs |
| FGF receptor 3 (FGFR3) | B-701 | | Tumor-associated calcium signal transducer 2 (Trop-2) | DS-1062 |

(continued)

| Exemplary Target Antigens | Exemplary Antibodies | | Exemplary Target Antigens | Exemplary Antibodies and Fc-fusion Agents |
|---|---|---|---|---|
| fms-like tyrosine kinase 3 (FLT3) | Flysyn | | TWEAK Receptor (TWEAKR) | MRT-101 |
| Folate receptor alpha (FOLR1) | farletuzumab; IMGN853; KHK2805 | | Tyrosine-protein kinase receptor TYRO3 (TYRO3) | ELB-031 |
| Folate receptor beta (FOLR2) | anti-FOLR beta mAbs | | Urokinase receptor (uPAR) | MNPR-101 |
| Frizzled-1; Frizzled-2; Frizzled-5; Frizzled-7; Frizzled-8; (FZD1,2,5,7,8) | vantictumab | | VEGF-2 (VEGFR2) | ramucirumab |
| Follistatin-like protein 1 (FSTL1) | anti-FSTL1 mAbs | | Vimentin | pritumumab |
| Fucosyl-GM1 | BMS-986012 | | V-domain Ig suppressor of T cell activation (VISTA) | JNJ-61610588 |
| Frizzled-10 (FZD10) | OTSA-101 | | Integrin alpha-4/beta-1 | natalizumab |
| GCSF-R (Also, CD114 and CSFR3) | CSL324 | | Immunoglobulin iota chain (VPREB 1) | anti-VPREB1 mAbs |
| Galectin 3 binding protein (LGALS3) | MP-1959 | | Wilms tumor protein (WT1/HLA); WT1 peptide presented in MHC | ESK1 |
| Guanylate cyclase 2C (GUCY2C) | TAK-164 | | Glypican-3 (GPC3) | codrituzumab |
| GD2 | dinutuximab | | Transmembrane glycoprotein NMB (GPNMB) | CDX-011 |
| GD3 | PF-06688992 | | Leucine-rich repeat-containing G-protein coupled receptor 5 (LGR5) | BNC-101 |
| glucocorticoid-induced TNFR-related protein (GITR) | BMS-986156 | | G-protein coupled receptor family C group 5 member D (GPRC5D) | JNJ-64407564 |
| glucocorticoid-induced TNFR-related protein ligand (GITRL) | EU-102 | | Ferritin | Ferritarg P |
| premelanocyte protein (PMEL) | anti-PMEL mAbs | | Erbb2 tyrosine kinase (HER2) | trastuzumab; pertuzumab; margetuximab |
| Cell surface A33 antigen (GPA33) | Anti-GPA33 mAbs | | Erbb3 tyrosine kinase (HER3) | patritumab |
| Glypican-1 (GPC1) | MIL-38 | | Globo H | OBI-888 |

The extracellular antigen binding domain may comprise an antigen binding fragment (*e.g.,* a scFv) derived from any of the antibodies listed in Table 1 depending upon the target antigen of interest.

In other embodiments, the extracellular antigen binding domain of any of the CAR polypeptides described herein may be specific to a pathogenic antigen, such as a bacterial antigen, a viral antigen, or a fungal antigen. Some examples are provided below: influenza virus neuraminidase, hemagglutinin, or M2 protein, human respiratory syncytial virus (RSV) F glycoprotein or G glycoprotein, herpes simplex virus glycoprotein gB, gC, gD, or gE, Chlamydia MOMP or PorB protein, Dengue virus core protein, matrix protein, or glycoprotein E, measles virus hemagglutinin, herpes simplex virus type 2 glycoprotein gB, poliovirus I VP1, envelope glycoproteins of HIV 1, hepatitis B core antigen or surface antigen, diptheria toxin, Streptococcus 24M epitope, Gonococcal pilin, pseudorabies virus g50 (gpD), pseudorabies virus II (gpB), pseudorabies virus III (gpC), pseudorabies virus glycoprotein H, pseudorabies virus glycoprotein E, transmissible gastroenteritis glycoprotein 195, transmissible gastroenteritis matrix protein, or human hepatitis C virus glycoprotein E1 or E2.

In addition, the extracellular antigen binding domain of the CAR polypeptide described herein may be specific to a tag conjugated to a therapeutic agent, which targets an antigen associated with a disease or disorder (*e.g.,* a tumor antigen or a pathogenic antigen as described herein). In some instances, the tag conjugated to the therapeutic agent can be antigenic and the extracellular antigen binding domain of the CAR polypeptide can be an antigen-binding fragment (*e.g.,* scFv) of an antibody having high binding affinity and/or specificity to the antigenic tag. Exemplary antigenic tags include, but are not limited to, biotin, avidin, a fluorescent molecule (*e.g.,* GFP, YRP, luciferase, or RFP), Myc, Flag, His (*e.g.,* poly His such as 6xHis), HA (hemeagglutinin), GST, MBP (maltose binding protein), KLH (keyhole limpet hemocyanins), trx, T7, HSV, VSV (*e.g.,* VSV-G), Glu-Glu, V5, e-tag, S-tag, KT3, E2, Au1, Au5, and/or thioredoxin.

In other instances, the tag conjugated to the therapeutic agent is a member of a ligand-receptor pair and the extracellular antigen binding domain comprises the other member of the ligand-receptor pair or a fragment thereof that binds the tag. For example, the tag conjugated to the therapeutic agent can be biotin and the extracellular antigen binding domain of the CAR polypeptide can comprise a biotin-binding fragment of avidin. See, *e.g.,* Urbanska et al., 2012, Lohmueller et al., 2018. Other examples include anti-Tag CAR, in which the extracellular antigen binding domain is a scFv fragment specific to a protein tag, such as FITC (Tamada *et al.,* 2012, Kim *et al.,* 2015; Cao *et al.,* 2016; and Ma *et al.,* 2016), PNE (Rodgers *et al.,* 2016), La-SS-B (Cartellieri *et al.,* 2016), Biotin (Lohmullular *et al.,* 2017), and Leucine-Zipper (Cho *et al.,* 2018). Selection of the antigen binding domain for use in the CAR polypeptides described herein will be apparent to one of skill in the art. For example, it may depend on factors such as the type of target antigen and the desired affinity of the binding interaction.

The extracellular antigen binding domain of any of the CAR polypeptides described herein may have suitable binding affinity for a target antigen (*e.g.,* any one of the targets described herein) or antigenic epitopes thereof. As used herein, "binding affinity" refers to the apparent association constant or $K_A$. The $K_A$ is the reciprocal of the dissociation constant ($K_D$). The extracellular antigen binding domain for use in the CAR polypeptides described herein may have a binding affinity ($K_D$) of at least $10^{-5}$, $10^{-6}$, $10^{-7}$, $10^{-8}$, $10^{-9}$, $10^{-10}$ M, or lower for the target antigen or antigenic epitope. An increased binding affinity corresponds to a decreased $K_D$. Higher affinity binding of an extracellular antigen binding domain for a first antigen relative to a second antigen can be indicated by a higher $K_A$ (or a smaller numerical value $K_D$) for binding the first antigen than the $K_A$ (or numerical value $K_D$) for binding the second antigen. In such cases, the extracellular antigen binding domain has specificity for the first antigen (*e.g.,* a first protein in a first conformation or mimic thereof) relative to the second antigen (*e.g.,* the same first protein in a second conformation or mimic thereof; or a second protein). Differences in binding affinity (*e.g.,* for specificity or other comparisons) can be at least 1.5, 2, 3, 4, 5, 10, 15, 20, 37.5, 50, 70, 80, 91, 100, 500, 1000, 10,000 or $10^5$ fold.

Binding affinity (or binding specificity) can be determined by a variety of methods including equilibrium dialysis, equilibrium binding, gel filtration, ELISA, surface plasmon resonance, or spectroscopy (*e.g.,* using a fluorescence assay). Exemplary conditions for evaluating binding affinity are in HBS-P buffer (10 mM HEPES pH7.4, 150 mM NaCl, 0.005% (v/v) Surfactant P20). These techniques can be used to measure the concentration of bound binding protein as a function of target protein concentration. The concentration of bound binding protein ([Bound]) is generally related to the concentration of free target protein ([Free]) by the following equation:

$$[\text{Bound}] = [\text{Free}]/(\text{Kd} + [\text{Free}])$$

It is not always necessary to make an exact determination of $K_A$, though, since sometimes it is sufficient to obtain a quantitative measurement of affinity, *e.g.,* determined using a method such as ELISA or FACS analysis, is proportional to $K_A$, and thus can be used for comparisons, such as determining whether a higher affinity is, *e.g.,* 2-fold higher, to obtain a qualitative measurement of affinity, or to obtain an inference of affinity, *e.g.,* by activity in a functional assay, *e.g.,* an *in vitro* or *in vivo* assay.

## B. Transmembrane domain

The transmembrane domain of the chimeric receptor polypeptides (*e.g.,* ACTR polypeptides or CAR polypepides) described herein can be in any form known in the art. As used herein, a "transmembrane domain" refers to any protein structure that is thermodynamically stable in a cell membrane, preferably a eukaryotic cell membrane. A transmembrane domain compatible for use in the chimeric receptor polypeptides used herein may be obtained from a naturally occurring protein. Alternatively, it can be a synthetic, non-naturally occurring protein segment, *e.g.,* a hydrophobic protein segment that is thermodynamically stable in a cell membrane.

Transmembrane domains are classified based on the three dimensional structure of the transmembrane domain. For example, transmembrane domains may form an alpha helix, a complex of more than one alpha helix, a beta-barrel, or any other stable structure capable of spanning the phospholipid bilayer of a cell. Furthermore, transmembrane domains may also or alternatively be classified based on the transmembrane domain topology, including the number of passes that the transmembrane domain makes across the membrane and the orientation of the protein. For example, single-pass membrane proteins cross the cell membrane once, and multi-pass membrane proteins cross the cell membrane at least twice (*e.g.,* 2, 3, 4, 5, 6, 7 or more times).

Membrane proteins may be defined as Type I, Type II or Type III depending upon the topology of their termini and membrane-passing segment(s) relative to the inside and outside of the cell. Type I membrane proteins have a single membrane-spanning region and are oriented such that the N-terminus of the protein is present on the extracellular side of the lipid bilayer of the cell and the C-terminus of the protein is present on the cytoplasmic side. Type II membrane proteins also have a single membrane-spanning region but are oriented such that the C-terminus of the protein is present on the extracellular side of the lipid bilayer of the cell and the N-terminus of the protein is present on the cytoplasmic side. Type III membrane proteins have multiple membrane-spanning segments and may be further sub-classified based on the number of transmembrane segments and the location of N- and C-termini.

In some embodiments, the transmembrane domain of the chimeric receptor polypeptide described herein is derived from a Type I single-pass membrane protein. Single-pass membrane proteins include, but are not limited to, CD8α, CD8β, 4-1BB/CD137, CD27, CD28, CD34, CD4, FcεRIγ, CD16, OX40/CD134, CD3ζ, CD3ε, CD3γ, CD3δ, TCRα, TCRβ, TCRζ, CD32, CD64, CD64, CD45, CD5, CD9, CD22, CD37, CD80, CD86, CD40, CD40L/CD154, VEGFR2, FAS, and FGFR2B. In some embodiments, the transmembrane domain is from a membrane protein selected from the following: CD8α, CD8β, 4-1BB/CD137, CD28, CD34, CD4, FcεRIγ, CD16, OX40/CD134, CD3ζ, CD3ε, CD3γ, CD3δ, TCRα, CD32, CD64, VEGFR2, FAS, and FGFR2B. In some examples, the transmembrane domain is of CD8 (*e.g.,* the transmembrane domain is of CD8α). In some examples, the transmembrane domain is of 4-1BB/CD137. In other examples, the transmembrane domain is of CD28. In some cases, the chimeric receptor polypeptide described herein may be free of a hinge domain from any non-CD16A receptor. In some instances, such a chimeric receptor polypeptide may be free of any hinge domain. In other examples, the transmembrane domain is of CD34. In yet other examples, the transmembrane domain is not derived from human CD8α. In some embodiments, the transmembrane domain of the chimeric receptor polypeptide is a single-pass alpha helix.

Transmembrane domains from multi-pass membrane proteins may also be compatible for use in the chimeric receptor polypeptides described herein. Multi-pass membrane proteins may comprise a complex alpha helical structure (e.g., at least 2, 3, 4, 5, 6, 7 or more alpha helices) or a beta sheet structure. Preferably, the N-terminus and the C-terminus of a multi-pass membrane protein are present on opposing sides of the lipid bilayer, *e.g.,* the N-terminus of the protein is present on the cytoplasmic side of the lipid bilayer and the C-terminus of the protein is present on the extracellular side. Either one or multiple helix passes from a multi-pass membrane protein can be used for constructing the chimeric receptor polypeptide described herein.

Transmembrane domains for use in the chimeric receptor polypeptides described herein can also comprise at least a portion of a synthetic, non-naturally occurring protein segment. In some embodiments, the transmembrane domain is a synthetic, non-naturally occurring alpha helix or beta sheet. In some embodiments, the protein segment is at least approximately 20 amino acids, *e.g.,* at least 18, 19, 20, 21, 22, 23, 24, 25, 26, 27, 28, 29, 30, or more amino acids. Examples of synthetic transmembrane domains are known in the art, for example in U.S. Patent No. 7,052,906 B1 and PCT Publication No. WO 2000/032776 A2.

In some embodiments, the amino acid sequence of the transmembrane domain does not comprise cysteine residues. In some embodiments, the amino acid sequence of the transmembrane domain comprises one cysteine residue. In some embodiments, the amino acid sequence of the transmembrane domain comprises two cysteine residues. In some embodiments, the amino acid sequence of the transmembrane domain comprises more than two cysteine residues (*e.g.,* 3, 4, 5, or more).

The transmembrane domain may comprise a transmembrane region and a cytoplasmic region located at the C-terminal side of the transmembrane domain. The cytoplasmic region of the transmembrane domain may comprise three or more amino acids and, in some embodiments, helps to orient the transmembrane domain in the lipid bilayer. In some embodiments, one or more cysteine residues are present in the transmembrane region of the transmembrane domain. In some embodiments, one or more cysteine residues are present in the cytoplasmic region of the transmembrane domain. In some embodiments, the cytoplasmic region of the transmembrane domain comprises positively charged amino acids. In some embodiments, the cytoplasmic region of the transmembrane domain comprises the amino acids arginine, serine, and lysine.

In some embodiments, the transmembrane region of the transmembrane domain comprises hydrophobic amino acid residues. In some embodiments, the transmembrane region comprises mostly hydrophobic amino acid residues, such as alanine, leucine, isoleucine, methionine, phenylalanine, tryptophan, or valine. In some embodiments, the transmembrane region is hydrophobic. In some embodiments, the transmembrane region comprises a poly-leucine-alanine sequence.

The hydropathy, hydrophobic or hydrophilic characteristics of a protein or protein segment, can be assessed by any method known in the art including, for example, the Kyte and Doolittle hydropathy analysis.

### C. *Co-stimulatory signaling domains*

Many immune cells require co-stimulation, in addition to stimulation of an antigen-specific signal, to promote cell proliferation, differentiation and survival, as well as to activate effector functions of the cell. In some embodiments, the chimeric receptor polypeptides, such as ACTR or CAR polypeptides, described herein comprise at least one co-stimulatory signaling domain. In certain embodiments, the chimeric polypeptides may contain a CD28 co-stimulatory signaling domain or a 4-1BB (CD137) co-stimulatory signaling domain. The term "co-stimulatory signaling domain," as used herein, refers to at least a fragment of a co-stimulatory signaling protein that mediates signal transduction within a cell to induce an immune response such as an effector function (a secondary signal). As known in the art, activation of immune cells such as T cells often requires two signals: (1) the antigen specific signal (primary signal) triggered by the engagement of T cell receptor (TCR) and antigenic peptide/MHC complexes presented by antigen presenting cells, which typically is driven by CD3ζ as a component of the TCR complex; and (ii) a co-stimulatory signal (secondary signal) triggered by the interaction between a co-stimulatory receptor and its ligand. A co-stimulatory receptor transduces a co-stimulatory signal (secondary signal) as an addition to the TCR-triggered signaling and modulates responses mediated by immune cells, such as T cells, NK cells, macrophages, neutrophils, or eosinophils.

Activation of a co-stimulatory signaling domain in a host cell (*e.g.,* an immune cell) may induce the cell to increase or decrease the production and secretion of cytokines, phagocytic properties, proliferation, differentiation, survival, and/or cytotoxicity. The co-stimulatory signaling domain of any co-stimulatory molecule may be compatible for use in the chimeric polypeptides described herein. The type(s) of co-stimulatory signaling domain is selected based on factors such as the type of the immune cells in which the chimeric polypeptides would be expressed (*e.g.,* T cells, NK cells, macrophages, neutrophils, or eosinophils) and the desired immune effector function (e.g., ADCC). Examples of co-stimulatory signaling domains for use in the chimeric polypeptides may be the cytoplasmic signaling domain of co-stimulatory proteins, including, without limitation, members of the B7/CD28 family (*e.g.,* B7-1/CD80, B7-2/CD86, B7-H1/PD-L1, B7-H2, B7-H3, B7-H4, B7-H6, B7-H7, BTLA/CD272, CD28, CTLA-4, Gi24/VISTA/B7-H5, ICOS/CD278, PD-1, PD-L2/B7-DC, and PDCD6); members of the TNF superfamily (e.g., 4-1BB/TNFRSF9/CD137, 4-1BB Ligand/TNFSF9, BAFF/-BLyS/TNFSF13B, BAFF R/TNFRSF13C, CD27/TNFRSF7, CD27 Ligand/TNFSF7, CD30/TNFRSF8, CD30 Ligand/TNFSF8, CD40/TNFRSF5, CD40/TNFRSF5, CD40 Ligand/TNFSF5, DR3/TNFRSF25, GITR/TNFRSF18, GITR Ligand/TNFSF18, HVEM/TNFRSF14, LIGHT/TNFSF14, Lymphotoxin-alpha/TNF-beta, OX40/TNFRSF4, OX40 Ligand/TNFSF4, RELT/TNFRSF19L, TACI/TNFRSF13B, TL1A/TNFSF15, TNF-alpha, and TNF RII/TNFRSF1B); members of the SLAM family (*e.g.,* 2B4/CD244/SLAMF4, BLAME/SLAMF8, CD2, CD2F-10/SLAMF9, CD48/SLAMF2, CD58/LFA-3, CD84/SLAMF5, CD229/SLAMF3, CRACC/SLAMF7, NTB-A/SLAMF6, and SLAM/CD150); and any other co-stimulatory molecules, such as CD2, CD7, CD53, CD82/Kai-1, CD90/Thy1, CD96, CD160, CD200, CD300a/LMIR1, HLA Class I, HLA-DR, Integrin alpha 4/CD49d, Integrin alpha 4 beta 1, Integrin alpha 4 beta 7/LPAM-1, LAG-3, TCL1A, TCL1B, CRTAM, DAP12, Dectin-1/CLEC7A, DPPIV/CD26, EphB6, TIM-1/KIM-1/HAVCR, TIM-4, TSLP, TSLP R, lymphocyte function associated antigen-1 (LFA-1), and NKG2C. In some embodiments, the co-stimulatory signaling domain is of 4-1BB, CD28, OX40, ICOS, CD27, GITR, HVEM, TIM1, LFA1(CD11a) or CD2, or any variant thereof.

Also within the scope of the present disclosure are variants of any of the co-stimulatory signaling domains described herein, such that the co-stimulatory signaling domain is capable of modulating the immune response of the immune cell. In some embodiments, the co-stimulatory signaling domains comprises up to 10 amino acid residue mutations (*e.g.,* 1, 2, 3, 4, 5, or 8) such as amino acid substitutions, deletions, or additions as compared to a wild-type counterpart. Such co-stimulatory signaling domains comprising one or more amino acid variations (*e.g.,* amino acid substitutions, deletions, or additions) may be referred to as variants.

Mutation of amino acid residues of the co-stimulatory signaling domain may result in an increase in signaling transduction and enhanced stimulation of immune responses relative to co-stimulatory signaling domains that do not comprise the mutation. Mutation of amino acid residues of the co-stimulatory signaling domain may result in a decrease in signaling transduction and reduced stimulation of immune responses relative to co-stimulatory signaling domains that do not comprise the mutation. For example, mutation of residues 186 and 187 of the native CD28 amino acid sequence may result in an increase in co-stimulatory activity and induction of immune responses by the co-stimulatory domain of the ACTR polypeptide. In some embodiments, the mutations are substitution of a lysine at each of positions 186 and 187 with a glycine residue of the CD28 co-stimulatory domain, referred to as a $CD28_{LL \rightarrow GG}$ variant. Additional mutations that can be made in co-stimulatory signaling domains that may enhance or reduce co-stimulatory activity of the domain will be evident to one of ordinary skill in the art. In some embodiments, the co-stimulatory signaling domain is of 4-1BB, CD28, OX40, or $CD28_{LL \rightarrow GG}$ variant.

In some embodiments, the chimeric polypeptides may contain a single co-stimulatory domain such as, for example, a CD27 co-stimulatory domain, a CD28 co-stimulatory domain, a 4-1BB co-stimulatory domain, an ICOS co-stimulatory domain, or an OX40 co-stimulatory domain.

In some embodiments, the chimeric polypeptides may comprise more than one co-stimulatory signaling domain (*e.g.,* 2, 3, or more). In some embodiments, the chimeric polypeptide comprises two or more of the same co-stimulatory signaling domains, for example, two copies of the co-stimulatory signaling domain of CD28. In some embodiments, the chimeric polypeptide comprises two or more co-stimulatory signaling domains from different co-stimulatory proteins, such as any two or more co-stimulatory proteins described herein. Selection of the type(s) of co-stimulatory signaling domains may be based on factors such as the type of host cells to be used with the chimeric receptor polypeptides (*e.g.,* T cells or NK cells) and the desired immune effector function. In some embodiments, the chimeric receptor polypeptide comprises two co-stimulatory signaling domains, for example, two copies of the co-stimulatory signaling domain of CD28. In some embodiments, the chimeric receptor polypeptide may comprise two or more co-stimulatory signaling domains from different co-stimulatory receptors, such as any two or more co-stimulatory receptors described herein, for example, CD28 and 4-1BB, CD28 and CD27, CD28 and ICOS, $CD28_{LL \to GG}$ variant and 4-1BB, CD28 and OX40, or $CD28_{LL \to GG}$ variant and OX40. In some embodiments, the two co-stimulatory signaling domains are CD28 and 4-1BB. In some embodiments, the two co-stimulatory signaling domains are $CD28_{LL \to GG}$ variant and 4-1BB. In some embodiments, the two co-stimulatory signaling domains are CD28 and OX40. In some embodiments, the two co-stimulatory signaling domains are $CD28_{LL \to GG}$ variant and OX40. In some embodiments, the chimeric receptor polypeptides described herein may contain a combination of a CD28 and ICOSL. In some embodiments, the chimeric receptor polypeptide described herein may contain a combination of CD28 and CD27. In certain embodiments, the 4-1BB co-stimulatory domain is located N-terminal to the CD28 or $CD28_{LL \to GG}$ variant co-stimulatory signaling domain.

In some embodiments, the chimeric receptor polypeptides described herein do not comprise a co-stimulatory signaling domain.

### D. Cytoplasmic signaling domain

Any cytoplasmic signaling domain can be used to create the chimeric receptor polypeptides described herein (e.g., ACTR polypeptides or CAR polypeptides). Such a cytoplasmic domain may be any signaling domain involved in triggering cell signaling (primary signaling) that leads to immune cell proliferation and/or activation. The cytoplasmic signaling domain as described herein is not a co-stimulatory signaling domain, which, as known in the art, relays a co-stimulatory or secondary signal for fully activating immune cells.

The cytoplasmic domain described herein may comprise an immunoreceptor tyrosine-based activation motif (ITAM) domain or may be ITAM free. An "ITAM," as used herein, is a conserved protein motif that is generally present in the tail portion of signaling molecules expressed in many immune cells. The motif may comprises two repeats of the amino acid sequence YxxL/I separated by 6-8 amino acids, wherein each x is independently any amino acid, producing the conserved motif $YxxL/Ix_{(6-8)}YxxL/I$. ITAMs within signaling molecules are important for signal transduction within the cell, which is mediated at least in part by phosphorylation of tyrosine residues in the ITAM following activation of the signaling molecule. ITAMs may also function as docking sites for other proteins involved in signaling pathways.

In some examples, the cytoplasmic signaling domain is of CD3ζ or FcεR1γ. In other examples, cytoplasmic signaling domain is not derived from human CD3ζ. In yet other examples, the cytoplasmic signaling domain is not derived from an Fc receptor, when the extracellular Fc-binding domain of the same chimeric receptor polypeptide is derived from CD16A.

In one specific embodiment, several signaling domains can be fused together for additive or synergistic effect. Non-limiting examples of useful additional signaling domains include part or all of one or more of TCR Zeta chain, CD28, OX40/CD134, 4-1BB/CD137, FcεRIy, ICOS/CD278, IL2R-beta/CD122, IL-2R-gamma/CD132, and CD40.

In other embodiments, the cytoplasmic signaling domain described herein is free of the ITAM motif. Examples include, but are not limited to, the cytoplasmic signaling domain of Jak/STAT, Toll-interleukin receptor (TIR), and tyrosine kinase.

### E. Hinge domain

In some embodiments, the chimeric receptor polypeptides such as ACTR polypeptides or CAR polypeptides described herein further comprise a hinge domain that is located between the extracellular ligand-binding domain and the transmembrane domain. A hinge domain is an amino acid segment that is generally found between two domains of a protein and may allow for flexibility of the protein and movement of one or both of the domains relative to one another. Any amino acid sequence that provides such flexibility and movement of the extracellular ligand-binding domain of an Fc receptor relative to the transmembrane domain of the chimeric receptor polypeptide can be used.

Hinge domains of any protein known in the art to comprise a hinge domain are compatible for use in the chimeric receptor polypeptides described herein. In some embodiments, the hinge domain is at least a portion of a hinge domain of a naturally occurring protein and confers flexibility to the chimeric receptor polypeptide. In some embodiments, the hinge domain is of CD8. In some embodiments, the hinge domain is a portion of the hinge domain of CD8, *e.g.,* a fragment containing at least 15 (*e.g.,* 20, 25, 30, 35, or 40) consecutive amino acids of the hinge domain of CD8. The hinge domain and/or the transmembrane domain may be linked to additional amino acids (*e.g.,* 15 aa, 10-aa, 8-aa, 6-aa, or 4-aa) at the N-terminal portion, at the C-terminal portion, or both. Examples can be found, *e.g.,* in Ying et al., Nature Medicine, 25(6):947-953 (2019).

In some embodiments, the hinge domain is of CD28. In some embodiments, the hinge domain is a portion of the hinge domain of CD28, *e.g.,* a fragment containing at least 15 (*e.g.,* 20, 25, 30, 35, or 40) consecutive amino acids of the hinge domain of CD28.

In some embodiments, the hinge domain is of CD16A receptor, for example, the whole hinge domain of a CD16A receptor or a portion thereof, which may consists of up to 40 consecutive amino acid residues of the CD16A receptor (*e.g.,* 20, 25, 30, 35, or 40). Such a chimeric receptor polypeptide (*e.g.,* an ACTR polypeptide) may contain no hinge domain from a different receptor (a non-CD16A receptor).

Hinge domains of antibodies, such as an IgG, IgA, IgM, IgE, or IgD antibodies, are also compatible for use in the chimeric polypeptides described herein. In some embodiments, the hinge domain is the hinge domain that joins the constant domains CH1 and CH2 of an antibody. In some embodiments, the hinge domain is of an antibody and comprises the hinge domain of the antibody and one or more constant regions of the antibody. In some embodiments, the hinge domain comprises the hinge domain of an antibody and the CH3 constant region of the antibody. In some embodiments, the hinge domain comprises the hinge domain of an antibody and the CH2 and CH3 constant regions of the antibody. In some embodiments, the antibody is an IgG, IgA, IgM, IgE, or IgD antibody. In some embodiments, the antibody is an IgG antibody. In some embodiments, the antibody is an IgG1, IgG2, IgG3, or IgG4 antibody. In some embodiments, the hinge region comprises the hinge region and the CH2 and CH3 constant regions of an IgG1 antibody. In some embodiments, the hinge region comprises the hinge region and the CH3 constant region of an IgG1 antibody.

Non-naturally occurring peptides may also be used as hinge domains for the chimeric receptor polypeptides described herein. In some embodiments, the hinge domain between the C-terminus of the extracellular target binding domain and the N-terminus of the transmembrane domain is a peptide linker, such as a $(Gly_xSer)_n$ linker, wherein x and n, independently can be an integer between 3 and 12, including 3, 4, 5, 6, 7, 8, 9, 10, 11, 12, or more. In some embodiments, the hinge domain is $(Gly_4Ser)_n$ (SEQ ID NO: 93), wherein n can be an integer between 3 and 60, including 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, or 60. In certain embodiments, n can be an integer greater than 60. In some embodiments, the hinge domain is $(Gly_4Ser)_3$ (SEQ ID NO: 94). In some embodiments, the hinge domain is $(Gly_4Ser)_6$ (SEQ ID NO: 95). In some embodiments, the hinge domain is $(Gly_4Ser)_9$ (SEQ ID NO: 96). In some embodiments, the hinge domain is $(Gly_4Ser)_{12}$ (SEQ ID NO: 97). In some embodiments, the hinge domain is $(Gly_4Ser)_{15}$ (SEQ ID NO: 98). In some embodiments, the hinge domain is $(Gly_4Ser)_{30}$ (SEQ ID NO: 99). In some embodiments, the hinge domain is $(Gly_4Ser)_{45}$ (SEQ ID NO: 100). In some embodiments, the hinge domain is $(Gly_4Ser)_{60}$ (SEQ ID NO: 101).

In other embodiments, the hinge domain is an extended recombinant polypeptide (XTEN), which is an unstructured polypeptide consisting of hydrophilic residues of varying lengths (*e.g.,* 10-80 amino acid residues). Amino acid sequences of XTEN peptides will be evident to one of skill in the art and can be found, for example, in U.S. Patent No. 8,673,860. In some embodiments, the hinge domain is an XTEN peptide and comprises 60 amino acids. In some embodiments, the hinge domain is an XTEN peptide and comprises 30 amino acids. In some embodiments, the hinge domain is an XTEN peptide and comprises 45 amino acids. In some embodiments, the hinge domain is an XTEN peptide and comprises 15 amino acids.

Any of the hinge domains used for making the chimeric receptor polypeptide as described herein may contain up to 250 amino acid residues. In some instances, the chimeric receptor polypeptide may contain a relatively long hinge domain, for example, containing 150-250 amino acid residues (*e.g.,* 150-180 amino acid residues, 180-200 amino acid residues, or 200-250 amino acid residues). In other instances, the chimeric receptor polypeptide may contain a medium sized hinge domain, which may contain 60-150 amino acid residues (*e.g.,* 60-80, 80-100, 100-120, or 120-150 amino acid residues). Alternatively, the chimeric receptor polypeptide may contain a short hinge domain, which may contain less than 60 amino acid residues (*e.g.,* 1-30 amino acids or 31-60 amino acids). In some embodiments, a chimeric receptor polypeptide (e.g., an ACTR polypeptide) described herein contains no hinge domain or no hinge domain from a non-CD16A receptor.

### F. *Signal peptide*

In some embodiments, the chimeric receptor polypeptide (*e.g.,* ACTR polypeptide or CAR polypeptide) may also comprise a signal peptide (also known as a signal sequence) at the N-terminus of the polypeptide. In general, signal sequences are peptide sequences that target a polypeptide to the desired site in a cell. In some embodiments, the signal sequence targets the chimeric receptor polypeptide to the secretory pathway of the cell and will allow for integration and anchoring of the chimeric receptor polypeptide into the lipid bilayer. Signal sequences including signal sequences of naturally occurring proteins or synthetic, non-naturally occurring signal sequences that are compatible for use in the chimeric receptor polypeptides described herein will be evident to one of skill in the art. In some embodiments, the signal sequence from CD8α. In some embodiments, the signal sequence is from CD28. In other embodiments, the signal sequence is from the murine kappa chain. In yet other embodiments, the signal sequence is from CD16.

### G. *Examples of ACTR polypeptides*

Exemplary ACTR constructs for use with the methods and compositions described herein may be found, for example, in the instant description and figures or may be found in PCT Patent Publication No.: WO2016040441A1, WO2017/161333, and PCT Application No.: PCT/US2018/015999. The ACTR polypeptides described herein may comprise a CD16A extracellular domain with binding affinity and specificity for the Fc portion of an IgG molecule, a transmembrane domain, and a CD3ζ cytoplasmic signaling domain. In some embodiments, the ACTR polypeptides may further include one or more co-stimulatory signaling domains, one of which may be a CD28 co-stimulatory signaling domain or a 4-1BB co-stimulatory signaling domain. The ACTR polypeptides are configured such that, when expressed on a host cell, the extracellular ligand-binding domain is located extracellularly for binding to a target molecule and the CD3ζ cytoplasmic signaling domain. The co-stimulatory signaling domain may be located in the cytoplasm for triggering activation and/or effector signaling.

In some embodiments, an ACTR polypeptide as described herein may comprise, from N-terminus to C-terminus, the Fc binding domain such as a CD16A extracellular domain, the transmembrane domain, the optional one or more co-stimulatory domains (*e.g.,* a CD28 co-stimulatory domain, a 4-1BB co-stimulatory signaling domain, an OX40 co-stimulatory signaling domain, a CD27 co-stimulatory signaling domain, or an ICOS co-stimulatory signaling domain), and the CD3ζ cytoplasmic signaling domain.

Alternatively or in addition, the ACTR polypeptides described herein may contain two or more co-stimulatory signaling domains, which may link to each other or be separated by the cytoplasmic signaling domain. The extracellular Fc binder, transmembrane domain, optional co-stimulatory signaling domain(s), and cytoplasmic signaling domain in an ACTR polypeptide may be linked to each other directly, or via a peptide linker. In some embodiments, any of the ACTR polypeptides described herein may comprise a signal sequence at the N-terminus.

Table 4 provides exemplary ACTR polypeptides described herein. These exemplary constructs have, from N-terminus to C-terminus in order, the signal sequence, the Fc binding domain (*e.g.,* an extracellular domain of an Fc receptor), the hinge domain, and the transmembrane, while the positions of the optional co-stimulatory domain and the cytoplasmic signaling domain can be switched.

### Table 4: Exemplary Components of ACTR polypeptides.

| Exemplary AA Sequence (SEQ ID NO) | Signal Sequence | Extracellular domain of Fc receptor | Hinge domain | Transmembrane domain | Co-stimulatory domain | Cytoplasmic Signaling domain |
|---|---|---|---|---|---|---|
| 1 | CD8α | CD16A-V158 | CD8α | CD8α | 4-1BB (CD 137) | CD3ζ |
| 2 | CD8α | CD16A-V158 | CD8α | 4-1BB (CD137) | 4-1BB (CD 137) | CD3ζ |
| 3 | CD8α | CD16A-V158 | CD8α | CD28 | 4-1BB (CD 137) | CD3ζ |
| 4 | CD8α | CD16A-V158 | CD8α | CD34 | 4-1BB (CD 137) | CD3ζ |
| 5 | CD8α | CD16A-V158 | CD8α | Designed hydrophobic TM domain | 4-1BB (CD 137) | CD3ζ |
| 6 | CD8α | CD32A | CD8α | CD8α | 4-1BB (CD 137) | CD3ζ |
| 7 | CD8α | CD16A-V158 | CD8α | CD8α | CD28 | cD3ζ |
| 8 | CD8α | CD16A-V158 | CD8α | CD8α | OX40 (CD 134) | cD3ζ |
| 9 | CD8α | CD16A-V158 | CD8α | CD8α | CD28 + 4-1BB | CD3ζ |
| 10 | CD8α | CD16A-V158 | None | CD8α | 4-1BB (CD 137) | CD3ζ |
| 11 | CD8α | CD16A-V158 | XTEN | CD8α | 4-1BB (CD 137) | CD3ζ |
| 12 | CD8α | CD16A-V158 | CD8α | CD8α | CD28 LL to GG mutant | CD3ζ |

(continued)

| Exemplary AA Sequence (SEQ ID NO) | Signal Sequence | Extracellular domain of Fc receptor | Hinge domain | Transmembrane domain | Co-stimulatory domain | Cytoplasmic Signaling domain |
|---|---|---|---|---|---|---|
| 13 | CD8α | CD16A-V158 | CD8α | CD8α | CD28 LL to GG mutant + 4-1BB | CD3ζ |
| 14 | CD8α | CD16A-V158 | CD8α | CD4 | 4-1BB (CD 137) | CD3ζ |
| 15 | CD8α | CD16A-V158 | CD8α | CD4 | CD28 LL to GG mutant + 4-1BB | CD3ζ |
| 16 | CD8α | CD16A-V158 | CD8α | FcεRIγ | 4-1BB (CD 137) | CD3ζ |
| 17 | CD8α | CD16A-V158 | CD8α | Designed hydro-phobic TM do-main, predicted dimerization | 4-1BB (CD 137) | CD3ζ |
| 18 | CD8α | CD16A-V158 | CD8α | CD8β | 4-1BB (CD 137) | CD3ζ |
| 19 | CD8α | CD16A-V158 | CD8α | C16α | 4-1BB (CD 137) | CD3ζ |
| 20 | CD8α | CD16A-V158 | CD8α | OX40 (CD134) | 4-1BB (CD 137) | CD3ζ |
| 21 | CD8α | CD16A-V158 | CD8α | CD3ζ | 4-1BB (CD 137) | CD3ζ |
| 22 | CD8α | CD16A-V158 | CD8α | CD3ε | 4-1BB (CD 137) | CD3ζ |
| 23 | CD8α | CD16A-V158 | CD8α | CD3γ | 4-1BB (CD 137) | CD3ζ |
| 24 | CD8α | CD16A-V158 | CD8α | CD3δ | 4-1BB (CD 137) | CD3ζ |
| 25 | CD8α | CD16A-V158 | CD8α | TCR-α | 4-1BB (CD 137) | CD3ζ |
| 26 | CD8α | CD16A-V158 | CD8α | CD32 | 4-1BB (CD 137) | CD3ζ |
| 27 | CD8α | CD16A-V158 | CD8α | CD64 | 4-1BB (CD 137) | CD3ζ |
| 28 | CD8α | CD16A-V158 | CD8α | VEGFR2 | 4-1BB (CD 137) | CD3ζ |
| 29 | CD8α | CD16A-V158 | CD8α | FAS | 4-1BB (CD 137) | CD3ζ |
| 30 | CD8α | CD16A-V158 | CD8α | FGFR2B | 4-1BB (CD 137) | CD3ζ |
| 31 | CD8α | CD16A-F158 | CD8α | CD8α | 4-1BB (CD 137) | CD3ζ |
| 32 | CD8α | CD64A | CD8α | CD8α | 4-1BB (CD 137) | CD3ζ |
| 33 | CD8α | CD16A-V158 | IgG1 (hinge-CH2-CH3) | CD8α | 4-1BB (CD 137) | CD3ζ |
| 34 | CD8α | CD16A-V158 | IgG1 (hinge-CH3) | CD8α | 4-1BB (CD 137) | CD3ζ |
| 35 | CD8α | CD16A-V158 | IgG1 (hinge) | CD8α | 4-1BB (CD 137) | CD3ζ |
| 36 | CD8α | CD16A-V158 | CD8-alpha fragment amino acids) | CD8α | 4-1BB (CD 137) | CD3ζ |
| 37 | CD8α | CD16A-V158 | CD8-alpha fragment 2 (15 amino acids) | CD8α | 4-1BB (CD 137) | CD3ζ |
| 38 | CD8α | CD16A-V158 | (Gly4Ser )x3 (60 amino acids) | CD8α | 4-1BB (CD 137) | CD3ζ |

(continued)

| Exemplary AA Sequence (SEQ ID NO) | Signal Sequence | Extracellular domain of Fc receptor | Hinge domain | Transmembrane domain | Co-stimulatory domain | Cytoplasmic Signaling domain |
|---|---|---|---|---|---|---|
| 39 | CD8α | CD16A-V158 | (Gly4Ser )x6 (45 amino acids) | CD8α | 4-1BB (CD 137) | CD3ζ |
| 40 | CD8α | CD16A-V158 | (Gly4Ser )x9 (30 amino acids) | CD8α | 4-1BB (CD 137) | CD3ζ |
| 41 | CD8α | CD16A-V158 | (Gly4Ser )x12 (15 amino acids) | CD8α | 4-1BB (CD 137) | CD3ζ |
| 42 | CD8α | CD16A-V158 | XTEN (60 amino acids) | CD8α | 4-1BB (CD 137) | CD3ζ |
| 43 | CD8α | CD16A-V158 | XTEN (30 amino acids) | CD8α | 4-1BB (CD 137) | CD3ζ |
| 44 | CD8α | CD16A-V158 | XTEN (15 amino acids) | CD8α | 4-1BB (CD 137) | CD3ζ |
| 45 | CD28 | CD16A-V158 | CD8α | CD8α | 4-1BB (CD 137) | CD3ζ |
| 46 | Murine kappa chain | CD16A-V158 | CD8α | CD8α | 4-1BB (CD 137) | CD3ζ |
| 47 | CD16 | CD16A-V158 | CD8α | CD8α | 4-1BB (CD 137) | CD3ζ |
| 48 | CD8α | CD16A-V158 | CD8α | CD8α | ICOS | cD3ζ |
| 49 | CD8α | CD16A-VI58 | CD8α | CD8α | CD27 | cD3ζ |
| 50 | CD8α | CD16A-VI58 | CD8α | CD8α | GITR | cD3ζ |
| 51 | CD8α | CD16A-VI58 | CD8α | CD8α | HVEM | CD3ζ |
| 52 | CD8α | CD16A-V158 | CD8α | CD8α | TIM1 | CD3ζ |
| 53 | CD8α | CD16A-V158 | CD8α | CD8α | LFA1 (CD11a) | CD3ζ |
| 54 | CD8α | CD16A-V158 | CD8α | CD8α | CD2 | CD3ζ |
| 55 | CD8α | CD16A-V158 | CD8α | FcεR1γ | 4-1BB (CD137) | FcεR1γ |
| 56 | CD8α | CD16A-V158 | CD8α | CD8α | 4-1BB (CD137) | FcεR1γ |
| 57 | CD8α | CD16A-V158 | CD28 (e.g., 39aa) | CD28 | CD28 | CD3ζ |
| 58 | CD8α | CD16A-V158 | none | CD8 | CD28 | cD3ζ |
| 59 | CD8α | CD16A-V158 | CD8 | CD8 | CD28 + CD27 | cD3ζ |
| 60 | CD8α | CD16A-V158 | CD8 | CD8 | CD28 + OX40 | cD3ζ |
| 61 | CD8α | CD16A-V158 | CD8 | CD8 | 4-1BB + CD28 | CD3ζ |
| 62 | CD8α | CD16A-V158 | CD28 | CD28 | CD28 + 4-1BB | CD3ζ |
| 63 | CD8α | CD16A-V158 | CD28 | CD28 | 4-1BB | cD3ζ |

(continued)

| Exemplary AA Sequence (SEQ ID NO) | Signal Sequence | Extracellular domain of Fc receptor | Hinge domain | Transmembrane domain | Co-stimulatory domain | Cytoplasmic Signaling domain |
|---|---|---|---|---|---|---|
| 64 | CD8α | CD16A-V158 | CD8 | CD8 | CD27 | cD3ζ |
| 65 | CD8α | CD16A-V158 | CD8 | CD8 | CD28 | cD3ζ |
| 66 | CD8α | CD16A-V158 | CD8 | CD8 | ICOS | cD3ζ |
| 67 | CD8α | CD16A-V158 | CD8 | CD8 | OX40 | cD3ζ |
| 68 | CD8α | CD16A-V158 | CD8 | CD8 | CD28 and ICOS | cD3ζ |
| 69 | CD8α | CD16A-V158 | none | CD8 | 4-1BB | cD3ζ |
| 70 | CD8α | CD16A-V158 | none | CD8 | CD27 | cD3ζ |
| 71 | CD8α | CD16A-V158 | none | CD8 | ICOS | cD3ζ |
| 72 | CD8α | CD16A-V158 | none | CD8 | OX40 | cD3ζ |
| 73 | CD8α | CD16A-V158 | none | CD8 + 4aa | 4-1BB | cD3ζ |
| 74 | CD8α | CD16A-V158 | none | CD8 + 4aa | CD28 | cD3ζ |
| 75 | CD8α | CD16A-V158 | CD8 | CD28 | CD28 | cD3ζ |
| 76 | CD8α | CD16A-V158 | CD28 (26aa) | CD28 | CD28 | cD3ζ |
| 77 | CD8α | CD16A-V158 | CD28 (16aa) | CD28 | CD28 | CD3ζ |
| 78 | CD8α | CD16A-V158 | none | CD28 | CD28 | cD3ζ |
| 79 | CD8α | CD16A-V158 | CD8 | CD8 | 41BB | cD3ζ |
| 80 | CD8α | CD16A-V158 | CD28 (39 aa) | CD8 | CD28 | cD3ζ |

Amino acid sequences of the example ACTR polypeptides are provided below (signal sequence italicized).

SEQ ID NO:1:

*MALPVTALLLPLALLLHAARP*GMRTEDLPKAVVFLEPQWYRVLEKDSVTLKCQGAYSPEDNSTQWFHNESLISSQ
ASSYFIDAATVDDSGEYRCQTNLSTLSDPVQLEVHIGWLLLQAPRWVFKEEDPIHLRCHSWKNTALHKVTYLQNG
KGRKYFHHNSDFYIPKATLKDSGSYFCRGLVGSKNVSSETVNITITQGLAVSTISSFFPPGYQTTTPAPRPPTPA
PTIASQPLSLRPEACRPAAGGAVHTRGLDFACDIYIWAPLAGTCGVLLLSLVITLYCKRGRKKLLYIFKQPFMRP
VQTTQEEDGCSCRFPEEEEGGCELRVKFSRSADAPAYQQGQNQLYNELNLGRREEYDVLDKRRGRDPEMGGKPRR
KNPQEGLYNELQKDKMAEAYSEIGMKGERRRGKGHDGLYQGLSTATKDTYDALHMQALPPR

SEQ ID NO:2:

*MALPVTALLLPLALLLHAARP*GMRTEDLPKAVVFLEPQWYRVLEKDSVTLKCQGAYSPEDNSTQWFHNESLI
SSQASSYFIDAATVDDSGEYRCQTNLSTLSDPVQLEVHIGWLLLQAPRWVFKEEDPIHLRCHSWKNTALHKV
TYLQNGKGRKYFHHNSDFYIPKATLKDSGSYFCRGLVGSKNVSSETVNITITQGLAVSTISSFFPPGYQTTT
PAPRPPTPAPTIASQPLSLRPEACRPAAGGAVHTRGLDFACDIISFFLALTSTALLFLLFFLTLRFSVVKRG
KRGRKKLLYIFKQPFMRPVQTTQEEDGCSCRFPEEEEGGCELRVKFSRSADAPAYQQGQNQLYNELNLGRRE
EYDVLDKRRGRDPEMGGKPRRKNPQEGLYNELQKDKMAEAYSEIGMKGERRRGKGHDGLYQGLSTATKDTYD
ALHMQALPPR

SEQ ID NO:3:

*MALPVTALLLPLALLLHAARP*GMRTEDLPKAVVFLEPQWYRVLEKDSVTLKCQGAYSPEDNSTQWFHNESLI
SSQASSYFIDAATVDDSGEYRCQTNLSTLSDPVQLEVHIGWLLLQAPRWVFKEEDPIHLRCHSWKNTALHKV
TYLQNGKGRKYFHHNSDFYIPKATLKDSGSYFCRGLVGSKNVSSETVNITITQGLAVSTISSFFPPGYQTTT
PAPRPPTPAPTIASQPLSLRPEACRPAAGGAVHTRGLDFACDFWVLVVGGVLACYSLLVTVAFIIFWVRSK
KRGRKKLLYIFKQPFMRPVQTTQEEDGCSCRFPEEEEGGCELRVKFSRSADAPAYQQGQNQLYNELNLGRRE
EYDVLDKRRGRDPEMGGKPRRKNPQEGLYNELQKDKMAEAYSEIGMKGERRRGKGHDGLYQGLSTATKDTYD
ALHMQALPPR

**SEQ ID NO:4:**

*MALPVTALLLPLALLLHAARP*GMRTEDLPKAVVFLEPQWYRVLEKDSVTLKCQGAYSPEDNSTQWFHNESLI
SSQASSYFIDAATVDDSGEYRCQTNLSTLSDPVQLEVHIGWLLLQAPRWVFKEEDPIHLRCHSWKNTALHKV
TYLQNGKGRKYFHHNSDFYIPKATLKDSGSYFCRGLVGSKNVSSETVNITITQGLAVSTISSFFPPGYQTTT
PAPRPPTPAPTIASQPLSLRPEACRPAAGGAVHTRGLDFACDLIALVTSGALLAVLGITGYFLMNRKRGRKK

LLYIFKQPFMRPVQTTQEEDGCSCRFPEEEEGGCELRVKFSRSADAPAYQQGQNQLYNELNLGRREEYDVLD
KRRGRDPEMGGKPRRKNPQEGLYNELQKDKMAEAYSEIGMKGERRRGKGHDGLYQGLSTATKDTYDALHMQA
LPPR

**SEQ ID NO:5:**

*MALPVTALLLPLALLLHAARP*GMRTEDLPKAVVFLEPQWYRVLEKDSVTLKCQGAYSPEDNSTQWFHNESLI
SSQASSYFIDAATVDDSGEYRCQTNLSTLSDPVQLEVHIGWLLLQAPRWVFKEEDPIHLRCHSWKNTALHKV
TYLQNGKGRKYFHHNSDFYIPKATLKDSGSYFCRGLVGSKNVSSETVNITITQGLAVSTISSFFPPGYQTTT
PAPRPPTPAPTIASQPLSLRPEACRPAAGGAVHTRGLDFACDLLAALLALLAALLALLAALLARSKKRGRKK
LLYIFKQPFMRPVQTTQEEDGCSCRFPEEEEGGCELRVKFSRSADAPAYQQGQNQLYNELNLGRREEYDVLD
KRRGRDPEMGGKPRRKNPQEGLYNELQKDKMAEAYSEIGMKGERRRGKGHDGLYQGLSTATKDTYDALHMQA
LPPR

**SEQ ID NO:6:**

*MALPVTALLLPLALLLHAARP*QAAAPPKAVLKLEPPWINVLQEDSVTLTCQGARSPESDSIQWFHNGNLIPT
HTQPSYRFKANNNDSGEYTCQTGQTSLSDPVHLTVLSEWLVLQTPHLEFQEGETIMLRCHSWKDKPLVKVTF
FQNGKSQKFSHLDPTFSIPQANHSHSGDYHCTGNIGYTLFSSKPVTITVQVPSMGSSSPMGTTTPAPRPPTP
APTIASQPLSLRPEACRPAAGGAVHTRGLDFACDIYIWAPLAGTCGVLLLSLVITLYCKRGRKKLLYIFKQP
FMRPVQTTQEEDGCSCRFPEEEEGGCELRVKFSRSADAPAYQQGQNQLYNELNLGRREEYDVLDKRRGRDPE
MGGKPRRKNPQEGLYNELQKDKMAEAYSEIGMKGERRRGKGHDGLYQGLSTATKDTYDALHMQALPPR

**SEQ ID NO:7:**

*MALPVTALLLPLALLLHAARP*GMRTEDLPKAVVFLEPQWYRVLEKDSVTLKCQGAYSPEDNSTQWFHNESLI
SSQASSYFIDAATVDDSGEYRCQTNLSTLSDPVQLEVHIGWLLLQAPRWVFKEEDPIHLRCHSWKNTALHKV
TYLQNGKGRKYFHHNSDFYIPKATLKDSGSYFCRGLVGSKNVSSETVNITITQGLAVSTISSFFPPGYQTTT
PAPRPPTPAPTIASQPLSLRPEACRPAAGGAVHTRGLDFACDIYIWAPLAGTCGVLLLSLVITLYCRSKRSR
LLHSDYMNMTPRRPGPTRKHYQPYAPPRDFAAYRSRVKFSRSADAPAYQQGQNQLYNELNLGRREEYDVLDK
RRGRDPEMGGKPRRKNPQEGLYNELQKDKMAEAYSEIGMKGERRRGKGHDGLYQGLSTATKDTYDALHMQAL
PPR

**SEQ ID NO:8:**

*MALPVTALLLPLALLLHAARP*GMRTEDLPKAVVFLEPQWYRVLEKDSVTLKCQGAYSPEDNSTQWFHNESLI
SSQASSYFIDAATVDDSGEYRCQTNLSTLSDPVQLEVHIGWLLLQAPRWVFKEEDPIHLRCHSWKNTALHKV
TYLQNGKGRKYFHHNSDFYIPKATLKDSGSYFCRGLVGSKNVSSETVNITITQGLAVSTISSFFPPGYQTTT
PAPRPPTPAPTIASQPLSLRPEACRPAAGGAVHTRGLDFACDIYIWAPLAGTCGVLLLSLVITLYCALYLLR
RDQRLPPDAHKPPGGGSFRTPIQEEQADAHSTLAKIRVKFSRSADAPAYQQGQNQLYNELNLGRREEYDVLD
KRRGRDPEMGGKPRRKNPQEGLYNELQKDKMAEAYSEIGMKGERRRGKGHDGLYQGLSTATKDTYDALHMQA
LPPR

**SEQ ID NO:9:**

*MALPVTALLLPLALLLHAARP*GMRTEDLPKAVVFLEPQWYRVLEKDSVTLKCQGAYSPEDNSTQWFHNESLI
SSQASSYFIDAATVDDSGEYRCQTNLSTLSDPVQLEVHIGWLLLQAPRWVFKEEDPIHLRCHSWKNTALHKV
TYLQNGKGRKYFHHNSDFYIPKATLKDSGSYFCRGLVGSKNVSSETVNITITQGLAVSTISSFFPPGYQTTT
PAPRPPTPAPTIASQPLSLRPEACRPAAGGAVHTRGLDFACDIYIWAPLAGTCGVLLLSLVITLYCRSKRSR
LLHSDYMNMTPRRPGPTRKHYQPYAPPRDFAAYRSKRGRKKLLYIFKQPFMRPVQTTQEEDGCSCRFPEEEE
GGCELRVKFSRSADAPAYQQGQNQLYNELNLGRREEYDVLDKRRGRDPEMGGKPRRKNPQEGLYNELQKDKM
AEAYSEIGMKGERRRGKGHDGLYQGLSTATKDTYDALHMQALPPR

**SEQ ID NO:10:**

*MALPVTALLLPLALLLHAARP*GMRTEDLPKAVVFLEPQWYRVLEKDSVTLKCQGAYSPEDNSTQWFHNESLI
SSQASSYFIDAATVDDSGEYRCQTNLSTLSDPVQLEVHIGWLLLQAPRWVFKEEDPIHLRCHSWKNTALHKV
TYLQNGKGRKYFHHNSDFYIPKATLKDSGSYFCRGLVGSKNVSSETVNITITQGLAVSTISSFFPPGYQIYI
WAPLAGTCGVLLLSLVITLYCKRGRKKLLYIFKQPFMRPVQTTQEEDGCSCRFPEEEEGGCELRVKFSRSAD
APAYQQGQNQLYNELNLGRREEYDVLDKRRGRDPEMGGKPRRKNPQEGLYNELQKDKMAEAYSEIGMKGERR
RGKGHDGLYQGLSTATKDTYDALHMQALPPR

**SEQ ID NO:11:**

*MALPVTALLLPLALLLHAARP*GMRTEDLPKAVVFLEPQWYRVLEKDSVTLKCQGAYSPEDNSTQWFHNESLI
SSQASSYFIDAATVDDSGEYRCQTNLSTLSDPVQLEVHIGWLLLQAPRWVFKEEDPIHLRCHSWKNTALHKV
TYLQNGKGRKYFHHNSDFYIPKATLKDSGSYFCRGLVGSKNVSSETVNITITQGLAVSTISSFFPPGYQGGS
PAGSPTSTEEGTSESATPESGPGTSTEPSEGSAPGSPAGSPTIYIWAPLAGTCGVLLLSLVITLYCKRGRKK
LLYIFKQPFMRPVQTTQEEDGCSCRFPEEEEGGCELRVKFSRSADAPAYQQGQNQLYNELNLGRREEYDVLD
KRRGRDPEMGGKPRRKNPQEGLYNELQKDKMAEAYSEIGMKGERRRGKGHDGLYQGLSTATKDTYDALHMQA
LPPR

**SEQ ID NO:12:**

*MALPVTALLLPLALLLHAARP*GMRTEDLPKAVVFLEPQWYRVLEKDSVTLKCQGAYSPEDNSTQWFHNESLI
SSQASSYFIDAATVDDSGEYRCQTNLSTLSDPVQLEVHIGWLLLQAPRWVFKEEDPIHLRCHSWKNTALHKV
TYLQNGKGRKYFHHNSDFYIPKATLKDSGSYFCRGLVGSKNVSSETVNITITQGLAVSTISSFFPPGYQTTT
PAPRPPTPAPTIASQPLSLRPEACRPAAGGAVHTRGLDFACDIYIWAPLAGTCGVLLLSLVITLYCRSKRSR
GGHSDYMNMTPRRPGPTRKHYQPYAPPRDFAAYRSRVKFSRSADAPAYQQGQNQLYNELNLGRREEYDVLDK
RRGRDPEMGGKPRRKNPQEGLYNELQKDKMAEAYSEIGMKGERRRGKGHDGLYQGLSTATKDTYDALHMQAL
PPR

**SEQ ID NO:13:**

*MALPVTALLLPLALLLHAARP*GMRTEDLPKAVVFLEPQWYRVLEKDSVTLKCQGAYSPEDNSTQWFHNESLI
SSQASSYFIDAATVDDSGEYRCQTNLSTLSDPVQLEVHIGWLLLQAPRWVFKEEDPIHLRCHSWKNTALHKV
TYLQNGKGRKYFHHNSDFYIPKATLKDSGSYFCRGLVGSKNVSSETVNITITQGLAVSTISSFFPPGYQTTT
PAPRPPTPAPTIASQPLSLRPEACRPAAGGAVHTRGLDFACDIYIWAPLAGTCGVLLLSLVITLYCRSKRSR
GGHSDYMNMTPRRPGPTRKHYQPYAPPRDFAAYRSKRGRKKLLYIFKQPFMRPVQTTQEEDGCSCRFPEEEE
GGCELRVKFSRSADAPAYQQGQNQLYNELNLGRREEYDVLDKRRGRDPEMGGKPRRKNPQEGLYNELQKDKM
AEAYSEIGMKGERRRGKGHDGLYQGLSTATKDTYDALHMQALPPR

**SEQ ID NO:14:**

*MALPVTALLLPLALLLHAARP*GMRTEDLPKAVVFLEPQWYRVLEKDSVTLKCQGAYSPEDNSTQWFHNESLI
SSQASSYFIDAATVDDSGEYRCQTNLSTLSDPVQLEVHIGWLLLQAPRWVFKEEDPIHLRCHSWKNTALHKV
TYLQNGKGRKYFHHNSDFYIPKATLKDSGSYFCRGLVGSKNVSSETVNITITQGLAVSTISSFFPPGYQTTT
PAPRPPTPAPTIASQPLSLRPEACRPAAGGAVHTRGLDFACDMALIVLGGVAGLLLFIGLGIFFCVRKRGRK
KLLYIFKQPFMRPVQTTQEEDGCSCRFPEEEEGGCELRVKFSRSADAPAYQQGQNQLYNELNLGRREEYDVL
DKRRGRDPEMGGKPRRKNPQEGLYNELQKDKMAEAYSEIGMKGERRRGKGHDGLYQGLSTATKDTYDALHMQ
ALPPR

**SEQ ID NO:15:**

*MALPVTALLLPLALLLHAARP*GMRTEDLPKAVVFLEPQWYRVLEKDSVTLKCQGAYSPEDNSTQWFHNESLI
SSQASSYFIDAATVDDSGEYRCQTNLSTLSDPVQLEVHIGWLLLQAPRWVFKEEDPIHLRCHSWKNTALHKV
TYLQNGKGRKYFHHNSDFYIPKATLKDSGSYFCRGLVGSKNVSSETVNITITQGLAVSTISSFFPPGYQTTT
PAPRPPTPAPTIASQPLSLRPEACRPAAGGAVHTRGLDFACDMALIVLGGVAGLLLFIGLGIFFCVRRSKRS
RGGHSDYMNMTPRRPGPTRKHYQPYAPPRDFAAYRSRVKFSRSADAPAYQQGQNQLYNELNLGRREEYDVLD
KRRGRDPEMGGKPRRKNPQEGLYNELQKDKMAEAYSEIGMKGERRRGKGHDGLYQGLSTATKDTYDALHMQA
LPPR

**SEQ ID NO:16:**

*MALPVTALLLPLALLLHAARP*GMRTEDLPKAVVFLEPQWYRVLEKDSVTLKCQGAYSPEDNSTQWFHNESLI
SSQASSYFIDAATVDDSGEYRCQTNLSTLSDPVQLEVHIGWLLLQAPRWVFKEEDPIHLRCHSWKNTALHKV
TYLQNGKGRKYFHHNSDFYIPKATLKDSGSYFCRGLVGSKNVSSETVNITITQGLAVSTISSFFPPGYQTTT
PAPRPPTPAPTIASQPLSLRPEACRPAAGGAVHTRGLDFACDLCYILDAILFLYGIVLTLLYCRLKKRGRKK
LLYIFKQPFMRPVQTTQEEDGCSCRFPEEEEGGCELRVKFSRSADAPAYQQGQNQLYNELNLGRREEYDVLD
KRRGRDPEMGGKPRRKNPQEGLYNELQKDKMAEAYSEIGMKGERRRGKGHDGLYQGLSTATKDTYDALHMQA
LPPR

**SEQ ID NO:17:**

*MALPVTALLLPLALLLHAARP*GMRTEDLPKAVVFLEPQWYRVLEKDSVTLKCQGAYSPEDNSTQWFHNESLI
SSQASSYFIDAATVDDSGEYRCQTNLSTLSDPVQLEVHIGWLLLQAPRWVFKEEDPIHLRCHSWKNTALHKV
TYLQNGKGRKYFHHNSDFYIPKATLKDSGSYFCRGLVGSKNVSSETVNITITQGLAVSTISSFFPPGYQTTT
PAPRPPTPAPTIASQPLSLRPEACRPAAGGAVHTRGLDFACDLLLILLGVLAGVLATLAALLARSKKRGRKK
LLYIFKQPFMRPVQTTQEEDGCSCRFPEEEEGGCELRVKFSRSADAPAYQQGQNQLYNELNLGRREEYDVLD
KRRGRDPEMGGKPRRKNPQEGLYNELQKDKMAEAYSEIGMKGERRRGKGHDGLYQGLSTATKDTYDALHMQA
LPPR

**SEQ ID NO:18:**

*MALPVTALLLPLALLLHAARP*GMRTEDLPKAVVFLEPQWYRVLEKDSVTLKCQGAYSPEDNSTQWFHNESLI
SSQASSYFIDAATVDDSGEYRCQTNLSTLSDPVQLEVHIGWLLLQAPRWVFKEEDPIHLRCHSWKNTALHKV
TYLQNGKGRKYFHHNSDFYIPKATLKDSGSYFCRGLVGSKNVSSETVNITITQGLAVSTISSFFPPGYQTTT
PAPRPPTPAPTIASQPLSLRPEACRPAAGGAVHTRGLDFACDITLGLLVAGVLVLLVSLGVAIHLCKRGRKK
LLYIFKQPFMRPVQTTQEEDGCSCRFPEEEEGGCELRVKFSRSADAPAYQQGQNQLYNELNLGRREEYDVLD
KRRGRDPEMGGKPRRKNPQEGLYNELQKDKMAEAYSEIGMKGERRRGKGHDGLYQGLSTATKDTYDALHMQA
LPPR

**SEQ ID NO:19:**

*MALPVTALLLPLALLLHAARP*GMRTEDLPKAVVFLEPQWYRVLEKDSVTLKCQGAYSPEDNSTQWFHNESLI
SSQASSYFIDAATVDDSGEYRCQTNLSTLSDPVQLEVHIGWLLLQAPRWVFKEEDPIHLRCHSWKNTALHKV
TYLQNGKGRKYFHHNSDFYIPKATLKDSGSYFCRGLVGSKNVSSETVNITITQGLAVSTISSFFPPGYQTTT
PAPRPPTPAPTIASQPLSLRPEACRPAAGGAVHTRGLDFACDVSFCLVMVLLFAVDTGLYFSVKTNKRGRKK
LLYIFKQPFMRPVQTTQEEDGCSCRFPEEEEGGCELRVKFSRSADAPAYQQGQNQLYNELNLGRREEYDVLD
KRRGRDPEMGGKPRRKNPQEGLYNELQKDKMAEAYSEIGMKGERRRGKGHDGLYQGLSTATKDTYDALHMQA
LPPR

**SEQ ID NO:20:**

*MALPVTALLLPLALLLHAARP*GMRTEDLPKAVVFLEPQWYRVLEKDSVTLKCQGAYSPEDNSTQWFHNESLI
SSQASSYFIDAATVDDSGEYRCQTNLSTLSDPVQLEVHIGWLLLQAPRWVFKEEDPIHLRCHSWKNTALHKV
TYLQNGKGRKYFHHNSDFYIPKATLKDSGSYFCRGLVGSKNVSSETVNITITQGLAVSTISSFFPPGYQTTT
PAPRPPTPAPTIASQPLSLRPEACRPAAGGAVHTRGLDFACDVAAILGLGLVLGLLGPLAILLALYKRGRKK
LLYIFKQPFMRPVQTTQEEDGCSCRFPEEEEGGCELRVKFSRSADAPAYQQGQNQLYNELNLGRREEYDVLD
KRRGRDPEMGGKPRRKNPQEGLYNELQKDKMAEAYSEIGMKGERRRGKGHDGLYQGLSTATKDTYDALHMQA
LPPR

**SEQ ID NO:21:**

*MALPVTALLLPLALLLHAARP*GMRTEDLPKAVVFLEPQWYRVLEKDSVTLKCQGAYSPEDNSTQWFHNESLI
SSQASSYFIDAATVDDSGEYRCQTNLSTLSDPVQLEVHIGWLLLQAPRWVFKEEDPIHLRCHSWKNTALHKV
TYLQNGKGRKYFHHNSDFYIPKATLKDSGSYFCRGLVGSKNVSSETVNITITQGLAVSTISSFFPPGYQTTT
PAPRPPTPAPTIASQPLSLRPEACRPAAGGAVHTRGLDFACDLCYLLDGILFIYGVILTALFLRVKKRGRKK
LLYIFKQPFMRPVQTTQEEDGCSCRFPEEEEGGCELRVKFSRSADAPAYQQGQNQLYNELNLGRREEYDVLD
KRRGRDPEMGGKPRRKNPQEGLYNELQKDKMAEAYSEIGMKGERRRGKGHDGLYQGLSTATKDTYDALHMQA
LPPR

**SEQ ID NO:22:**

*MALPVTALLLPLALLLHAARP*GMRTEDLPKAVVFLEPQWYRVLEKDSVTLKCQGAYSPEDNSTQWFHNESLI
SSQASSYFIDAATVDDSGEYRCQTNLSTLSDPVQLEVHIGWLLLQAPRWVFKEEDPIHLRCHSWKNTALHKV
TYLQNGKGRKYFHHNSDFYIPKATLKDSGSYFCRGLVGSKNVSSETVNITITQGLAVSTISSFFPPGYQTTT
PAPRPPTPAPTIASQPLSLRPEACRPAAGGAVHTRGLDFACDVMSVATIVIVDICITGGLLLLVYYWSKNRK
RGRKKLLYIFKQPFMRPVQTTQEEDGCSCRFPEEEEGGCELRVKFSRSADAPAYQQGQNQLYNELNLGRREE
YDVLDKRRGRDPEMGGKPRRKNPQEGLYNELQKDKMAEAYSEIGMKGERRRGKGHDGLYQGLSTATKDTYDA
LHMQALPPR

**SEQ ID NO:23:**

*MALPVTALLLPLALLLHAARP*GMRTEDLPKAVVFLEPQWYRVLEKDSVTLKCQGAYSPEDNSTQWFHNESLI
SSQASSYFIDAATVDDSGEYRCQTNLSTLSDPVQLEVHIGWLLLQAPRWVFKEEDPIHLRCHSWKNTALHKV
TYLQNGKGRKYFHHNSDFYIPKATLKDSGSYFCRGLVGSKNVSSETVNITITQGLAVSTISSFFPPGYQTTT
PAPRPPTPAPTIASQPLSLRPEACRPAAGGAVHTRGLDFACDGFLFAEIVSIFVLAVGVYFIAGQDKRGRKK
LLYIFKQPFMRPVQTTQEEDGCSCRFPEEEEGGCELRVKFSRSADAPAYQQGQNQLYNELNLGRREEYDVLD
KRRGRDPEMGGKPRRKNPQEGLYNELQKDKMAEAYSEIGMKGERRRGKGHDGLYQGLSTATKDTYDALHMQA
LPPR

**SEQ ID NO:24:**

*MALPVTALLLPLALLLHAARP*GMRTEDLPKAVVFLEPQWYRVLEKDSVTLKCQGAYSPEDNSTQWFHNESLI
SSQASSYFIDAATVDDSGEYRCQTNLSTLSDPVQLEVHIGWLLLQAPRWVFKEEDPIHLRCHSWKNTALHKV
TYLQNGKGRKYFHHNSDFYIPKATLKDSGSYFCRGLVGSKNVSSETVNITITQGLAVSTISSFFPPGYQTTT
PAPRPPTPAPTIASQPLSLRPEACRPAAGGAVHTRGLDFACDGIIVTDVIATLLLALGVFCFAGHETKRGRK
KLLYIFKQPFMRPVQTTQEEDGCSCRFPEEEEGGCELRVKFSRSADAPAYQQGQNQLYNELNLGRREEYDVL
DKRRGRDPEMGGKPRRKNPQEGLYNELQKDKMAEAYSEIGMKGERRRGKGHDGLYQGLSTATKDTYDALHMQ
ALPPR

**SEQ ID NO:25:**

*MALPVTALLLPLALLLHAARP*GMRTEDLPKAVVFLEPQWYRVLEKDSVTLKCQGAYSPEDNSTQWFHNESLI
SSQASSYFIDAATVDDSGEYRCQTNLSTLSDPVQLEVHIGWLLLQAPRWVFKEEDPIHLRCHSWKNTALHKV
TYLQNGKGRKYFHHNSDFYIPKATLKDSGSYFCRGLVGSKNVSSETVNITITQGLAVSTISSFFPPGYQTTT
PAPRPPTPAPTIASQPLSLRPEACRPAAGGAVHTRGLDFACDVIGFRILLLKVAGFNLLMTLRLWKRGRKKL
LYIFKQPFMRPVQTTQEEDGCSCRFPEEEEGGCELRVKFSRSADAPAYQQGQNQLYNELNLGRREEYDVLDK
RRGRDPEMGGKPRRKNPQEGLYNELQKDKMAEAYSEIGMKGERRRGKGHDGLYQGLSTATKDTYDALHMQAL
PPR

**SEQ ID NO:26:**

*MALPVTALLLPLALLLHAARP*GMRTEDLPKAVVFLEPQWYRVLEKDSVTLKCQGAYSPEDNSTQWFHNESLI
SSQASSYFIDAATVDDSGEYRCQTNLSTLSDPVQLEVHIGWLLLQAPRWVFKEEDPIHLRCHSWKNTALHKV
TYLQNGKGRKYFHHNSDFYIPKATLKDSGSYFCRGLVGSKNVSSETVNITITQGLAVSTISSFFPPGYQTTT
PAPRPPTPAPTIASQPLSLRPEACRPAAGGAVHTRGLDFACDIIVAVVIATAVAAIVAAVVALIYCRKKRGR
KKLLYIFKQPFMRPVQTTQEEDGCSCRFPEEEEGGCELRVKFSRSADAPAYQQGQNQLYNELNLGRREEYDV
LDKRRGRDPEMGGKPRRKNPQEGLYNELQKDKMAEAYSEIGMKGERRRGKGHDGLYQGLSTATKDTYDALHM
QALPPR

**SEQ ID NO:27:**

*MALPVTALLLPLALLLHAARP*GMRTEDLPKAVVFLEPQWYRVLEKDSVTLKCQGAYSPEDNSTQWFHNESLI
SSQASSYFIDAATVDDSGEYRCQTNLSTLSDPVQLEVHIGWLLLQAPRWVFKEEDPIHLRCHSWKNTALHKV
TYLQNGKGRKYFHHNSDFYIPKATLKDSGSYFCRGLVGSKNVSSETVNITITQGLAVSTISSFFPPGYQTTT
PAPRPPTPAPTIASQPLSLRPEACRPAAGGAVHTRGLDFACDVLFYLAVGIMFLVNTVLWVTIRKEKRGRKK
LLYIFKQPFMRPVQTTQEEDGCSCRFPEEEEGGCELRVKFSRSADAPAYQQGQNQLYNELNLGRREEYDVLD
KRRGRDPEMGGKPRRKNPQEGLYNELQKDKMAEAYSEIGMKGERRRGKGHDGLYQGLSTATKDTYDALHMQA
LPPR

**SEQ ID NO:28:**

*MALPVTALLLPLALLLHAARP*GMRTEDLPKAVVFLEPQWYRVLEKDSVTLKCQGAYSPEDNSTQWFHNESLI
SSQASSYFIDAATVDDSGEYRCQTNLSTLSDPVQLEVHIGWLLLQAPRWVFKEEDPIHLRCHSWKNTALHKV
TYLQNGKGRKYFHHNSDFYIPKATLKDSGSYFCRGLVGSKNVSSETVNITITQGLAVSTISSFFPPGYQTTT
PAPRPPTPAPTIASQPLSLRPEACRPAAGGAVHTRGLDFACDIIILVGTAVIAMFFWLLLVIILRTKRGRKK
LLYIFKQPFMRPVQTTQEEDGCSCRFPEEEEGGCELRVKFSRSADAPAYQQGQNQLYNELNLGRREEYDVLD
KRRGRDPEMGGKPRRKNPQEGLYNELQKDKMAEAYSEIGMKGERRRGKGHDGLYQGLSTATKDTYDALHMQA
LPPR

SEQ ID NO:29:

*MALPVTALLLPLALLLHAARP*GMRTEDLPKAVVFLEPQWYRVLEKDSVTLKCQGAYSPEDNSTQWFHNESLI
SSQASSYFIDAATVDDSGEYRCQTNLSTLSDPVQLEVHIGWLLLQAPRWVFKEEDPIHLRCHSWKNTALHKV
TYLQNGKGRKYFHHNSDFYIPKATLKDSGSYFCRGLVGSKNVSSETVNITITQGLAVSTISSFFPPGYQTTT
PAPRPPTPAPTIASQPLSLRPEACRPAAGGAVHTRGLDFACDLGWLCLLLLPIPLIVWVKRKKRGRKKLLYI
FKQPFMRPVQTTQEEDGCSCRFPEEEEGGCELRVKFSRSADAPAYQQGQNQLYNELNLGRREEYDVLDKRRG
RDPEMGGKPRRKNPQEGLYNELQKDKMAEAYSEIGMKGERRRGKGHDGLYQGLSTATKDTYDALHMQALPPR

SEQ ID NO:30:

*MALPVTALLLPLALLLHAARP*GMRTEDLPKAVVFLEPQWYRVLEKDSVTLKCQGAYSPEDNSTQWFHNESLI
SSQASSYFIDAATVDDSGEYRCQTNLSTLSDPVQLEVHIGWLLLQAPRWVFKEEDPIHLRCHSWKNTALHKV
TYLQNGKGRKYFHHNSDFYIPKATLKDSGSYFCRGLVGSKNVSSETVNITITQGLAVSTISSFFPPGYQTTT
PAPRPPTPAPTIASQPLSLRPEACRPAAGGAVHTRGLDFACDIAIYCIGVFLIACMVVTVILCRMKKRGRKK
LLYIFKQPFMRPVQTTQEEDGCSCRFPEEEEGGCELRVKFSRSADAPAYQQGQNQLYNELNLGRREEYDVLD
KRRGRDPEMGGKPRRKNPQEGLYNELQKDKMAEAYSEIGMKGERRRGKGHDGLYQGLSTATKDTYDALHMQA
LPPR

SEQ ID NO:31:

*MALPVTALLLPLALLLHAARP*GMRTEDLPKAVVFLEPQWYRVLEKDSVTLKCQGAYSPEDNSTQWFHNESLI
SSQASSYFIDAATVDDSGEYRCQTNLSTLSDPVQLEVHIGWLLLQAPRWVFKEEDPIHLRCHSWKNTALHKV
TYLQNGKGRKYFHHNSDFYIPKATLKDSGSYFCRGLFGSKNVSSETVNITITQGLAVSTISSFFPPGYQTTT
PAPRPPTPAPTIASQPLSLRPEACRPAAGGAVHTRGLDFACDIYIWAPLAGTCGVLLLSLVITLYCKRGRKK
LLYIFKQPFMRPVQTTQEEDGCSCRFPEEEEGGCELRVKFSRSADAPAYQQGQNQLYNELNLGRREEYDVLD
KRRGRDPEMGGKPRRKNPQEGLYNELQKDKMAEAYSEIGMKGERRRGKGHDGLYQGLSTATKDTYDALHMQA
LPPR

SEQ ID NO:32:

*MALPVTALLLPLALLLHAARP*QVDTTKAVITLQPPWVSVFQEETVTLHCEVLHLPGSSSTQWFLNGTATQTS
TPSYRITSASVNDSGEYRCQRGLSGRSDPIQLEIHRGWLLLQVSSRVFTEGEPLALRCHAWKDKLVYNVLYY
RNGKAFKFFHWNSNLTILKTNISHNGTYHCSGMGKHRYTSAGISVTVKELFPAPVLNASVTSPLLEGNLVTL
SCETKLLLQRPGLQLYFSFYMGSKTLRGRNTSSEYQILTARREDSGLYWCEAATEDGNVLKRSPELELQVLG
LQLPTPVWFHIYIWAPLAGTCGVLLLSLVITLYCKRGRKKLLYIFKQPFMRPVQTTQEEDGCSCRFPEEEEG
GCELRVKFSRSADAPAYQQGQNQLYNELNLGRREEYDVLDKRRGRDPEMGGKPRRKNPQEGLYNELQKDKMA
EAYSEIGMKGERRRGKGHDGLYQGLSTATKDTYDALHMQALPPR

SEQ ID NO:33:

*MALPVTALLLPLALLLHAARP*GMRTEDLPKAVVFLEPQWYRVLEKDSVTLKCQGAYSPEDNSTQWFHNESLI
SSQASSYFIDAATVDDSGEYRCQTNLSTLSDPVQLEVHIGWLLLQAPRWVFKEEDPIHLRCHSWKNTALHKV
TYLQNGKGRKYFHHNSDFYIPKATLKDSGSYFCRGLVGSKNVSSETVNITITQGLAVSTISSFFPPGYQEPK
SCDKTHTCPPCPAPELLGGPSVFLFPPKPKDTLMISRTPEVTCVVVDVSHEDPEVKFNWYVDGVEVHNAKTK
PREEQYNSTYRVVSVLTVLHQDWLNGKEYKCKVSNKALPAPIEKTISKAKGQPREPQVYTLPPSRDELTKNQ
VSLTCLVKGFYPSDIAVEWESNGQPENNYKTTPPVLDSDGSFFLYSKLTVDKSRWQQGNVFSCSVMHEALHN
HYTQKSLSLSPGKIYIWAPLAGTCGVLLLSLVITLYCKRGRKKLLYIFKQPFMRPVQTTQEEDGCSCRFPEE
EEGGCELRVKFSRSADAPAYQQGQNQLYNELNLGRREEYDVLDKRRGRDPEMGGKPRRKNPQEGLYNELQKD
KMAEAYSEIGMKGERRRGKGHDGLYQGLSTATKDTYDALHMQALPPR

SEQ ID NO:34:

*MALPVTALLLPLALLLHAARP*GMRTEDLPKAVVFLEPQWYRVLEKDSVTLKCQGAYSPEDNSTQWFHNESLI
SSQASSYFIDAATVDDSGEYRCQTNLSTLSDPVQLEVHIGWLLLQAPRWVFKEEDPIHLRCHSWKNTALHKV
TYLQNGKGRKYFHHNSDFYIPKATLKDSGSYFCRGLVGSKNVSSETVNITITQGLAVSTISSFFPPGYQEPK
SCDKTHTCPGQPREPQVYTLPPSRDELTKNQVSLTCLVKGFYPSDIAVEWESNGQPENNYKTTPPVLDSDGS
FFLYSKLTVDKSRWQQGNVFSCSVMHEALHNHYTQKSLSLSPGKIYIWAPLAGTCGVLLLSLVITLYCKRGR
KKLLYIFKQPFMRPVQTTQEEDGCSCRFPEEEEGGCELRVKFSRSADAPAYQQGQNQLYNELNLGRREEYDV
LDKRRGRDPEMGGKPRRKNPQEGLYNELQKDKMAEAYSEIGMKGERRRGKGHDGLYQGLSTATKDTYDALHM
QALPPR

**SEQ ID NO:35:**

*MALPVTALLLPLALLLHAARP*GMRTEDLPKAVVFLEPQWYRVLEKDSVTLKCQGAYSPEDNSTQWFHNESLI
SSQASSYFIDAATVDDSGEYRCQTNLSTLSDPVQLEVHIGWLLLQAPRWVFKEEDPIHLRCHSWKNTALHKV
TYLQNGKGRKYFHHNSDFYIPKATLKDSGSYFCRGLVGSKNVSSETVNITITQGLAVSTISSFFPPGYQEPK
SCDKTHTCPIYIWAPLAGTCGVLLLSLVITLYCKRGRKKLLYIFKQPFMRPVQTTQEEDGCSCRFPEEEEGG
CELRVKFSRSADAPAYQQGQNQLYNELNLGRREEYDVLDKRRGRDPEMGGKPRRKNPQEGLYNELQKDKMAE
AYSEIGMKGERRRGKGHDGLYQGLSTATKDTYDALHMQALPPR

**SEQ ID NO:36:**

*MALPVTALLLPLALLLHAARP*GMRTEDLPKAVVFLEPQWYRVLEKDSVTLKCQGAYSPEDNSTQWFHNESLI
SSQASSYFIDAATVDDSGEYRCQTNLSTLSDPVQLEVHIGWLLLQAPRWVFKEEDPIHLRCHSWKNTALHKV
TYLQNGKGRKYFHHNSDFYIPKATLKDSGSYFCRGLVGSKNVSSETVNITITQGLAVSTISSFFPPGYQTTT
PAPRPPTPAPTIASQPLSLRPEAFACDIYIWAPLAGTCGVLLLSLVITLYCKRGRKKLLYIFKQPFMRPVQT
TQEEDGCSCRFPEEEEGGCELRVKFSRSADAPAYQQGQNQLYNELNLGRREEYDVLDKRRGRDPEMGGKPRR
KNPQEGLYNELQKDKMAEAYSEIGMKGERRRGKGHDGLYQGLSTATKDTYDALHMQALPPR

**SEQ ID NO:37:**

*MALPVTALLLPLALLLHAARP*GMRTEDLPKAVVFLEPQWYRVLEKDSVTLKCQGAYSPEDNSTQWFHNESLI
SSQASSYFIDAATVDDSGEYRCQTNLSTLSDPVQLEVHIGWLLLQAPRWVFKEEDPIHLRCHSWKNTALHKV
TYLQNGKGRKYFHHNSDFYIPKATLKDSGSYFCRGLVGSKNVSSETVNITITQGLAVSTISSFFPPGYQTTT
PAPRPPTPFACDIYIWAPLAGTCGVLLLSLVITLYCKRGRKKLLYIFKQPFMRPVQTTQEEDGCSCRFPEEE
EGGCELRVKFSRSADAPAYQQGQNQLYNELNLGRREEYDVLDKRRGRDPEMGGKPRRKNPQEGLYNELQKDK
MAEAYSEIGMKGERRRGKGHDGLYQGLSTATKDTYDALHMQALPPR

**SEQ ID NO:38:**

*MALPVTALLLPLALLLHAARP*GMRTEDLPKAVVFLEPQWYRVLEKDSVTLKCQGAYSPEDNSTQWFHNESLI
SSQASSYFIDAATVDDSGEYRCQTNLSTLSDPVQLEVHIGWLLLQAPRWVFKEEDPIHLRCHSWKNTALHKV
TYLQNGKGRKYFHHNSDFYIPKATLKDSGSYFCRGLVGSKNVSSETVNITITQGLAVSTISSFFPPGYQGGG
GSGGGGSGGGGSIYIWAPLAGTCGVLLLSLVITLYCKRGRKKLLYIFKQPFMRPVQTTQEEDGCSCRFPEEE
EGGCELRVKFSRSADAPAYQQGQNQLYNELNLGRREEYDVLDKRRGRDPEMGGKPRRKNPQEGLYNELQKDK
MAEAYSEIGMKGERRRGKGHDGLYQGLSTATKDTYDALHMQALPPR

**SEQ ID NO:39:**

*MALPVTALLLPLALLLHAARP*GMRTEDLPKAVVFLEPQWYRVLEKDSVTLKCQGAYSPEDNSTQWFHNESLI
SSQASSYFIDAATVDDSGEYRCQTNLSTLSDPVQLEVHIGWLLLQAPRWVFKEEDPIHLRCHSWKNTALHKV
TYLQNGKGRKYFHHNSDFYIPKATLKDSGSYFCRGLVGSKNVSSETVNITITQGLAVSTISSFFPPGYQGGG
GSGGGGSGGGGSGGGGSGGGGSGGGGSIYIWAPLAGTCGVLLLSLVITLYCKRGRKKLLYIFKQPFMRPVQT
TQEEDGCSCRFPEEEEGGCELRVKFSRSADAPAYQQGQNQLYNELNLGRREEYDVLDKRRGRDPEMGGKPRR
KNPQEGLYNELQKDKMAEAYSEIGMKGERRRGKGHDGLYQGLSTATKDTYDALHMQALPPR

**SEQ ID NO:40:**

*MALPVTALLLPLALLLHAARP*GMRTEDLPKAVVFLEPQWYRVLEKDSVTLKCQGAYSPEDNSTQWFHNESLI
SSQASSYFIDAATVDDSGEYRCQTNLSTLSDPVQLEVHIGWLLLQAPRWVFKEEDPIHLRCHSWKNTALHKV
TYLQNGKGRKYFHHNSDFYIPKATLKDSGSYFCRGLVGSKNVSSETVNITITQGLAVSTISSFFPPGYQGGG
GSGGGGSGGGGSGGGGSGGGGSGGGGSGGGGSGGGGSIYIWAPLAGTCGVLLLSLVITLYCKRGRKK
LLYIFKQPFMRPVQTTQEEDGCSCRFPEEEEGGCELRVKFSRSADAPAYQQGQNQLYNELNLGRREEYDVLD
KRRGRDPEMGGKPRRKNPQEGLYNELQKDKMAEAYSEIGMKGERRRGKGHDGLYQGLSTATKDTYDALHMQA
LPPR

**SEQ ID NO:41:**

*MALPVTALLLPLALLLHAARP*GMRTEDLPKAVVFLEPQWYRVLEKDSVTLKCQGAYSPEDNSTQWFHNESLI
SSQASSYFIDAATVDDSGEYRCQTNLSTLSDPVQLEVHIGWLLLQAPRWVFKEEDPIHLRCHSWKNTALHKV
TYLQNGKGRKYFHHNSDFYIPKATLKDSGSYFCRGLVGSKNVSSETVNITITQGLAVSTISSFFPPGYQGGG
GSGGGGSGGGGSGGGGSGGGGSGGGGSGGGGSGGGGSGGGGSGGGGSIYIWAPLAGTCGVLL
LSLVITLYCKRGRKKLLYIFKQPFMRPVQTTQEEDGCSCRFPEEEEGGCELRVKFSRSADAPAYQQGQNQLY
NELNLGRREEYDVLDKRRGRDPEMGGKPRRKNPQEGLYNELQKDKMAEAYSEIGMKGERRRGKGHDGLYQGL
STATKDTYDALHMQALPPR

**SEQ ID NO:42:**

*MALPVTALLLPLALLLHAARP*GMRTEDLPKAVVFLEPQWYRVLEKDSVTLKCQGAYSPEDNSTQWFHNESLI
SSQASSYFIDAATVDDSGEYRCQTNLSTLSDPVQLEVHIGWLLLQAPRWVFKEEDPIHLRCHSWKNTALHKV
TYLQNGKGRKYFHHNSDFYIPKATLKDSGSYFCRGLVGSKNVSSETVNITITQGLAVSTISSFFPPGYQGGS
PAGSPTSTEEGTSESATPESGPGTSTEPSEGSAPGSPAGSPTSTEEGTSTEPSEGSAIYIWAPLAGTCGVLL
LSLVITLYCKRGRKKLLYIFKQPFMRPVQTTQEEDGCSCRFPEEEEGGCELRVKFSRSADAPAYQQGQNQLY
NELNLGRREEYDVLDKRRGRDPEMGGKPRRKNPQEGLYNELQKDKMAEAYSEIGMKGERRRGKGHDGLYQGL
STATKDTYDALHMQALPPR

**SEQ ID NO:43:**

*MALPVTALLLPLALLLHAARP*GMRTEDLPKAVVFLEPQWYRVLEKDSVTLKCQGAYSPEDNSTQWFHNESLI
SSQASSYFIDAATVDDSGEYRCQTNLSTLSDPVQLEVHIGWLLLQAPRWVFKEEDPIHLRCHSWKNTALHKV
TYLQNGKGRKYFHHNSDFYIPKATLKDSGSYFCRGLVGSKNVSSETVNITITQGLAVSTISSFFPPGYQGGS
PAGSPTSTEEGTSESATPESGPGTSTEIYIWAPLAGTCGVLLLSLVITLYCKRGRKKLLYIFKQPFMRPVQT
TQEEDGCSCRFPEEEEGGCELRVKFSRSADAPAYQQGQNQLYNELNLGRREEYDVLDKRRGRDPEMGGKPRR
KNPQEGLYNELQKDKMAEAYSEIGMKGERRRGKGHDGLYQGLSTATKDTYDALHMQALPPR

**SEQ ID NO:44:**

*MALPVTALLLPLALLLHAARP*GMRTEDLPKAVVFLEPQWYRVLEKDSVTLKCQGAYSPEDNSTQWFHNESLI
SSQASSYFIDAATVDDSGEYRCQTNLSTLSDPVQLEVHIGWLLLQAPRWVFKEEDPIHLRCHSWKNTALHKV
TYLQNGKGRKYFHHNSDFYIPKATLKDSGSYFCRGLVGSKNVSSETVNITITQGLAVSTISSFFPPGYQGGS
PAGSPTSTEEGTIYIWAPLAGTCGVLLLSLVITLYCKRGRKKLLYIFKQPFMRPVQTTQEEDGCSCRFPEEE
EGGCELRVKFSRSADAPAYQQGQNQLYNELNLGRREEYDVLDKRRGRDPEMGGKPRRKNPQEGLYNELQKDK
MAEAYSEIGMKGERRRGKGHDGLYQGLSTATKDTYDALHMQALPPR

**SEQ ID NO:45:**

36

*MLRLLLALNLFPSIQVTG*GMRTEDLPKAVVFLEPQWYRVLEKDSVTLKCQGAYSPEDNSTQWFHNESLISSQ
ASSYFIDAATVDDSGEYRCQTNLSTLSDPVQLEVHIGWLLLQAPRWVFKEEDPIHLRCHSWKNTALHKVTYL
QNGKGRKYFHHNSDFYIPKATLKDSGSYFCRGLVGSKNVSSETVNITITQGLAVSTISSFFPPGYQTTTPAP
RPPTPAPTIASQPLSLRPEACRPAAGGAVHTRGLDFACDIYIWAPLAGTCGVLLLSLVITLYCKRGRKKLLY
IFKQPFMRPVQTTQEEDGCSCRFPEEEEGGCELRVKFSRSADAPAYQQGQNQLYNELNLGRREEYDVLDKRR
GRDPEMGGKPRRKNPQEGLYNELQKDKMAEAYSEIGMKGERRRGKGHDGLYQGLSTATKDTYDALHMQALPP
R

**SEQ ID NO:46:**

*METDTLLLWVLLLWVPGSTGD*GMRTEDLPKAVVFLEPQWYRVLEKDSVTLKCQGAYSPEDNSTQWFHNESLI
SSQASSYFIDAATVDDSGEYRCQTNLSTLSDPVQLEVHIGWLLLQAPRWVFKEEDPIHLRCHSWKNTALHKV
TYLQNGKGRKYFHHNSDFYIPKATLKDSGSYFCRGLVGSKNVSSETVNITITQGLAVSTISSFFPPGYQTTT
PAPRPPTPAPTIASQPLSLRPEACRPAAGGAVHTRGLDFACDIYIWAPLAGTCGVLLLSLVITLYCKRGRKK
LLYIFKQPFMRPVQTTQEEDGCSCRFPEEEEGGCELRVKFSRSADAPAYQQGQNQLYNELNLGRREEYDVLD
KRRGRDPEMGGKPRRKNPQEGLYNELQKDKMAEAYSEIGMKGERRRGKGHDGLYQGLSTATKDTYDALHMQA
LPPR

**SEQ ID NO:47:**

*MWQLLLPTALLLLVSA*GMRTEDLPKAVVFLEPQWYRVLEKDSVTLKCQGAYSPEDNSTQWFHNESLISSQAS
SYFIDAATVDDSGEYRCQTNLSTLSDPVQLEVHIGWLLLQAPRWVFKEEDPIHLRCHSWKNTALHKVTYLQN
GKGRKYFHHNSDFYIPKATLKDSGSYFCRGLVGSKNVSSETVNITITQGLAVSTISSFFPPGYQTTTPAPRP
PTPAPTIASQPLSLRPEACRPAAGGAVHTRGLDFACDIYIWAPLAGTCGVLLLSLVITLYCKRGRKKLLYIF
KQPFMRPVQTTQEEDGCSCRFPEEEEGGCELRVKFSRSADAPAYQQGQNQLYNELNLGRREEYDVLDKRRGR
DPEMGGKPRRKNPQEGLYNELQKDKMAEAYSEIGMKGERRRGKGHDGLYQGLSTATKDTYDALHMQALPPR

**SEQ ID NO:48:**

*MALPVTALLLPLALLLHAARP*GMRTEDLPKAVVFLEPQWYRVLEKDSVTLKCQGAYSPEDNSTQWFHNESLI
SSQASSYFIDAATVDDSGEYRCQTNLSTLSDPVQLEVHIGWLLLQAPRWVFKEEDPIHLRCHSWKNTALHKV
TYLQNGKGRKYFHHNSDFYIPKATLKDSGSYFCRGLVGSKNVSSETVNITITQGLAVSTISSFFPPGYQTTT
PAPRPPTPAPTIASQPLSLRPEACRPAAGGAVHTRGLDFACDIYIWAPLAGTCGVLLLSLVITLYCCWLTKK
KYSSSVHDPNGEYMFMRAVNTAKKSRLTDVTLRVKFSRSADAPAYQQGQNQLYNELNLGRREEYDVLDKRRG
RDPEMGGKPRRKNPQEGLYNELQKDKMAEAYSEIGMKGERRRGKGHDGLYQGLSTATKDTYDALHMQALPPR

**SEQ ID NO:49:**

*MALPVTALLLPLALLLHAARP*GMRTEDLPKAVVFLEPQWYRVLEKDSVTLKCQGAYSPEDNSTQWFHNESLI
SSQASSYFIDAATVDDSGEYRCQTNLSTLSDPVQLEVHIGWLLLQAPRWVFKEEDPIHLRCHSWKNTALHKV
TYLQNGKGRKYFHHNSDFYIPKATLKDSGSYFCRGLVGSKNVSSETVNITITQGLAVSTISSFFPPGYQTTT
PAPRPPTPAPTIASQPLSLRPEACRPAAGGAVHTRGLDFACDIYIWAPLAGTCGVLLLSLVITLYCQRRKYR
SNKGESPVEPAEPCRYSCPREEEGSTIPIQEDYRKPEPACSPRVKFSRSADAPAYQQGQNQLYNELNLGRRE
EYDVLDKRRGRDPEMGGKPRRKNPQEGLYNELQKDKMAEAYSEIGMKGERRRGKGHDGLYQGLSTATKDTYD
ALHMQALPPR

**SEQ ID NO:50:**

*MALPVTALLLPLALLLHAARP*GMRTEDLPKAVVFLEPQWYRVLEKDSVTLKCQGAYSPEDNSTQWFHNESLI
SSQASSYFIDAATVDDSGEYRCQTNLSTLSDPVQLEVHIGWLLLQAPRWVFKEEDPIHLRCHSWKNTALHKV
TYLQNGKGRKYFHHNSDFYIPKATLKDSGSYFCRGLVGSKNVSSETVNITITQGLAVSTISSFFPPGYQTTT
PAPRPPTPAPTIASQPLSLRPEACRPAAGGAVHTRGLDFACDIYIWAPLAGTCGVLLLSLVITLYCQLGLHI
WQLRSQCMWPRETQLLLEVPPSTEDARSCQFPEEERGERSAEEKGRLGDLWVRVKFSRSADAPAYQQGQNQL
YNELNLGRREEYDVLDKRRGRDPEMGGKPRRKNPQEGLYNELQKDKMAEAYSEIGMKGERRRGKGHDGLYQG
LSTATKDTYDALHMQALPPR

**SEQ ID NO:51:**

*MALPVTALLLPLALLLHAARP*GMRTEDLPKAVVFLEPQWYRVLEKDSVTLKCQGAYSPEDNSTQWFHNESLI
SSQASSYFIDAATVDDSGEYRCQTNLSTLSDPVQLEVHIGWLLLQAPRWVFKEEDPIHLRCHSWKNTALHKV
TYLQNGKGRKYFHHNSDFYIPKATLKDSGSYFCRGLVGSKNVSSETVNITITQGLAVSTISSFFPPGYQTTT
PAPRPPTPAPTIASQPLSLRPEACRPAAGGAVHTRGLDFACDIYIWAPLAGTCGVLLLSLVITLYCCVKRRK
PRGDVVKVIVSVQRKRQEAEGEATVIEALQAPPDVTTVAVEETIPSFTGRSPNHRVKFSRSADAPAYQQGQN
QLYNELNLGRREEYDVLDKRRGRDPEMGGKPRRKNPQEGLYNELQKDKMAEAYSEIGMKGERRRGKGHDGLY
QGLSTATKDTYDALHMQALPPR

**SEQ ID NO:52:**

*MALPVTALLLPLALLLHAARP*GMRTEDLPKAVVFLEPQWYRVLEKDSVTLKCQGAYSPEDNSTQWFHNESLI
SSQASSYFIDAATVDDSGEYRCQTNLSTLSDPVQLEVHIGWLLLQAPRWVFKEEDPIHLRCHSWKNTALHKV
TYLQNGKGRKYFHHNSDFYIPKATLKDSGSYFCRGLVGSKNVSSETVNITITQGLAVSTISSFFPPGYQTTT
PAPRPPTPAPTIASQPLSLRPEACRPAAGGAVHTRGLDFACDIYIWAPLAGTCGVLLLSLVITLYCKKYFFK
KEVQQLSVSFSSLQIKALQNAVEKEVQAEDNIYIENSLYATDRVKFSRSADAPAYQQGQNQLYNELNLGRRE
EYDVLDKRRGRDPEMGGKPRRKNPQEGLYNELQKDKMAEAYSEIGMKGERRRGKGHDGLYQGLSTATKDTYD
ALHMQALPPR

**SEQ ID NO:53:**

*MALPVTALLLPLALLLHAARP*GMRTEDLPKAVVFLEPQWYRVLEKDSVTLKCQGAYSPEDNSTQWFHNESLI
SSQASSYFIDAATVDDSGEYRCQTNLSTLSDPVQLEVHIGWLLLQAPRWVFKEEDPIHLRCHSWKNTALHKV
TYLQNGKGRKYFHHNSDFYIPKATLKDSGSYFCRGLVGSKNVSSETVNITITQGLAVSTISSFFPPGYQTTT
PAPRPPTPAPTIASQPLSLRPEACRPAAGGAVHTRGLDFACDIYIWAPLAGTCGVLLLSLVITLYCYKVGFF
KRNLKEKMEAGRGVPNGIPAEDSEQLASGQEAGDPGCLKPLHEKDSESGGGKDRVKFSRSADAPAYQQGQNQ
LYNELNLGRREEYDVLDKRRGRDPEMGGKPRRKNPQEGLYNELQKDKMAEAYSEIGMKGERRRGKGHDGLYQ
GLSTATKDTYDALHMQALPPR

**SEQ ID NO:54:**

*MALPVTALLLPLALLLHAARP*GMRTEDLPKAVVFLEPQWYRVLEKDSVTLKCQGAYSPEDNSTQWFHNESLI
SSQASSYFIDAATVDDSGEYRCQTNLSTLSDPVQLEVHIGWLLLQAPRWVFKEEDPIHLRCHSWKNTALHKV
TYLQNGKGRKYFHHNSDFYIPKATLKDSGSYFCRGLVGSKNVSSETVNITITQGLAVSTISSFFPPGYQTTT
PAPRPPTPAPTIASQPLSLRPEACRPAAGGAVHTRGLDFACDIYIWAPLAGTCGVLLLSLVITLYCKRKKQR
SRRNDEELETRAHRVATEERGRKPHQIPASTPQNPATSQHPPPPPGHRSQAPSHRPPPPGHRVQHQPQKRPP
APSGTQVHQQKGPPLPRPRVQPKPPHGAAENSLSPSSNRVKFSRSADAPAYQQGQNQLYNELNLGRREEYDV
LDKRRGRDPEMGGKPRRKNPQEGLYNELQKDKMAEAYSEIGMKGERRRGKGHDGLYQGLSTATKDTYDALHM
QALPPR

**SEQ ID NO:55:**

*MALPVTALLLPLALLLHAARP*GMRTEDLPKAVVFLEPQWYRVLEKDSVTLKCQGAYSPEDNSTQWFHNESLI
SSQASSYFIDAATVDDSGEYRCQTNLSTLSDPVQLEVHIGWLLLQAPRWVFKEEDPIHLRCHSWKNTALHKV
TYLQNGKGRKYFHHNSDFYIPKATLKDSGSYFCRGLVGSKNVSSETVNITITQGLAVSTISSFFPPGYQTTT
PAPRPPTPAPTIASQPLSLRPEACRPAAGGAVHTRGLDFACDPQLCYILDAILFLYGIVLTLLYCRLKIQVR
KAAITSYEKSDGVYTGLSTRNQETYETLKHEKPPQKRGRKKLLYIFKQPFMRPVQTTQEEDGCSCRFPEEEE
GGCEL

**SEQ ID NO:56:**

*MALPVTALLLPLALLLHAARP*GMRTEDLPKAVVFLEPQWYRVLEKDSVTLKCQGAYSPEDNSTQWFHNESLI
SSQASSYFIDAATVDDSGEYRCQTNLSTLSDPVQLEVHIGWLLLQAPRWVFKEEDPIHLRCHSWKNTALHKV
TYLQNGKGRKYFHHNSDFYIPKATLKDSGSYFCRGLVGSKNVSSETVNITITQGLAVSTISSFFPPGYQTTT
PAPRPPTPAPTIASQPLSLRPEACRPAAGGAVHTRGLDFACDIYIWAPLAGTCGVLLLSLVITLYCKRGRKK
LLYIFKQPFMRPVQTTQEEDGCSCRFPEEEEGGCELRLKIQVRKAAITSYEKSDGVYTGLSTRNQETYETLK
HEKPPQ

**SEQ ID NO:57:**

*MALPVTALLLPLALLLHAARP*GMRTEDLPKAVVFLEPQWYRVLEKDSVTLKCQGAYSPEDNSTQWFHNESLI
SSQASSYFIDAATVDDSGEYRCQTNLSTLSDPVQLEVHIGWLLLQAPRWVFKEEDPIHLRCHSWKNTALHKV
TYLQNGKGRKYFHHNSDFYIPKATLKDSGSYFCRGLVGSKNVSSETVNITITQGLAVSTISSFFPPGYQIEV
MYPPPYLDNEKSNGTIIHVKGKHLCPSPLFPGPSKPFWVLVVVGGVLACYSLLVTVAFIIFWVRSKRSRLLH
SDYMNMTPRRPGPTRKHYQPYAPPRDFAAYRSRVKFSRSADAPAYQQGQNQLYNELNLGRREEYDVLDKRRG
RDPEMGGKPRRKNPQEGLYNELQKDKMAEAYSEIGMKGERRRGKGHDGLYQGLSTATKDTYDALHMQALPPR

**SEQ ID NO:58:**

*MALPVTALLLPLALLLHAARP*GMRTEDLPKAVVFLEPQWYRVLEKDSVTLKCQGAYSPEDNSTQWFHNESLI
SSQASSYFIDAATVDDSGEYRCQTNLSTLSDPVQLEVHIGWLLLQAPRWVFKEEDPIHLRCHSWKNTALHKV
TYLQNGKGRKYFHHNSDFYIPKATLKDSGSYFCRGLVGSKNVSSETVNITITQGLAVSTISSFFPPGYQIYI
WAPLAGTCGVLLLSLVITLYCRSKRSRLLHSDYMNMTPRRPGPTRKHYQPYAPPRDFAAYRSRVKFSRSADA
PAYQQGQNQLYNELNLGRREEYDVLDKRRGRDPEMGGKPRRKNPQEGLYNELQKDKMAEAYSEIGMKGERRR
GKGHDGLYQGLSTATKDTYDALHMQALPPR

**SEQ ID NO:59:**

*MALPVTALLLPLALLLHAARP*GMRTEDLPKAVVFLEPQWYRVLEKDSVTLKCQGAYSPEDNSTQWFHNESLI
SSQASSYFIDAATVDDSGEYRCQTNLSTLSDPVQLEVHIGWLLLQAPRWVFKEEDPIHLRCHSWKNTALHKV
TYLQNGKGRKYFHHNSDFYIPKATLKDSGSYFCRGLVGSKNVSSETVNITITQGLAVSTISSFFPPGYQTTT
PAPRPPTPAPTIASQPLSLRPEACRPAAGGAVHTRGLDFACDIYIWAPLAGTCGVLLLSLVITLYCRSKRSR
LLHSDYMNMTPRRPGPTRKHYQPYAPPRDFAAYRSQRRKYRSNKGESPVEPAEPCHYSCPREEEGSTIPIQE
DYRKPEPACSPRVKFSRSADAPAYQQGQNQLYNELNLGRREEYDVLDKRRGRDPEMGGKPRRKNPQEGLYNE
LQKDKMAEAYSEIGMKGERRRGKGHDGLYQGLSTATKDTYDALHMQALPPR

**SEQ ID NO:60:**

*MALPVTALLLPLALLLHAARP*GMRTEDLPKAVVFLEPQWYRVLEKDSVTLKCQGAYSPEDNSTQWFHNESLI
SSQASSYFIDAATVDDSGEYRCQTNLSTLSDPVQLEVHIGWLLLQAPRWVFKEEDPIHLRCHSWKNTALHKV
TYLQNGKGRKYFHHNSDFYIPKATLKDSGSYFCRGLVGSKNVSSETVNITITQGLAVSTISSFFPPGYQTTT
PAPRPPTPAPTIASQPLSLRPEACRPAAGGAVHTRGLDFACDIYIWAPLAGTCGVLLLSLVITLYCRSKRSR
LLHSDYMNMTPRRPGPTRKHYQPYAPPRDFAAYRSRRDQRLPPDAHKPPGGGSFRTPIQEEQADAHSTLAKI
RVKFSRSADAPAYQQGQNQLYNELNLGRREEYDVLDKRRGRDPEMGGKPRRKNPQEGLYNELQKDKMAEAYS
EIGMKGERRRGKGHDGLYQGLSTATKDTYDALHMQALPPR

**SEQ ID NO:61:**

*MALPVTALLLPLALLLHAARP*GMRTEDLPKAVVFLEPQWYRVLEKDSVTLKCQGAYSPEDNSTQWFHNESLI
SSQASSYFIDAATVDDSGEYRCQTNLSTLSDPVQLEVHIGWLLLQAPRWVFKEEDPIHLRCHSWKNTALHKV
TYLQNGKGRKYFHHNSDFYIPKATLKDSGSYFCRGLVGSKNVSSETVNITITQGLAVSTISSFFPPGYQTTT
PAPRPPTPAPTIASQPLSLRPEACRPAAGGAVHTRGLDFACDIYIWAPLAGTCGVLLLSLVITLYCKRGRKK
LLYIFKQPFMRPVQTTQEEDGCSCRFPEEEEGGCELRSKRSRLLHSDYMNMTPRRPGPTRKHYQPYAPPRDF
AAYRSRVKFSRSADAPAYQQGQNQLYNELNLGRREEYDVLDKRRGRDPEMGGKPRRKNPQEGLYNELQKDKM
AEAYSEIGMKGERRRGKGHDGLYQGLSTATKDTYDALHMQALPPR

**SEQ ID NO:62:**

*MALPVTALLLPLALLLHAARP*GMRTEDLPKAVVFLEPQWYRVLEKDSVTLKCQGAYSPEDNSTQWFHNESLI
SSQASSYFIDAATVDDSGEYRCQTNLSTLSDPVQLEVHIGWLLLQAPRWVFKEEDPIHLRCHSWKNTALHKV
TYLQNGKGRKYFHHNSDFYIPKATLKDSGSYFCRGLVGSKNVSSETVNITITQGLAVSTISSFFPPGYQIEV
MYPPPYLDNEKSNGTIIHVKGKHLCPSPLFPGPSKPFWVLVVVGGVLACYSLLVTVAFIIFWVRSKRSRLLH
SDYMNMTPRRPGPTRKHYQPYAPPRDFAAYRSKRGRKKLLYIFKQPFMRPVQTTQEEDGCSCRFPEEEEGGC
ELRVKFSRSADAPAYQQGQNQLYNELNLGRREEYDVLDKRRGRDPEMGGKPRRKNPQEGLYNELQKDKMAEA
YSEIGMKGERRRGKGHDGLYQGLSTATKDTYDALHMQALPPR

**SEQ ID NO:63:**

*MALPVTALLLPLALLLHAARP*GMRTEDLPKAVVFLEPQWYRVLEKDSVTLKCQGAYSPEDNSTQWFHNESLI
SSQASSYFIDAATVDDSGEYRCQTNLSTLSDPVQLEVHIGWLLLQAPRWVFKEEDPIHLRCHSWKNTALHKV
TYLQNGKGRKYFHHNSDFYIPKATLKDSGSYFCRGLVGSKNVSSETVNITITQGLAVSTISSFFPPGYQIEV
MYPPPYLDNEKSNGTIIHVKGKHLCPSPLFPGPSKPFWVLVVVGGVLACYSLLVTVAFIIFWVKRGRKKLLY
IFKQPFMRPVQTTQEEDGCSCRFPEEEEGGCELRVKFSRSADAPAYQQGQNQLYNELNLGRREEYDVLDKRR
GRDPEMGGKPRRKNPQEGLYNELQKDKMAEAYSEIGMKGERRRGKGHDGLYQGLSTATKDTYDALHMQALPP
R

**SEQ ID NO:64:**

*MALPVTALLLPLALLLHAARP*GMRTEDLPKAVVFLEPQWYRVLEKDSVTLKCQGAYSPEDNSTQWFHNESLI
SSQASSYFIDAATVDDSGEYRCQTNLSTLSDPVQLEVHIGWLLLQAPRWVFKEEDPIHLRCHSWKNTALHKV
TYLQNGKGRKYFHHNSDFYIPKATLKDSGSYFCRGLVGSKNVSSETVNITITQGLAVSTISSFFPPGYQTTT
PAPRPPTPAPTIASQPLSLRPEACRPAAGGAVHTRGLDFACDIYIWAPLAGTCGVLLLSLVITLYCQRRKYR
SNKGESPVEPAEPCHYSCPREEEGSTIPIQEDYRKPEPACSPRVKFSRSADAPAYQQGQNQLYNELNLGRRE
EYDVLDKRRGRDPEMGGKPRRKNPQEGLYNELQKDKMAEAYSEIGMKGERRRGKGHDGLYQGLSTATKDTYD
ALHMQALPPR

**SEQ ID NO:65:**

*MALPVTALLLPLALLLHAARP*GMRTEDLPKAVVFLEPQWYRVLEKDSVTLKCQGAYSPEDNSTQWFHNESLI
SSQASSYFIDAATVDDSGEYRCQTNLSTLSDPVQLEVHIGWLLLQAPRWVFKEEDPIHLRCHSWKNTALHKV
TYLQNGKGRKYFHHNSDFYIPKATLKDSGSYFCRGLVGSKNVSSETVNITITQGLAVSTISSFFPPGYQTTT
PAPRPPTPAPTIASQPLSLRPEACRPAAGGAVHTRGLDFACDIYIWAPLAGTCGVLLLSLVITLYCRSKRSR
LLHSDYMNMTPRRPGPTRKHYQPYAPPRDFAAYRSRVKFSRSADAPAYQQGQNQLYNELNLGRREEYDVLDK
RRGRDPEMGGKPRRKNPQEGLYNELQKDKMAEAYSEIGMKGERRRGKGHDGLYQGLSTATKDTYDALHMQAL
PPR

**SEQ ID NO:66:**

*MALPVTALLLPLALLLHAARP*GMRTEDLPKAVVFLEPQWYRVLEKDSVTLKCQGAYSPEDNSTQWFHNESLI
SSQASSYFIDAATVDDSGEYRCQTNLSTLSDPVQLEVHIGWLLLQAPRWVFKEEDPIHLRCHSWKNTALHKV
TYLQNGKGRKYFHHNSDFYIPKATLKDSGSYFCRGLVGSKNVSSETVNITITQGLAVSTISSFFPPGYQTTT
PAPRPPTPAPTIASQPLSLRPEACRPAAGGAVHTRGLDFACDIYIWAPLAGTCGVLLLSLVITLYCKKKYSS
SVHDPNGEYMFMRAVNTAKKSRLTDVTLRVKFSRSADAPAYQQGQNQLYNELNLGRREEYDVLDKRRGRDPE
MGGKPRRKNPQEGLYNELQKDKMAEAYSEIGMKGERRRGKGHDGLYQGLSTATKDTYDALHMQALPPR

**SEQ ID NO:67:**

*MALPVTALLLPLALLLHAARP*GMRTEDLPKAVVFLEPQWYRVLEKDSVTLKCQGAYSPEDNSTQWFHNESLI
SSQASSYFIDAATVDDSGEYRCQTNLSTLSDPVQLEVHIGWLLLQAPRWVFKEEDPIHLRCHSWKNTALHKV
TYLQNGKGRKYFHHNSDFYIPKATLKDSGSYFCRGLVGSKNVSSETVNITITQGLAVSTISSFFPPGYQTTT
PAPRPPTPAPTIASQPLSLRPEACRPAAGGAVHTRGLDFACDIYIWAPLAGTCGVLLLSLVITLYCRRDQRL
PPDAHKPPGGGSFRTPIQEEQADAHSTLAKIRVKFSRSADAPAYQQGQNQLYNELNLGRREEYDVLDKRRGR
DPEMGGKPRRKNPQEGLYNELQKDKMAEAYSEIGMKGERRRGKGHDGLYQGLSTATKDTYDALHMQALPPR

**SEQ ID NO:68:**

*MALPVTALLLPLALLLHAARP*GMRTEDLPKAVVFLEPQWYRVLEKDSVTLKCQGAYSPEDNSTQWFHNESLI
SSQASSYFIDAATVDDSGEYRCQTNLSTLSDPVQLEVHIGWLLLQAPRWVFKEEDPIHLRCHSWKNTALHKV
TYLQNGKGRKYFHHNSDFYIPKATLKDSGSYFCRGLVGSKNVSSETVNITITQGLAVSTISSFFPPGYQTTT
PAPRPPTPAPTIASQPLSLRPEACRPAAGGAVHTRGLDFACDIYIWAPLAGTCGVLLLSLVITLYCRSKRSR
LLHSDYMNMTPRRPGPTRKHYQPYAPPRDFAAYRSKKKYSSSVHDPNGEYMFMRAVNTAKKSRLTDVTLRVK
FSRSADAPAYQQGQNQLYNELNLGRREEYDVLDKRRGRDPEMGGKPRRKNPQEGLYNELQKDKMAEAYSEIG
MKGERRRGKGHDGLYQGLSTATKDTYDALHMQALPPR

**SEQ ID NO:69:**

*MALPVTALLLPLALLLHAARP*GMRTEDLPKAVVFLEPQWYRVLEKDSVTLKCQGAYSPEDNSTQWFHNESLI
SSQASSYFIDAATVDDSGEYRCQTNLSTLSDPVQLEVHIGWLLLQAPRWVFKEEDPIHLRCHSWKNTALHKV
TYLQNGKGRKYFHHNSDFYIPKATLKDSGSYFCRGLVGSKNVSSETVNITITQGLAVSTISSFFPPGYQIYI
WAPLAGTCGVLLLSLVITLYCKRGRKKLLYIFKQPFMRPVQTTQEEDGCSCRFPEEEEGGCELRVKFSRSAD
APAYQQGQNQLYNELNLGRREEYDVLDKRRGRDPEMGGKPRRKNPQEGLYNELQKDKMAEAYSEIGMKGERR
RGKGHDGLYQGLSTATKDTYDALHMQALPPR

SEQ ID NO:70:

*MALPVTALLLPLALLLHAARP*GMRTEDLPKAVVFLEPQWYRVLEKDSVTLKCQGAYSPEDNSTQWFHNESLI
SSQASSYFIDAATVDDSGEYRCQTNLSTLSDPVQLEVHIGWLLLQAPRWVFKEEDPIHLRCHSWKNTALHKV
TYLQNGKGRKYFHHNSDFYIPKATLKDSGSYFCRGLVGSKNVSSETVNITITQGLAVSTISSFFPPGYQIYI
WAPLAGTCGVLLLSLVITLYCQRRKYRSNKGESPVEPAEPCHYSCPREEEGSTIPIQEDYRKPEPACSPRVK
FSRSADAPAYQQGQNQLYNELNLGRREEYDVLDKRRGRDPEMGGKPRRKNPQEGLYNELQKDKMAEAYSEIG
MKGERRRGKGHDGLYQGLSTATKDTYDALHMQALPPR

SEQ ID NO:71:

*MALPVTALLLPLALLLHAARP*GMRTEDLPKAVVFLEPQWYRVLEKDSVTLKCQGAYSPEDNSTQWFHNESLI
SSQASSYFIDAATVDDSGEYRCQTNLSTLSDPVQLEVHIGWLLLQAPRWVFKEEDPIHLRCHSWKNTALHKV
TYLQNGKGRKYFHHNSDFYIPKATLKDSGSYFCRGLVGSKNVSSETVNITITQGLAVSTISSFFPPGYQIYI
WAPLAGTCGVLLLSLVITLYCKKKYSSSVHDPNGEYMFMRAVNTAKKSRLTDVTLRVKFSRSADAPAYQQGQ
NQLYNELNLGRREEYDVLDKRRGRDPEMGGKPRRKNPQEGLYNELQKDKMAEAYSEIGMKGERRRGKGHDGL
YQGLSTATKDTYDALHMQALPPR

SEQ ID NO:72:

*MALPVTALLLPLALLLHAARP*GMRTEDLPKAVVFLEPQWYRVLEKDSVTLKCQGAYSPEDNSTQWFHNESLI
SSQASSYFIDAATVDDSGEYRCQTNLSTLSDPVQLEVHIGWLLLQAPRWVFKEEDPIHLRCHSWKNTALHKV
TYLQNGKGRKYFHHNSDFYIPKATLKDSGSYFCRGLVGSKNVSSETVNITITQGLAVSTISSFFPPGYQIYI
WAPLAGTCGVLLLSLVITLYCRRDQRLPPDAHKPPGGGSFRTPIQEEQADAHSTLAKIRVKFSRSADAPAYQ
QGQNQLYNELNLGRREEYDVLDKRRGRDPEMGGKPRRKNPQEGLYNELQKDKMAEAYSEIGMKGERRRGKGH
DGLYQGLSTATKDTYDALHMQALPPR

# SEQ ID NO:73:

*MALPVTALLLPLALLLHAARP*GMRTEDLPKAVVFLEPQWYRVLEKDSVTLKCQGAYSPEDNSTQWFHNESLI
SSQASSYFIDAATVDDSGEYRCQTNLSTLSDPVQLEVHIGWLLLQAPRWVFKEEDPIHLRCHSWKNTALHKV
TYLQNGKGRKYFHHNSDFYIPKATLKDSGSYFCRGLVGSKNVSSETVNITITQGLAVSTISSFFPPGYQFAC
DIYIWAPLAGTCGVLLLSLVITLYCKRGRKKLLYIFKQPFMRPVQTTQEEDGCSCRFPEEEEGGCELRVKFS
RSADAPAYQQGQNQLYNELNLGRREEYDVLDKRRGRDPEMGGKPRRKNPQEGLYNELQKDKMAEAYSEIGMK
GERRRGKGHDGLYQGLSTATKDTYDALHMQALPPR

SEQ ID NO:74:

*MALPVTALLLPLALLLHAARP*GMRTEDLPKAVVFLEPQWYRVLEKDSVTLKCQGAYSPEDNSTQWFHNESLI
SSQASSYFIDAATVDDSGEYRCQTNLSTLSDPVQLEVHIGWLLLQAPRWVFKEEDPIHLRCHSWKNTALHKV
TYLQNGKGRKYFHHNSDFYIPKATLKDSGSYFCRGLVGSKNVSSETVNITITQGLAVSTISSFFPPGYQFAC
DIYIWAPLAGTCGVLLLSLVITLYCRSKRSRLLHSDYMNMTPRRPGPTRKHYQPYAPPRDFAAYRSRVKFSR
SADAPAYQQGQNQLYNELNLGRREEYDVLDKRRGRDPEMGGKPRRKNPQEGLYNELQKDKMAEAYSEIGMKG
ERRRGKGHDGLYQGLSTATKDTYDALHMQALPPR

SEQ ID NO:75:

*MALPVTALLLPLALLLHAARP*GMRTEDLPKAVVFLEPQWYRVLEKDSVTLKCQGAYSPEDNSTQWFHNESLI
SSQASSYFIDAATVDDSGEYRCQTNLSTLSDPVQLEVHIGWLLLQAPRWVFKEEDPIHLRCHSWKNTALHKV
TYLQNGKGRKYFHHNSDFYIPKATLKDSGSYFCRGLVGSKNVSSETVNITITQGLAVSTISSFFPPGYQTTT
PAPRPPTPAPTIASQPLSLRPEACRPAAGGAVHTRGLDFACDFWVLVVVGGVLACYSLLVTVAFIIFWVRSK
RSRLLHSDYMNMTPRRPGPTRKHYQPYAPPRDFAAYRSRVKFSRSADAPAYQQGQNQLYNELNLGRREEYDV
LDKRRGRDPEMGGKPRRKNPQEGLYNELQKDKMAEAYSEIGMKGERRRGKGHDGLYQGLSTATKDTYDALHM
QALPPR

**SEQ ID NO:76:**

*MALPVTALLLPLALLLHAARP*GMRTEDLPKAVVFLEPQWYRVLEKDSVTLKCQGAYSPEDNSTQWFHNESLI
SSQASSYFIDAATVDDSGEYRCQTNLSTLSDPVQLEVHIGWLLLQAPRWVFKEEDPIHLRCHSWKNTALHKV
TYLQNGKGRKYFHHNSDFYIPKATLKDSGSYFCRGLVGSKNVSSETVNITITQGLAVSTISSFFPPGYQKSN
GTIIHVKGKHLCPSPLFPGPSKPFWVLVVVGGVLACYSLLVTVAFIIFWVRSKRSRLLHSDYMNMTPRRPGP
TRKHYQPYAPPRDFAAYRSRVKFSRSADAPAYQQGQNQLYNELNLGRREEYDVLDKRRGRDPEMGGKPRRKN
PQEGLYNELQKDKMAEAYSEIGMKGERRRGKGHDGLYQGLSTATKDTYDALHMQALPPR

**SEQ ID NO:77:**

*MALPVTALLLPLALLLHAARP*GMRTEDLPKAVVFLEPQWYRVLEKDSVTLKCQGAYSPEDNSTQWFHNESLI
SSQASSYFIDAATVDDSGEYRCQTNLSTLSDPVQLEVHIGWLLLQAPRWVFKEEDPIHLRCHSWKNTALHKV
TYLQNGKGRKYFHHNSDFYIPKATLKDSGSYFCRGLVGSKNVSSETVNITITQGLAVSTISSFFPPGYQGKH
LCPSPLFPGPSKPFWVLVVVGGVLACYSLLVTVAFIIFWVRSKRSRLLHSDYMNMTPRRPGPTRKHYQPYAP
PRDFAAYRSRVKFSRSADAPAYQQGQNQLYNELNLGRREEYDVLDKRRGRDPEMGGKPRRKNPQEGLYNELQ
KDKMAEAYSEIGMKGERRRGKGHDGLYQGLSTATKDTYDALHMQALPPR

**SEQ ID NO:78:**

*MALPVTALLLPLALLLHAARP*GMRTEDLPKAVVFLEPQWYRVLEKDSVTLKCQGAYSPEDNSTQWFHNESLI
SSQASSYFIDAATVDDSGEYRCQTNLSTLSDPVQLEVHIGWLLLQAPRWVFKEEDPIHLRCHSWKNTALHKV
TYLQNGKGRKYFHHNSDFYIPKATLKDSGSYFCRGLVGSKNVSSETVNITITQGLAVSTISSFFPPGYQFWV
LVVVGGVLACYSLLVTVAFIIFWVRSKRSRLLHSDYMNMTPRRPGPTRKHYQPYAPPRDFAAYRSRVKFSRS
ADAPAYQQGQNQLYNELNLGRREEYDVLDKRRGRDPEMGGKPRRKNPQEGLYNELQKDKMAEAYSEIGMKGE
RRRGKGHDGLYQGLSTATKDTYDALHMQALPPR

**SEQ ID NO:79:**

*MALPVTALLLPLALLLHAARP*GMRTEDLPKAVVFLEPQWYRVLEKDSVTLKCQGAYSPEDNSTQWFHNESLI
SSQASSYFIDAATVDDSGEYRCQTNLSTLSDPVQLEVHIGWLLLQAPRWVFKEEDPIHLRCHSWKNTALHKV
TYLQNGKGRKYFHHNSDFYIPKATLKDSGSYFCRGLVGSKNVSSETVNITITQGLAVSTISSFFPPGYQTTT
PAPRPPTPAPTIASQPLSLRPEACRPAAGGAVHTRGLDFACDIYIWAPLAGTCGVLLLSLVITLYCKRGRKK
LLYIFKQPFMRPVQTTQEEDGCSCRFPEEEEGGCELRVKFSRSADAPAYQQGQNQLYNELNLGRREEYDVLD
KRRGRDPEMGGKPRRKNPQEGLYNELQKDKMAEAYSEIGMKGERRRGKGHDGLYQGLSTATKDTYDALHMQA
LPPR

**SEQ ID NO:80:**

*MALPVTALLLPLALLLHAARP*GMRTEDLPKAVVFLEPQWYRVLEKDSVTLKCQGAYSPEDNSTQWFHNESLISSQ
ASSYFIDAATVDDSGEYRCQTNLSTLSDPVQLEVHIGWLLLQAPRWVFKEEDPIHLRCHSWKNTALHKVTYLQNG
KGRKYFHHNSDFYIPKATLKDSGSYFCRGLVGSKNVSSETVNITITQGLAVSTISSFFPPGYQIEVMYPPPYLDN
EKSNGTIIHVKGKHLCPSPLFPGPSKPIYIWAPLAGTCGVLLLSLVITLYCRSKRSRLLHSDYMNMTPRRPGPTR
KHYQPYAPPRDFAAYRSRVKFSRSADAPAYQQGQNQLYNELNLGRREEYDVLDKRRGRDPEMGGKPRRKNPQEGL
YNELQKDKMAEAYSEIGMKGERRRGKGHDGLYQGLSTATKDTYDALHMQALPPR

### H. *Examples of CAR polypeptides*

Exemplary CAR polypeptides for use with the methods and compositions described herein may be found, for example, in the instant description and figures or as those known in the art. The CAR polypeptides described herein may comprise an extracellular domain comprising a single-chain antibody fragment (scFv) with binding affinity and specificity for an antigen of interest (e.g., those listed in Table 3 above), a transmembrane domain, and a CD3ζ cytoplasmic signaling domain. In some embodiments, the CAR polypeptides may further include one or more co-stimulatory signaling domains, one of which may be a CD28 co-stimulatory signaling domain or a 4-1BB co-stimulatory signaling domain. The CAR polypeptides are configured such that, when expressed on a host cell, the extracellular antigen-binding domain is located extracellularly for binding to a target molecule and the CD3ζ cytoplasmic signaling domain. The co-stimulatory signaling domain may be located in the cytoplasm for triggering activation and/or effector signaling.

In some embodiments, a CAR polypeptide as described herein may comprise, from N-terminus to C-terminus, the extracellular antigen binding domain, the transmembrane domain, the optional one or more co-stimulatory domains (e.g., a CD28 co-stimulatory domain, a 4-1BB co-stimulatory signaling domain, an OX40 co-stimulatory signaling domain, a CD27 co-stimulatory signaling domain, or an ICOS co-stimulatory signaling domain), and the CD3ζ cytoplasmic signaling domain.

Alternatively or in addition, the CAR polypeptides described herein may contain two or more co-stimulatory signaling domains, which may link to each other or be separated by the cytoplasmic signaling domain. The extracellular antigen binding domain, transmembrane domain, optional co-stimulatory signaling domain(s), and cytoplasmic signaling domain in a CAR polypeptide may be linked to each other directly, or via a peptide linker. In some embodiments, any of the CAR polypeptides described herein may comprise a signal sequence at the N-terminus.

Table 5 provides exemplary CAR polypeptides described herein. These exemplary constructs have, from N-terminus to C-terminus in order, the signal sequence, the antigen binding domain (e.g., a scFv fragment targeting an antigen such as a tumor antigen or a pathogenic antigen), the hinge domain, and the transmembrane, while the positions of the optional co-stimulatory domain and the cytoplasmic signaling domain can be switched.

**Table 5: Exemplary Components of CAR polypeptides.**

| Signal Sequence | Extracellular domain (antigen binding) | Hinge domain | Transmembrane domain | Co-stimulatory domain | Cytoplasmic Signaling domain |
|---|---|---|---|---|---|
| CD8α | scFv (e.g., anti-GPC3 scFv) | CD8 | CD8 | 4-1BB | CD3ζ |
| CD8α | scFv (e.g., anti-GPC3 scFv) | CD28 | CD28 | CD28 | CD3ζ |

Amino acid sequences of the example CAR polypeptides are provided below (signal sequence italicized).

**SEQ ID NO:104:**

*MALPVTALLLPLALLLHAARP*DVVMTQSPLSLPVTPGEPASISCRSSQSLVHSNRNTYLHWYLQKPGQSPQLLIY
KVSNRFSGVPDRFSGSGSGTDFTLKISRVEAEDVGVYYCSQNTHVPPTFGQGTKLEIKRGGGGSGGGGSGGGGSQ
VQLVQSGAEVKKPGASVKVSCKASGYTFTDYEMHWVRQAPGQGLEWMGALDPKTGDTAYSQKFKGRVTLTADKST
STAYMELSSLTSEDTAVYYCTRFYSYTYWGQGTLVTVSSTTTPAPRPPTPAPTIASQPLSLRPEACRPAAGGAVH
TRGLDFACDIYIWAPLAGTCGVLLLSLVITLYCKRGRKKLLYIFKQPFMRPVQTTQEEDGCSCRFPEEEEGGCEL
RVKFSRSADAPAYQQGQNQLYNELNLGRREEYDVLDKRRGRDPEMGGKPRRKNPQEGLYNELQKDKMAEAYSEIG
MKGERRRGKGHDGLYQGLSTATKDTYDALHMQALPPR

**SEQ ID NO:105:**

*MALPVTALLLPLALLLHAARP*DVVMTQSPLSLPVTPGEPASISCRSSQSLVHSNRNTYLHWYLQKPGQSPQL
LIYKVSNRFSGVPDRFSGSGSGTDFTLKISRVEAEDVGVYYCSQNTHVPPTFGQGTKLEIKRGGGGSGGGGS
GGGGSQVQLVQSGAEVKKPGASVKVSCKASGYTFTDYEMHWVRQAPGQGLEWMGALDPKTGDTAYSQKFKGR
VTLTADKSTSTAYMELSSLTSEDTAVYYCTRFYSYTYWGQGTLVTVSSIEVMYPPPYLDNEKSNGTIIHVKG
KHLCPSPLFPGPSKPFWVLVVVGGVLACYSLLVTVAFIIFWVRSKRSRLLHSDYMNMTPRRPGPTRKHYQPY
APPRDFAAYRSRVKFSRSADAPAYQQGQNQLYNELNLGRREEYDVLDKRRGRDPEMGGKPRRKNPQEGLYNE
LQKDKMAEAYSEIGMKGERRRGKGHDGLYQGLSTATKDTYDALHMQALPPR

### III. Immune Cells Expressing Glucose Importation Polypeptides and Optionally Chimeric Receptor Polypeptides

Provided herein are genetically engineered host cells (e.g., immune cells such as T cells or NK cells) expressing one or more of the glucose importation polypeptides as described herein. The genetically engineered host cells may further express a chimeric receptor polypeptides (e.g., ACTR-expressing cells, e.g., ACTR T cells or CAR-expressing T cells) as also described herein.

Alternatively, the genetically engineered host cells disclosed herein may not express any chimeric receptor polypeptides. In some embodiments, the genetically engineered immune cells, which may overly express one or more glucose importation polypeptides as disclosed herein, may be derived from tumor-infiltrating lymphocytes (TILs). Overexpression of the glucose importation polypeptides may enhance the anti-tumor activity or the TILs in tumor microenvironment. Alternatively or in addition, the genetically engineered immune cells may be T cells, which may further have genetically engineered T cell receptors. The TILs and/or genetically modified TCRs may target peptide-MHC complex, in which the peptide may be derived from a pathogen, a tumor antigen, or an auto-antigen. Some examples are provided in Table 6 below.

Any of the CAR constructs disclosed herein or an antibody to be co-used with ACTR T cells may also target any of the peptide in such peptide/MHC complex.

**Table 6. Exemplary Peptide-MHC Targets**

| Targets | Indications |
|---|---|
| NY-ESO-1 | Sarcoma, MM |
| MAGE-A10 | NSCLC, Bladder, HNSCC |
| MAGE-A4 | Sarcomas, others |
| PMEL | Melanoma |
| WT-1 | Ovarian |
| AFP | HCC |
| HPV-16 E6 | Cervical |
| HPV-16 E7 | Cervical |

In some embodiments, the host cells are immune cells, such as T cells or NK cells. In some embodiments, the immune cells are T cells. For example, the T cells can be CD4+ helper cells or CD8+ cytotoxic cells, or a combination thereof. Alternatively or in addition, the T cells can be suppressive T cells such as $T_{reg}$ cells. In some embodiments, the immune cells are NK cells. In other embodiments, the immune cells can be established cell lines, for example, NK-92 cells. In some examples, the immune cells can be a mixture of different types of T cells and/or NK cells as known in the art. For example, the immune cells can be a population of immune cells isolated from a suitable donor (*e.g.*, a human patient). See disclosures below.

In some instances, the glucose importation polypeptide to be introduced into the host cells is identical to an endogenous protein of the host cell. Introducing additional copies of the coding sequences of the glucose importation polypeptide into the host cell would enhance the expression level of the polypeptide (*i.e.*, over-express) as relative to the native counterpart. In some instances, the glucose importation polypeptide to be introduced into the host cells is heterologous to the host cell, *i.e.*, does not exist or not expressed in the host cell. Such a heterologous glucose importation polypeptide may be a naturally-occurring protein not expressed in the host cell in nature (*e.g.*, from a different species). Alternatively, the heterologous glucose importation polypeptide may be a variant of a native protein, such as those described herein. In some examples, the exogenous (*i.e.*, not native to the host cells) copy of the coding nucleic acid may exist extrachromosomally. In other examples, the exogenous copy of the coding sequence may be integrated into the chromosome of the host cell, and may be located at a site that is different from the native loci of the endogenous gene.

Such genetically engineered host cells have an enhanced capacity of taking glucose from the environment, for example, low glucose environment and thus exhibit better growth and/or bioactivities under low glucose conditions. The genetically engineered cells, when expressing a chimeric receptor polypeptide as disclosed herein, can recognize and inhibit target cells, either directly (*e.g.,* by CAR-expressing immune cells) or via an Fc-containing therapeutic agents such as an anti-tumor antibodies (*e.g.*, by ACTR-expressing immune cells). Given their expected high proliferation rate, bioactivity, and/or survival rate in low glucose environments, the genetically engineered cells such as T cell and NK cells would be expected to have higher therapeutic efficacy as relative to ACTR-expressing or CAR-expressing T cells that do not express or express a lower level or less active form of the glucose importation polypeptide.

The population of immune cells can be obtained from any source, such as peripheral blood mononuclear cells (PBMCs), bone marrow, or tissues such as spleen, lymph node, thymus, stem cells, or tumor tissue. Alternatively, the immune cell population may be derived from stem cells, for example, hematopoietic stem cells and induced pluripotent stem cells (iPSCs). A source suitable for obtaining the type of host cells desired would be evident to one of skill in the art. In some embodiments, the population of immune cells is derived from PBMCs, which may be obtained from a patient (*e.g.*, a human patient) who needs the treatment described herein. The type of host cells desired (*e.g.*, T cells, NK cells, or T cells and NK cells) may be expanded within the population of cells obtained by co-incubating the cells with stimulatory molecules. As a non-limiting example, anti-CD3 and anti-CD28 antibodies may be used for expansion of T cells.

To construct the immune cells that express any of the glucose importation polypeptides and optionally the chimeric receptor polypeptide described herein, expression vectors for stable or transient expression of the glucose importation polypeptides and/or the chimeric receptor polypeptide may be created via conventional methods as described herein and introduced into immune host cells. For example, nucleic acids encoding the glucose importation polypeptides and/or the chimeric receptor polypeptides may be cloned into one or two suitable expression vectors, such as a viral vector in operable linkage to a suitable promoter. In some instances, each of the coding sequences for the chimeric receptor polypeptide and the glucose importation polypeptide are on two separate nucleic acid molecules and can be cloned into two separate vectors, which may be introduced into suitable host cells simultaneously or sequentially. Alternatively, the coding sequences for the chimeric receptor polypeptide and the glucose importation polypeptide are on one nucleic acid molecule and can be cloned into one vector. The coding sequences of the chimeric receptor polypeptide and the glucose importation polypeptide may be in operable linkage to two distinct promoters such that the expression of the two polypeptides is controlled by different promoters. Alternatively, the coding sequences of the chimeric receptor polypeptide and the glucose importation polypeptide may be in operable linkage to one promoter such that the expression of the two polypeptides is controlled by a single promoter. Suitable sequences may be inserted between the coding sequences of the two polypeptides so that two separate polypeptides can be translated from a single mRNA molecule. Such sequences, for example, IRES or ribosomal skipping site, are well known in the art. Additional descriptions are provided below.

The nucleic acids and the vector(s) may be contacted, under suitable conditions, with a restriction enzyme to create complementary ends on each molecule that can pair with each other and be joined with a ligase. Alternatively, synthetic nucleic acid linkers can be ligated to the termini of the nucleic acid encoding the glucose importation polypeptides and/or the chimeric receptor polypeptides. The synthetic linkers may contain nucleic acid sequences that correspond to a particular restriction site in the vector. The selection of expression vectors/plasmids/viral vectors would depend on the type of host cells for expression of the glucose importation polypeptides and/or the chimeric receptor polypeptides, but should be suitable for integration and replication in eukaryotic cells.

A variety of promoters can be used for expression of the glucose importation polypeptides and/or the chimeric receptor polypeptides described herein, including, without limitation, cytomegalovirus (CMV) intermediate early promoter, a viral LTR such as the *Rous sarcoma* virus LTR, HIV-LTR, HTLV-1 LTR, the simian virus 40 (SV40) early promoter, the human EF 1-alpha promoter, or herpes simplex tk virus promoter. Additional promoters for expression of the glucose importation polypeptides and/or the chimeric receptor polypeptides include any constitutively active promoter in an immune cell. Alternatively, any regulatable promoter may be used, such that its expression can be modulated within an immune cell.

Additionally, the vector may contain, for example, some or all of the following: a selectable marker gene, such as the neomycin gene or the kanamycin gene for selection of stable or transient transfectants in host cells; enhancer/promoter sequences from the immediate early gene of human CMV for high levels of transcription; intron sequences of the human EF 1-alpha gene; transcription termination and RNA processing signals from SV40 for mRNA stability; SV40 polyomavirus origins of replication and ColE1 for proper episomal replication; internal ribosome binding sites (IRESes), versatile multiple cloning sites; T7 and SP6 RNA promoters for *in vitro* transcription of sense and antisense RNA; a "suicide switch" or "suicide gene" which when triggered causes cells carrying the vector to die (*e.g.,* HSV thymidine kinase or an inducible caspase such as iCasp9), and reporter gene for assessing expression of the glucose importation polypeptides and/or the chimeric receptor polypeptide.

In one specific embodiment, such vectors also include a suicide gene. As used herein, the term "suicide gene" refers to a gene that causes the cell expressing the suicide gene to die. The suicide gene can be a gene that confers sensitivity to an agent, *e.g.*, a drug, upon the cell in which the gene is expressed, and causes the cell to die when the cell is contacted with or exposed to the agent. Suicide genes are known in the art (see, for example, Suicide Gene Therapy: Methods and Reviews, Springer, Caroline J. (Cancer Research UK Centre for Cancer Therapeutics at the Institute of Cancer Research, Sutton, Surrey, UK), Humana Press, 2004) and include, for example, the Herpes Simplex Virus (HSV) thymidine kinase (TK) gene, cytosine deaminase, purine nucleoside phosphorylase, nitroreductase, and caspases such as caspase 8.

Suitable vectors and methods for producing vectors containing transgenes are well known and available in the art. Examples of the preparation of vectors for expression of glucose importation polypeptides and/or chimeric receptor polypeptides can be found, for example, in US2014/0106449.

Any of the vectors comprising a nucleic acid sequence that encodes a glucose importation polypeptide and/or a chimeric receptor polypeptide described herein is also within the scope of the present disclosure. Such a vector, or the sequence encoding a glucose importation polypeptides and/or a chimeric receptor polypeptide contained therein, may be delivered into host cells such as host immune cells by any suitable method. Methods of delivering vectors to immune cells are well known in the art and may include DNA electroporation, RNA electroporation, transfection using reagents such as liposomes, or viral transduction (*e.g.*, retroviral transduction such as lentiviral transduction).

In some embodiments, the vectors for expression of the glucose importation polypeptides and/or the chimeric receptor polypeptides are delivered to host cells by viral transduction (*e.g.*, retroviral transduction such as lentiviral transduction). Exemplary viral methods for delivery include, but are not limited to, recombinant retroviruses (see, *e.g.,* PCT Publication Nos. WO 90/07936; WO 94/03622; WO 93/25698; WO 93/25234; WO 93/11230; WO 93/10218; and WO 91/02805; U.S. Pat. Nos. 5,219,740 and 4,777,127; GB Patent No. 2,200,651; and EP Patent No. 0 345 242), alphavirus-based vectors, and adeno-associated virus (AAV) vectors (see, *e.g.,* PCT Publication Nos. WO 94/12649, WO 93/03769; WO 93/19191; WO 94/28938; WO 95/11984; and WO 95/00655). In some embodiments, the vectors for expression of the glucose importation polypeptides and/or the chimeric receptor polypeptides are retroviruses. In some embodiments, the vectors for expression of the glucose importation polypeptides and/or the chimeric receptor polypeptides are lentiviruses.

Examples of references describing retroviral transduction include Anderson et al., U.S. Pat. No. 5,399,346; Mann et al., Cell 33:153 (1983); Temin et al., U.S. Pat. No. 4,650,764; Temin et al., U.S. Pat. No. 4,980,289; Markowitz et al., J. Virol. 62:1120 (1988); Temin et al., U.S. Pat. No. 5,124,263; International Patent Publication No. WO 95/07358, published Mar. 16, 1995, by Dougherty et al.; and Kuo et al., Blood 82:845 (1993). International Patent Publication No. WO 95/07358 describes high efficiency transduction of primary B lymphocytes. See also WO 2016/040441 A1.

In examples in which the vectors encoding glucose importation polypeptides and/or chimeric receptor polypeptides are introduced to the host cells using a viral vector, viral particles that are capable of infecting the immune cells and carry the vector may be produced by any method known in the art and can be found, for example in PCT Application No. WO 1991/002805 A2, WO 1998/009271 A1, and U.S. Patent 6,194,191. The viral particles are harvested from the cell culture supernatant and may be isolated and/or purified prior to contacting the viral particles with the immune cells.

In some embodiments, RNA molecules encoding any of the glucose importation polypeptides and/or the chimeric receptor polypeptides as described herein may be prepared by a conventional method (*e.g.*, *in vitro* transcription) and then introduced into suitable host cells, *e.g.,* those described herein, via known methods, *e.g.,* Rabinovich et al., Human Gene Therapy 17:1027-1035.

In some instances, the nucleic acid encoding a glucose importation polypeptide and the nucleic acid encoding a suitable chimeric receptor polypeptide may be cloned into separate expression vectors, which may be introduced into suitable host cells concurrently or sequentially. For example, an expression vector (or an RNA molecule) for expressing the glucose importation polypeptide may be introduced into host cells first and transfected host cells expressing the glucose importation polypeptide may be isolated and cultured *in vitro.* An expression vector (or an RNA molecule) for expressing a suitable chimeric receptor polypeptide can then introduced into the host cells that express the glucose importation polypeptide and transfected cells expressing both polypeptides can be isolated. In another example, expression vectors (or RNA molecules) each for expressing the glucose importation polypeptide and the chimeric receptor polypeptide can be introduced into host cells simultaneously and transfected host cells expressing both polypeptides can be isolated *via* routine methodology.

In other instances, the nucleic acid encoding the glucose importation polypeptide and the nucleic acid encoding the chimeric receptor polypeptide may be cloned into the same expression vector. Polynucleotides (including vectors in which such polynucleotides are operably linked to at least one regulatory element) for expression of the chimeric receptor polypeptide and glucose importation polypeptide are also within the scope of the present disclosure. Non-limiting examples of useful vectors of the disclosure include viral vectors such as, *e.g.*, retroviral vectors including gamma retroviral vectors, adeno-associated virus vectors (AAV vectors), and lentiviral vectors.

In some instances, the nucleic acid(s) encoding the glucose importation polypeptide and/or the chimeric receptor polypeptide may be delivered into host cells via transposons. In some instances, the encoding nucleic acid(s) may be delivered into host cells *via* gene editing, for example, by CRISPR, TALEN, ZFN, or meganucleases.

In some instances, the nucleic acid described herein may comprise two coding sequences, one encoding a chimeric receptor polypeptide as described herein, and the other encoding a polypeptide capable of enhancing glucose importation (*i.e.,* a glucose importation polypeptide polypeptide). The nucleic acid comprising the two coding sequences described herein may be configured such that the polypeptides encoded by the two coding sequences can be expressed as independent (and physically separate) polypeptides. To achieve this goal, the nucleic acid described herein may contain a third nucleotide sequence located between the first and second coding sequences. This third nucleotide sequence may, for example, encode a ribosomal skipping site. A ribosomal skipping site is a sequence that impairs normal peptide bond formation. This mechanism results in the translation of additional open reading frames from one messenger RNA. This third nucleotide sequence may, for example, encode a P2A, T2A, or F2A peptide (see, for example, Kim et al., PLoS One. 2011;6(4):e18556). As a non-limiting example, an exemplary P2A peptide may have the amino acid sequence of ATNFSLLKQAGDVEENPGP SEQ ID NO.: 102.

In another embodiment, the third nucleotide sequence may encode an internal ribosome entry site (IRES). An IRES is an RNA element that allows translation initiation in an end-independent manner, also permitting the translation of additional open reading frames from one messenger RNA. Alternatively, the third nucleotide sequence may encode a second promoter controlling the expression of the second polypeptide. The third nucleotide sequence may also encode more than one ribosomal skipping sequence, IRES sequence, additional promoter sequence, or a combination thereof.

The nucleic acid may also include additional coding sequences (including, but not limited to, fourth and fifth coding sequences) and may be configured such that the polypeptides encoded by the additional coding sequences are expressed as further independent and physically separate polypeptides. To this end, the additional coding sequences may be separated from other coding sequences by one or more nucleotide sequences encoding one or more ribosomal skipping sequences, IRES sequences, or additional promoter sequences.

In some examples, the nucleic acid (*e.g.*, an expression vector or an RNA molecule as described herein) may comprise coding sequences for both the glucose importation polypeptide (*e.g.*, those described herein) and a suitable ACTR polypeptide, the two coding sequences, in any order, being separated by a third nucleotide sequence coding for a P2A peptide (*e.g.*, ATNFSLLKQAGDVEENPGP; SEQ ID NO: 102). As a result, two separate polypeptides, the glucose importation polypeptide and the ACTR) can be produced from such a nucleic acid, wherein the P2A portion ATNFSLLKQAGDVEENPG (SEQ ID NO: 103) is linked to the upstream polypeptide (encoded by the upstream coding sequence) and residue P from the P2A peptide is linked to the downstream polypeptide (encoded by the downstream coding sequence). In some examples, the ACTR polypeptide is the upstream one and the glucose importation polypeptide is the downstream one. In other examples, the glucose importation polypeptide is the upstream one and the ACTR polypeptide is the downstream one.

In some examples, the nucleic acid described above may further encode a linker (*e.g.*, a GSG linker) between two segments of the encoded sequences, for example, between the upstream polypeptide and the P2A peptide.

In specific examples, the nucleic acid described herein is configured such that it expresses two separate polypeptides in the host cell to which the nucleic acid is transfected: (i) the first polypeptide that contains, from the N-terminus to the C-terminus, a suitable ACTR (*e.g.*, any of SEQ ID NOs: 1-80 described herein, for example, SEQ ID NO: 1 or SEQ ID NO:57), a peptide linker (*e.g.*, the GSG linker), and the ATNFSLLKQAGDVEENPG (SEQ ID NO:103) segment derived from the P2A peptide; and (ii) a second polypeptide that contains, from the N-terminus to the C-terminus, the P residue derived from the P2A peptide and the glucose importation polypeptide (*e.g.*, any of SEQ ID NOs:81-90).

In other specific examples, the nucleic acid described herein is configured such that it expresses two separate polypeptides in the host cell to which the nucleic acid is transfected: (i) the first polypeptide that contains, from the N-terminus to the C-terminus, a suitable CAR (*e.g.*, any of SEQ ID NOs: 104-105 described herein, for example, SEQ ID NO: 104 or SEQ ID NO: 105), a peptide linker (*e.g.*, the GSG linker), and the ATNFSLLKQAGDVEENPG (SEQ ID NO: 103) segment derived from the P2A peptide; and (ii) a second polypeptide that contains, from the N-terminus to the C-terminus, the P residue derived from the P2A peptide and the glucose importation polypeptide (*e.g.*, any of SEQ ID NOs:81-90).

In some instances, additional polypeptides of interest may also be introduced into the host immune cells.

Following introduction into the host cells a vector encoding any of the glucose importation polypeptides and/or the chimeric receptor polypeptides provided herein, or the nucleic acid encoding the chimeric receptor and/or glucose importation polypeptide (*e.g.*, an RNA molecule), the cells may be cultured under conditions that allow for expression of the glucose importation polypeptides and/or the chimeric receptor polypeptide. In examples in which the nucleic acid encoding the glucose importation polypeptides and/or the chimeric receptor polypeptide is regulated by a regulatable promoter, the host cells may be cultured in conditions wherein the regulatable promoter is activated. In some embodiments, the promoter is an inducible promoter and the immune cells are cultured in the presence of the inducing molecule or in conditions in which the inducing molecule is produced. Determining whether the glucose importation polypeptide and/or the chimeric receptor polypeptide is expressed will be evident to one of skill in the art and may be assessed by any known method, for example, detection of the glucose importation polypeptide and/or the chimeric receptor polypeptide-encoding mRNA by quantitative reverse transcriptase PCR (qRT-PCR) or detection of the glucose importation polypeptide and/or the chimeric receptor polypeptide protein by methods including Western blotting, fluorescence microscopy, and flow cytometry.

Alternatively, expression of the chimeric polypeptide may take place *in vivo* after the immune cells are administered to a subject. As used herein, the term "subject" refers to any mammal such as a human, monkey, mouse, rabbit, or domestic mammal. For example, the subject may be a primate. In a preferred embodiment, the subject is human.

Alternatively, expression of a glucose importation polypeptide and/or a chimeric receptor polypeptide in any of the immune cells disclosed herein can be achieved by introducing RNA molecules encoding the glucose importation polypeptides and/or the chimeric receptor polypeptides. Such RNA molecules can be prepared by *in vitro* transcription or by chemical synthesis. The RNA molecules can then be introduced into suitable host cells such as immune cells (*e.g.*, T cells, NK cells, or both T cells and NK cells) by, *e.g.*, electroporation. For example, RNA molecules can be synthesized and introduced into host immune cells following the methods described in Rabinovich et al., Human Gene Therapy, 17:1027-1035 and WO WO2013/040557.

In certain embodiments, a vector(s) or RNA molecule(s) comprising the glucose importation polypeptide and/or the chimeric receptor polypeptide may be introduced to the host cells or immune cells *in vivo.* As a non-limiting example, this may be accomplished by administering a vector or RNA molecule encoding one or more glucose importation polypeptides and/or one or more chimeric receptor polypeptides described herein directly to the subject (*e.g.*, through intravenous administration), producing host cells comprising glucose importation polypeptides and/or chimeric receptor polypeptides *in vivo.*

Methods for preparing host cells expressing any of the glucose importation polypeptides and/or the chimeric receptor polypeptides described herein may also comprise activating the host cells *ex vivo.* Activating a host cell means stimulating a host cell into an activated state in which the cell may be able to perform effector functions (*e.g.*, cytotoxicity such as ADCC). Methods of activating a host cell will depend on the type of host cell used for expression of the glucose importation polypeptides and/or chimeric receptor polypeptides. For example, T cells may be activated *ex vivo* in the presence of one or more molecules including, but not limited to: an anti-CD3 antibody, an anti-CD28 antibody, IL-2, phytohemagglutinin, engineered artificial stimulatory cells or particles, or a combination thereof. The engineered artificial stimulatory cells may be artificial antigen-presenting cells as known in the art. See, *e.g.,* Neal et al., J. Immunol. Res. Ther. 2017, 2(1):68-79 and Turtle et al., Cancer J. 2010, 16(4):374-381.

In other examples, NK cells may be activated *ex vivo* in the presence of one or more molecules such as a 4-1BB ligand, an anti-4-1BB antibody, IL-15, an anti-IL-15 receptor antibody, IL-2, IL12, IL-21, K562 cells, and/or engineered artificial stimulatory cells or particles. In some embodiments, the host cells expressing any of the glucose importation polypeptides and/or the chimeric receptor polypeptides (ACTR-/CAR- and/or glucose importation polypeptide-expressing cells) described herein are activated *ex vivo* prior to administration to a subject. Determining whether a host cell is activated will be evident to one of skill in the art and may include assessing expression of one or more cell surface markers associated with cell activation, expression or secretion of cytokines, and cell morphology.

Methods for preparing host cells expressing any of the glucose importation polypeptides and/or the chimeric receptor polypeptides described herein may comprise expanding the host cells *ex vivo.* Expanding host cells may involve any method that results in an increase in the number of cells expressing glucose importation polypeptides and/or chimeric receptor polypeptides, for example, allowing the host cells to proliferate or stimulating the host cells to proliferate. Methods for stimulating expansion of host cells will depend on the type of host cell used for expression of the glucose importation polypeptides and/or the chimeric receptor polypeptides and will be evident to one of skill in the art. In some embodiments, the host cells expressing any of the glucose importation polypeptides and/or the chimeric receptor polypeptides described herein are expanded *ex vivo* prior to administration to a subject.

In some embodiments, the host cells expressing the glucose importation polypeptides and/or the chimeric receptor polypeptides are expanded and activated *ex vivo* prior to administration of the cells to the subject. Host cell activation and expansion may be used to allow integration of a viral vector into the genome and expression of the gene encoding a glucose importation polypeptide and/or a chimeric receptor polypeptide as described herein. If mRNA electroporation is used, no activation and/or expansion may be required, although electroporation may be more effective when performed on activated cells. In some instances, a glucose importation polypeptide and/or a chimeric receptor polypeptide is transiently expressed in a suitable host cell (*e.g.*, for 3-5 days). Transient expression may be advantageous if there is a potential toxicity and should be helpful in initial phases of clinical testing for possible side effects.

Any of the host cells expressing the glucose importation polypeptides and/or the chimeric receptor polypeptides may be mixed with a pharmaceutically acceptable carrier to form a pharmaceutical composition, which is also within the scope of the present disclosure.

The phrase "pharmaceutically acceptable", as used in connection with compositions of the present disclosure, refers to molecular entities and other ingredients of such compositions that are physiologically tolerable and do not typically produce untoward reactions when administered to a mammal (*e.g.*, a human). Preferably, as used herein, the term "pharmaceutically acceptable" means approved by a regulatory agency of the Federal or a state government or listed in the U.S. Pharmacopeia or other generally recognized pharmacopeia for use in mammals, and more particularly in humans. "Acceptable" means that the carrier is compatible with the active ingredient of the composition (*e.g.*, the nucleic acids, vectors, cells, or therapeutic antibodies) and does not negatively affect the subject to which the composition(s) are administered. Any of the pharmaceutical compositions to be used in the present methods can comprise pharmaceutically acceptable carriers, excipients, or stabilizers in the form of lyophilized formations or aqueous solutions.

Pharmaceutically acceptable carriers, including buffers, are well known in the art, and may comprise phosphate, citrate, and other organic acids; antioxidants including ascorbic acid and methionine; preservatives; low molecular weight polypeptides; proteins, such as serum albumin, gelatin, or immunoglobulins; amino acids; hydrophobic polymers; monosaccharides; disaccharides; and other carbohydrates; metal complexes; and/or nonionic surfactants. See, *e.g*. Remington: The Science and Practice of Pharmacy 20th Ed. (2000) Lippincott Williams and Wilkins, Ed. K. E. Hoover.

The pharmaceutical compositions of the disclosure may also contain one or more additional active compounds as necessary for the particular indication being treated and/or for the enhancement of ADCC, preferably those with complementary activities that do not adversely affect each other. Non-limiting examples of possible additional active compounds include, *e.g.,* IL-2 as well as various agents known in the field and listed in the discussion of combination treatments, below.

## IV. Therapeutic Applications of Genetically-Engineered Immune Cells

In one aspect, the invention provides a population of the genetically-engineered immune cells disclosed herein for use in inhibiting cells expressing a target antigen in a subject. This use may include immunotherapy against various disorders, for example, cancer, infectious diseases, and autoimmune diseases.

The references to methods of treatment in the following paragraphs of this description are to be interpreted as references to the compounds, pharmaceutical compositions and medicaments of the present invention for use in a method for treatment of a subject.

### (a) Combined Immunotherapy of Genetically Engineered Immune Cells Expressing ACTR Polypeptides and Fc-Containing Therapeutic Agents

The exemplary ACTR polypeptides of the present disclosure confer antibody-dependent cell cytotoxicity (ADCC) capacity to T lymphocytes and enhance ADCC in NK cells. When the receptor is engaged by an antibody bound to cells, it triggers T-cell activation, sustained proliferation and specific cytotoxicity against the bound cells.

The degree of affinity of CD16 for the Fc portion of Ig is a critical determinant of ADCC and thus to clinical responses to antibody immunotherapy. The CD16 with the V158 polymorphism which has a high binding affinity for Ig and mediates superior ADCC was selected as an example. Although the F158 receptor has lower potency than the V158 receptor in induction of T cell proliferation and ADCC, the F158 receptor may have lower *in vivo* toxicity than the V158 receptor making it useful in some clinical contexts.

The glucose importation polypeptides to be co-expressed with ACTR polypeptides in immune cells would facilitate cell-based immune therapy such as T-cell therapy or NK-cell therapy by allowing the cells to grow and/or function effectively in a low glucose environment. Antibody-directed cytotoxicity could be stopped whenever required by simple withdrawal of antibody administration. Clinical safety can be further enhanced by using mRNA electroporation to express the glucose importation polypeptides and/or the ACTR polypeptides transiently, to limit any potential autoimmune reactivity.

Thus, in one embodiment, the disclosure provides a population of the genetically engineered immune cells disclosed herein for use in a method for enhancing efficacy of an antibody-based immunotherapy of a cancer in a subject in need thereof, which subject is being treated with an Fc-containing therapeutic agent such as a therapeutic antibody, which can bind to antigen-expressing cells. The Fc-containing therapeutic agent contains an Fc portion, for example, a human or humanized Fc portion, which can be recognized and bound by the Fc-binding portion (*e.g.*, the extracellular domain of human CD16A) of the ACTR expressed on the engineered immune cells.

The methods described herein may comprise introducing into the subject a therapeutically effective amount an antibody and a therapeutically effective amount of the genetically engineered host cells such as immune cells (*e.g.*, T lymphocytes or NK cells), which co-express a glucose importation polypeptides and an ACTR polypeptide of the disclosure. The subject (e.g., a human patient such as a human cancer patient) has been treated or is being treating with an Fc-containing therapeutic agent specific to a target antigen. A target antigen may be any molecule that is associated with a disease or condition, including, but are not limited to, tumor antigens, pathogenic antigens (e.g., bacterial or viral), or antigens present on diseased cells, such as those described herein.

In the context of the present disclosure insofar as it relates to any of the disease conditions recited herein, the terms "treat", "treatment", and the like mean to relieve or alleviate at least one symptom associated with such condition, or to slow or reverse the progression of such condition. Within the meaning of the present disclosure, the term "treat" also denotes to arrest, delay the onset (*i.e.*, the period prior to clinical manifestation of a disease) and/or reduce the risk of developing or worsening a disease. For example, in connection with cancer the term "treat" may mean eliminate or reduce a patient's tumor burden, or prevent, delay or inhibit metastasis, *etc.*

As used herein the term "therapeutically effective" applied to dose or amount refers to that quantity of a compound or pharmaceutical composition that is sufficient to result in a desired activity upon administration to a subject in need thereof. Note that when a combination of active ingredients is administered (*e.g.*, a first pharmaceutical composition comprising an antibody, and a second pharmaceutical composition comprising a population of T lymphocytes or NK cells that express a glucose importation polypeptide and/or an antibody-coupled T-cell receptor (ACTR) construct), the effective amount of the combination may or may not include amounts of each ingredient that would have been effective if administered individually. Within the context of the present disclosure, the term "therapeutically effective" refers to that quantity of a compound or pharmaceutical composition that is sufficient to delay the manifestation, arrest the progression, relieve or alleviate at least one symptom of a disorder treated by the methods of the present disclosure.

Host cells (*e.g.*, immune cells such as T cells and NK cells) expressing glucose importation polypeptides and ACTR polypeptides described herein are useful for enhancing ADCC in a subject and/or for enhancing the efficacy of an antibody-based immunotherapy and/or for enhancing growth and/or proliferation of immune cells in a low-glucose environment. In some embodiments, the subject is a mammal, such as a human, monkey, mouse, rabbit, or domestic mammal. In some embodiments, the subject is a human. In some embodiments, the subject is a human cancer patient. In some embodiments, the subject has been treated or is being treated with any of the therapeutic antibodies described herein.

To practice the method described herein, an effective amount of the immune cells (NK cells and/or T lymphocytes) expressing any of the glucose importation polypeptides and the ACTR polypeptides described herein and an effective amount of an antibody, or compositions thereof may be administered to a subject in need of the treatment via a suitable route, such as intravenous administration. As used herein, an effective amount refers to the amount of the respective agent (*e.g.*, the NK cells and/or T lymphocytes expressing glucose importation polypeptides, ACTR polypeptides, antibodies, or compositions thereof) that upon administration confers a therapeutic effect on the subject. Determination of whether an amount of the cells or compositions described herein achieved the therapeutic effect would be evident to one of skill in the art. Effective amounts vary, as recognized by those skilled in the art, depending on the particular condition being treated, the severity of the condition, the individual patient parameters including age, physical condition, size, gender, sex, and weight, the duration of the treatment, the nature of concurrent therapy (if any), the specific route of administration and like factors within the knowledge and expertise of the health practitioner. In some embodiments, the effective amount alleviates, relieves, ameliorates, improves, reduces the symptoms, or delays the progression of any disease or disorder in the subject. In some embodiments, the subject is a human. In some embodiments, the subject in need of treatment is a human cancer patient. In some embodiments, the subject in need of treatment suffers from one or more pathogenic infections (*e.g.*, viral, bacterial, and/or fungal infections).

The methods of the disclosure may be used for treatment of any cancer or any pathogen. Specific non-limiting examples of cancers which can be treated by the methods of the disclosure include, for example, lymphoma, breast cancer, gastric cancer, neuroblastoma, osteosarcoma, lung cancer, skin cancer, prostate cancer, colorectal cancer, renal cell carcinoma, ovarian cancer, rhabdomyosarcoma, leukemia, mesothelioma, pancreatic cancer, head and neck cancer, retinoblastoma, glioma, glioblastoma, thyroid cancer, hepatocellular cancer, esophageal cancer, and cervical cancer. In certain embodiments, the cancer may be a solid tumor.

The methods of this disclosure may also be used for treating infectious diseases, which may be caused by bacterial infection, viral infection, or fungus infection. In such instances, the genetically engineered immune cells can be co-used with an Fc-containing therapeutic agent (*e.g.*, an antibody) that targets a pathogenic antigen (*e.g.*, an antigen associated with the bacterium, virus, or fungus that causes the infection). Specific non-limiting examples of pathogenic antigens include, but are not limited to, bacterial, viral, and/or fungal antigens. Some examples are provided below: influenza virus neuraminidase, hemagglutinin, or M2 protein, human respiratory syncytial virus (RSV) F glycoprotein or G glycoprotein, herpes simplex virus glycoprotein gB, gC, gD, or gE, Chlamydia MOMP or PorB protein, Dengue virus core protein, matrix protein, or glycoprotein E, measles virus hemagglutinin, herpes simplex virus type 2 glycoprotein gB, poliovirus I VP1, envelope glycoproteins of HIV 1, hepatitis B core antigen or surface antigen, diptheria toxin, Streptococcus 24M epitope, Gonococcal pilin, pseudorabies virus g50 (gpD), pseudorabies virus II (gpB), pseudorabies virus III (gpC), pseudorabies virus glycoprotein H, pseudorabies virus glycoprotein E, transmissible gastroenteritis glycoprotein 195, transmissible gastroenteritis matrix protein, or human hepatitis C virus glycoprotein E1 or E2.

In some embodiments, the immune cells are administered to a subject in an amount effective in enhancing ADCC activity by least 20% and/or by at least 2-fold, *e.g.*, enhancing ADCC by 50%, 80%, 100%, 2-fold, 5-fold, 10-fold, 20-fold, 50-fold, 100-fold, or more.

The immune cells are co-administered with an Fc-containing therapeutic agent such as a therapeutic antibody in order to target cells expressing the antigen to which the Fc-containing therapeutic agent binds. In some embodiments, more than one Fc-containing therapeutic agents, such as more than one antibodies can be co-used with the immune cells. Antibody-based immunotherapy may be used to treat, alleviate, or reduce the symptoms of any disease or disorder for which the immunotherapy is considered useful in a subject.

An antibody (interchangeably used in plural form) is an immunoglobulin molecule capable of specific binding to a target, such as a carbohydrate, polynucleotide, lipid, polypeptide, *etc.*, through at least one antigen recognition site, located in the variable region of the immunoglobulin molecule. As used herein, the term "antibody" encompasses not only intact (*i.e.*, full-length) polyclonal or monoclonal antibodies, but also antigen-binding fragments thereof which comprise an Fc region, mutants thereof, fusion proteins comprising an antibody portion, humanized antibodies, chimeric antibodies, diabodies, nanobodies, linear antibodies, multispecific antibodies (*e.g.*, bispecific antibodies) and any other modified configuration of the immunoglobulin molecule that comprises an antigen recognition site of the required specificity and an Fc region, including glycosylation variants of antibodies, amino acid sequence variants of antibodies, and covalently modified antibodies. An antibody includes an antibody of any class, such as IgD, IgE, IgG, IgA, or IgM (or sub-class thereof), and the antibody need not be of any particular class. Depending on the antibody amino acid sequence of the constant domain of its heavy chains, immunoglobulins can be assigned to different classes. There are five major classes of immunoglobulins: IgA, IgD, IgE, IgG, and IgM, and several of these may be further divided into subclasses (isotypes), *e.g.*, IgG1, IgG2, IgG3, IgG4, IgA1 and IgA2. The heavy-chain constant domains that correspond to the different classes of immunoglobulins are called alpha, delta, epsilon, gamma, and mu, respectively. The subunit structures and three-dimensional configurations of different classes of immunoglobulins are well known. The antibody for use in the present disclosure contains an Fc region recognizable by the co-used ACTR- and/or glucose importation polypeptide-expressing immune cells. The Fc region may be a human or humanized Fc region.

Any of the antibodies described herein can be either monoclonal or polyclonal. A "monoclonal antibody" refers to a homogenous antibody population and a "polyclonal antibody" refers to a heterogeneous antibody population. These two terms do not limit the source of an antibody or the manner in which it is made.

In one example, the antibody used in the methods described herein is a humanized antibody. Humanized antibodies refer to forms of non-human (*e.g.* murine) antibodies that are specific chimeric immunoglobulins, immunoglobulin chains, or antigen-binding fragments thereof that contain minimal sequence derived from non-human immunoglobulin. For the most part, humanized antibodies are human immunoglobulins (recipient antibody) in which residues from a complementary determining region (CDR) of the recipient are replaced by residues from a CDR of a non-human species (donor antibody) such as mouse, rat, or rabbit having the desired specificity, affinity, and capacity. In some instances, Fv framework region (FR) residues of the human immunoglobulin are replaced by corresponding non-human residues. Furthermore, the humanized antibody may comprise residues that are found neither in the recipient antibody nor in the imported CDR or framework sequences, but are included to further refine and optimize antibody performance. In general, the humanized antibody will comprise substantially all of at least one, and typically two, variable domains, in which all or substantially all of the CDR regions correspond to those of a non-human immunoglobulin and all or substantially all of the FR regions are those of a human immunoglobulin consensus sequence. The humanized antibody optimally also will comprise at least a portion of an immunoglobulin constant region or domain (Fc), typically that of a human immunoglobulin. Antibodies may have Fc regions modified as described in WO 99/58572. The antibodies used herein may be glycosylated (*e.g.*, fucosylated) or afucoslylated. Other forms of humanized antibodies have one or more CDRs (one, two, three, four, five, six) which are altered with respect to the original antibody, which are also termed one or more CDRs "derived from" one or more CDRs from the original antibody. Humanized antibodies may also involve affinity maturation.

In another example, the antibody described herein is a chimeric antibody, which can include a heavy constant region and a light constant region from a human antibody. Chimeric antibodies refer to antibodies having a variable region or part of variable region from a first species and a constant region from a second species. Typically, in these chimeric antibodies, the variable region of both light and heavy chains mimics the variable regions of antibodies derived from one species of mammals (*e.g.*, a non-human mammal such as mouse, rabbit, and rat), while the constant portions are homologous to the sequences in antibodies derived from another mammal such as a human. Amino acid modifications can be made in the variable region and/or the constant region.

The immune cells (*e.g.*, T lymphocytes and/or NK cells) expressing any of the glucose importation polypeptides and/or the ACTR polypeptides disclosed herein may be administered to a subject who has been treated or is being treated with an Fc-containing antibody. For example, the immune cells may be administered to a human subject simultaneously with an antibody. Alternatively, the immune cells may be administered to a human subject during the course of an antibody-based immunotherapy. In some examples, the immune cells and an antibody can be administered to a human subject at least 4 hours apart, *e.g.*, at least 12 hours apart, at least 1 day apart, at least 3 days apart, at least one week apart, at least two weeks apart, or at least one month apart.

The antibodies described herein specifically bind to the corresponding target antigen or an epitope thereof. An antibody that "specifically binds" to an antigen or an epitope is a term well understood in the art. A molecule is said to exhibit "specific binding" if it reacts more frequently, more rapidly, with greater duration and/or with greater affinity with a particular target antigen than it does with alternative targets. An antibody "specifically binds" to a target antigen or epitope if it binds with greater affinity, avidity, more readily, and/or with greater duration than it binds to other substances. For example, an antibody that specifically (or preferentially) binds to an antigen or an antigenic epitope therein is an antibody that binds this target antigen with greater affinity, avidity, more readily, and/or with greater duration than it binds to other antigens or other epitopes in the same antigen. It is also understood with this definition that, for example, an antibody that specifically binds to a first target antigen may or may not specifically or preferentially bind to a second target antigen. As such, "specific binding" or "preferential binding" does not necessarily require (although it can include) exclusive binding. In some examples, an antibody that "specifically binds" to a target antigen or an epitope thereof may not bind to other antigens or other epitopes in the same antigen.

An antibody as described herein has a suitable binding affinity for the target antigen (*e.g.*, any one of the targets described herein) or antigenic epitopes thereof as disclosed herein.

The antibodies for use in the immune therapy methods described herein may bind to (*e.g.*, specifically bind to) a target antigen of interest, or a specific region or an antigenic epitope therein. Exemplary target antigens of interest and exemplary antibodies specific to such are provided in Table 5 above.

The efficacy of an antibody-based immunotherapy may be assessed by any method known in the art and would be evident to a skilled medical professional. For example, the efficacy of the antibody-based immunotherapy may be assessed by survival of the subject or tumor or cancer burden in the subject or tissue or sample thereof. In some embodiments, the immune cells are administered to a subject in need of the treatment in an amount effective in enhancing the efficacy of an antibody-based immunotherapy by at least 20 % and/or by at least 2-fold, *e.g.*, enhancing the efficacy of an antibody-based immunotherapy by 50 %, 80 %, 100 %, 2-fold, 5-fold, 10-fold, 20-fold, 50-fold, 100-fold or more, as compared to the efficacy in the absence of the immune cells expressing the glucose importation polypeptide and/or the ACTR polypeptide and/or the antibody.

In any of the compositions or methods described herein, the immune cells (*e.g.*, NK and/or T cells) may be autologous to the subject, *i.e.,* the immune cells may be obtained from the subject in need of the treatment, genetically engineered for expression of the glucose importation polypeptide and/or the ACTR polypeptides, and then administered to the same subject. In one specific embodiment, prior to re-introduction into the subject, the autologous immune cells (*e.g.*, T lymphocytes or NK cells) are activated and/or expanded *ex vivo.* Administration of autologous cells to a subject may result in reduced rejection of the host cells as compared to administration of non-autologous cells.

Alternatively, the host cells are allogeneic cells, *i.e.,* the cells are obtained from a first subject, genetically engineered for expression of the glucose importation polypeptide and/or the ACTR polypeptide, and administered to a second subject that is different from the first subject but of the same species. For example, allogeneic immune cells may be derived from a human donor and administered to a human recipient who is different from the donor. In a specific embodiment, the T lymphocytes are allogeneic T lymphocytes in which the expression of the endogenous T cell receptor has been inhibited or eliminated. In one specific embodiment, prior to introduction into the subject, the allogeneic T lymphocytes are activated and/or expanded *ex vivo.* T lymphocytes can be activated by any method known in the art, *e.g.,* in the presence of anti-CD3/CD28, IL-2, phytohemagglutinin, engineered artificial stimulatory cells or particles, or a combination thereof.

NK cells can be activated by any method known in the art, *e.g.,* in the presence of one or more agents selected from the group consisting of CD137 ligand protein, CD137 antibody, IL-15 protein, IL-15 receptor antibody, IL-2 protein, IL-12 protein, IL-21 protein, K562 cell line, and/or engineered artificial stimulatory cells or particles. See, *e.g.,* U.S. Patents Nos. 7,435,596 and 8,026,097 for the description of useful methods for expanding NK cells. For example, NK cells used in the compositions or methods of the disclosure may be preferentially expanded by exposure to cells that lack or poorly express major histocompatibility complex I and/or II molecules and which have been genetically modified to express membrane bound IL-15 and 4-1BB ligand (CDI37L). Such cell lines include, but are not necessarily limited to, K562 [ATCC, CCL 243; Lozzio et al., Blood 45(3): 321-334 (1975); Klein et al., Int. J. Cancer 18: 421-431 (1976)], and the Wilms tumor cell line HFWT (Fehniger et al., Int Rev Immunol 20(3-4):503-534 (2001); Harada H, et al., Exp Hematol 32(7):614-621 (2004)), the uterine endometrium tumor cell line HHUA, the melanoma cell line HMV-II, the hepatoblastoma cell line HuH-6, the lung small cell carcinoma cell lines Lu-130 and Lu-134-A, the neuroblastoma cell lines NB 19 and N1369, the embryonal carcinoma cell line from testis NEC 14, the cervix carcinoma cell line TCO-2, and the bone marrow-metastasized neuroblastoma cell line TNB 1 [Harada, et al., Jpn. J. Cancer Res 93: 313-319 (2002)]. Preferably the cell line used lacks or poorly expresses both MHC I and II molecules, such as the K562 and HFWT cell lines. A solid support may be used instead of a cell line. Such support should preferably have attached on its surface at least one molecule capable of binding to NK cells and inducing a primary activation event and/or a proliferative response or capable of binding a molecule having such an affect thereby acting as a scaffold. The support may have attached to its surface the CD137 ligand protein, a CD137 antibody, the IL-15 protein or an IL-15 receptor antibody. Preferably, the support will have IL-15 receptor antibody and CD137 antibody bound on its surface.

In one embodiment of the described compositions or methods, introduction (or re-introduction) of T lymphocytes, NK cells, or T lymphocytes and NK cells to the subject is followed by administering to the subject a therapeutically effective amount of IL-2.

In accordance with the present disclosure, patients can be treated by infusing therapeutically effective doses of immune cells such as T lymphocytes or NK cells comprising a glucose importation polypeptide and/or an ACTR polypeptide of the disclosure in the range of about $10^5$ to $10^{10}$ or more cells per kilogram of body weight (cells/Kg). The infusion can be repeated as often and as many times as the patient can tolerate until the desired response is achieved. The appropriate infusion dose and schedule will vary from patient to patient, but can be determined by the treating physician for a particular patient. Typically, initial doses of approximately $10^6$ cells/Kg will be infused, escalating to $10^8$ or more cells/Kg. IL-2 can be co-administered to expand infused cells. The amount of IL-2 can about 1-5 x $10^6$ international units per square meter of body surface.

In some embodiments, the antibody is administered to the subject in one or more doses of about 100-500 mg, 500-1000 mg, 1000-1500 mg or 1500-2000 mg. In some embodiments, the antibody is administered to the subject in one or more doses of about 500 mg, about 600 mg, about 700 mg, about 800 mg, or about 900 mg. In some embodiments, the antibody is administered to the subject in one or more doses of about 1000 mg, about 1100 mg, about 1200 mg, about 1300 mg, about 1400 mg, about 1500 mg, about 1600 mg, about 1700 mg, or about 1800 mg. In some embodiments, the antibody is administered to the subject in one or more doses of about 1600 mg.

The term "about" or "approximately" means within an acceptable error range for the particular value as determined by one of ordinary skill in the art, which will depend in part on how the value is measured or determined, *i.e.,* the limitations of the measurement system. For example, "about" can mean within an acceptable standard deviation, per the practice in the art. Alternatively, "about" can mean a range of up to ± 20 %, preferably up to ± 10 %, more preferably up to ± 5 %, and more preferably still up to ± 1 % of a given value. Alternatively, particularly with respect to biological systems or processes, the term can mean within an order of magnitude, preferably within 2-fold, of a value. Where particular values are described in the application and claims, unless otherwise stated, the term "about" is implicit and in this context means within an acceptable error range for the particular value.

The particular dosage regimen, *i.e.,* dose, timing and repetition, used in the method described herein will depend on the particular subject and that subject's medical history. The appropriate dosage of the antibody used will depend on the type of cancer to be treated, the severity and course of the disease, previous therapy, the patient's clinical history and response to the antibody, and the discretion of the attending physician. The antibody can be administered to the patient at one time or over a series of treatments. The progress of the therapy of the disclosure can be easily monitored by conventional techniques and assays.

The administration of the antibody can be performed by any suitable route, including systemic administration as well as administration directly to the site of the disease (*e.g.*, to a tumor).

In some embodiments, the method involves administering the antibody to the subject in one dose. In some embodiments, the method involves administering the antibody to the subject in multiple dose (*e.g.*, at least 2, 3, 4, 5, 6, 7, or 8 doses). In some embodiments, the antibody is administered to the subject in multiple doses, with the first dose of the antibody administered to the subject about 1, 2, 3, 4, 5, 6, or 7 days prior to administration of the immune cells expressing the glucose importation polypeptide and/or the ACTR polypeptide. In some embodiments, the first dose of the antibody is administered to the subject between about 24-48 hours prior to the administration of the immune cells expressing the glucose importation polypeptide and/or the ACTR polypeptide.

In some embodiments, the antibody is administered to the subject prior to administration of the immune cells expressing the glucose importation polypeptide and/or the ACTR polypeptide and then subsequently about every two weeks. In some embodiments, the first two doses of the antibody are administered about one week (*e.g.*, about 6, 7, 8, or 9 days) apart. In certain embodiments, the third and following doses are administered about every two weeks.

In any of the embodiments described herein, the timing of the administration of the antibody is approximate and includes three days prior to and three days following the indicated day (*e.g.*, administration every three weeks encompasses administration on day 18, day 19, day 20, day 21, day 22, day 23, or day 24).

The efficacy of the compositions or methods described herein may be assessed by any method known in the art and would be evident to a skilled medical professional. For example, the efficacy of the antibody-based immunotherapy may be assessed by survival of the subject or cancer burden in the subject or tissue or sample thereof. In some embodiments, the antibody-based immunotherapy is assessed based on the safety or toxicity of the therapy (*e.g.*, administration of the antibody and the immune cells expressing the glucose importation polypeptides and/or the ACTR polypeptides) in the subject, for example by the overall health of the subject and/or the presence of adverse events or severe adverse events.

### (b) Immunotherapy Using the Genetically Engineered Immune Cells Expressing CAR Polypeptides

The genetically engineered immune cells described herein, co-expressing a glucose importation polypeptides and a CAR polypeptide are for use in immune therapy such as T-cell therapy or NK-cell therapy for inhibiting diseased cells expressing an antigen to which the CAR polypeptide targets, directly or indirectly (*e.g.*, *via* a therapeutic agent conjugated to a tag to which the CAR polypeptide binds). The glucose importation polypeptide co-expressed with a CAR polypeptide in immune cells would facilitate the cell-based immune therapy by allowing the cells to grow and/or function effectively in, *e.g.,* tumor microenvironment such as a low glucose environment. Clinical safety may be further enhanced by using mRNA electroporation to express the glucose importation polypeptides and/or the CAR polypeptides transiently, to limit any potential non-tumor specific reactivity.

The references to methods of treatment in the following paragraphs of this description are to be interpreted as references to the compounds, pharmaceutical compositions and medicaments of the present invention for use in a method for treatment of a subject.

The methods described herein may comprise introducing into the subject a therapeutically effective amount of genetically engineered host cells such as immune cells (*e.g.,* T lymphocytes or NK cells), which co-express a glucose importation polypeptides and a CAR polypeptide of the disclosure. The subject (*e.g.*, a human patient such as a human cancer patient) may additionally have been treated or is being treated with an anti-cancer or anti-infection therapy including, but not limited to, an anti-cancer therapeutic agent or anti-infection agent. The CAR has an antigen-binding domain that may bind any target antigen. Such a target antigen may be any molecule that is associated with a disease or condition, including, but are not limited to, tumor antigens, pathogenic antigens (*e.g.*, bacterial, fungal, or viral), or antigens present on diseased cells, such as those described herein.

Host cells (*e.g.*, immune cells such as T cells and NK cells) expressing glucose importation polypeptides and CAR polypeptides described herein are useful for inhibiting cells expressing a target antigen and/or for enhancing growth and/or proliferation of immune cells in a low-glucose environment. In some embodiments, the subject is a mammal, such as a human, monkey, mouse, rabbit, or domestic mammal. In some embodiments, the subject is a human. In some embodiments, the subject is a human cancer patient. In some embodiments, the subject has additionally been treated or is being treated with any of the therapeutic antibodies described herein.

To practice the method described herein, an effective amount of the immune cells (NK cells and/or T lymphocytes) expressing any of the glucose importation polypeptides and the CAR polypeptides described herein, or compositions thereof may be administered to a subject in need of the treatment via a suitable route, such as intravenous administration. As used herein, an effective amount refers to the amount of the respective agent (*e.g.,* the NK cells and/or T lymphocytes expressing glucose importation polypeptides, CAR polypeptides, or compositions thereof) that upon administration confers a therapeutic effect on the subject. Determination of whether an amount of the cells or compositions described herein achieved the therapeutic effect would be evident to one of skill in the art. Effective amounts vary, as recognized by those skilled in the art, depending on the particular condition being treated, the severity of the condition, the individual patient parameters including age, physical condition, size, gender, sex, and weight, the duration of the treatment, the nature of concurrent therapy (if any), the specific route of administration and like factors within the knowledge and expertise of the health practitioner. In some embodiments, the effective amount alleviates, relieves, ameliorates, improves, reduces the symptoms, or delays the progression of any disease or disorder in the subject. In some embodiments, the subject is a human. In some embodiments, the subject in need of treatment is a human cancer patient. In some embodiments, the subject in need of treatment suffers from one or more pathogenic infections (*e.g.*, viral, bacterial, and/or fungal infections).

The methods of the disclosure may be used for treatment of any cancer or any pathogen. Specific non-limiting examples of cancers are provided herein (see, e.g., disclosures in Section IV above).

The methods of this disclosure may also be used for treating infectious diseases, which may be caused by bacterial infection, viral infection, or fungus infection. In such instances, genetically engineered immune cells expressing a CAR polypeptide specific to a pathogenic antigen, (*e.g.*, an antigen associated with the bacterium, virus, or fungus that causes the infection) can be used to eliminate infected cells. Specific non-limiting examples of pathogenic antigens include, but are not limited to, bacterial, viral, and/or fungal antigens.

In some embodiments, the immune cells are administered to a subject in an amount effective in inhibiting cells expressing the target antigen by least 20% and/or by at least 2-fold, *e.g.*, inhibiting cells expressing the target antigen by 50%, 80%, 100%, 2-fold, 5-fold, 10-fold, 20-fold, 50-fold, 100-fold, or more.

Additional therapeutic agents (*e.g.*, antibody-based immunotherapeutic agents) may be used to treat, alleviate, or reduce the symptoms of any disease or disorder for which the therapeutic agent is considered useful in a subject.

The efficacy of the cell-based immunotherapy as described herein may be assessed by any method known in the art and would be evident to a skilled medical professional. For example, the efficacy of the cell-based immunotherapy may be assessed by survival of the subject or tumor or cancer burden in the subject or tissue or sample thereof. In some embodiments, the immune cells are administered to a subject in need of the treatment in an amount effective in enhancing the efficacy of an cell-based immunotherapy by at least 20% and/or by at least 2-fold, *e.g.*, enhancing the efficacy of an immunotherapy by 50%, 80%, 100%, 2-fold, 5-fold, 10-fold, 20-fold, 50-fold, 100-fold or more, as compared to the efficacy using the same type of immune cells that do not express the glucose importation polypeptide.

In any of the compositions or methods described herein, the immune cells (*e.g.*, NK and/or T cells) may be autologous to the subject, *i.e.,* the immune cells may be obtained from the subject in need of the treatment, genetically engineered for expression of the glucose importation polypeptide and/or the CAR polypeptides, and then administered to the same subject. Alternatively, the host cells are allogeneic cells, *i.e.,* the cells are obtained from a first subject, genetically engineered for expression of the glucose importation polypeptide and/or the CAR polypeptide, and administered to a second subject that is different from the first subject but of the same species. Either autologous or allogeneic immune cells may be activated and/or expanded *ex vivo* prior to the delivery to the subject. See descriptions in Section IV above.

In accordance with the present disclosure, patients can be treated by infusing therapeutically effective doses of immune cells such as T lymphocytes or NK cells comprising a glucose importation polypeptide and/or a CAR polypeptide of the disclosure in the range of about $10^5$ to $10^{10}$ or more cells per kilogram of body weight (cells/Kg). The infusion can be repeated as often and as many times as the patient can tolerate until the desired response is achieved. The appropriate infusion dose and schedule will vary from patient to patient, but can be determined by the treating physician for a particular patient. Typically, initial doses of approximately $10^6$ cells/Kg will be infused, escalating to $10^8$ or more cells/Kg. IL-2 can be co-administered to expand infused cells. The amount of IL-2 can about $1\text{-}5 \times 10^6$ international units per square meter of body surface.

The efficacy of the compositions or methods described herein may be assessed by any method known in the art and would be evident to a skilled medical professional. For example, the efficacy of the compositions or methods described herein may be assessed by survival of the subject or cancer or pathogen burden in the subject or tissue or sample thereof. In some embodiments, the compositions and methods described herein may be assessed based on the safety or toxicity of the therapy (*e.g.*, administration of the immune cells expressing the glucose importation polypeptides and the CAR polypeptides) in the subject, for example, by the overall health of the subject and/or the presence of adverse events or severe adverse events.

### (c) Other Immunotherapies

In some embodiments, the genetically-engineered immune cells, expressing one or more of the glucose importation polypeptides, may be derived from natural immune cells specific to diseased cells (*e.g.*, cancer cells or pathogen infected cells). Such genetically-engineered immune cells (*e.g.*, tumor-infiltrating lymphocytes or TILs) may not co-express any chimeric receptor polypeptide and can be used to destroy the target disease cells, *e.g.,* cancer cells. The genetically-engineered TILs, expressing one or more glucose importation polypeptides but not chimeric receptors, may be co-used with a bispecific antibody capable of binding to the target tumor cells and the TILs (BiTE).

### V. Combination Treatments

The references to methods of treatment in the following paragraphs of this description are to be interpreted as references to the compounds, pharmaceutical compositions and medicaments of the present invention for use in a method for treatment of a subject.

The compositions and methods described in the present disclosure may be utilized in conjunction with other types of therapy for cancer, such as chemotherapy, surgery, radiation, gene therapy, and so forth, or anti-infection therapy. Such therapies can be administered simultaneously or sequentially (in any order) with the immunotherapy according to the present disclosure.

When co-administered with an additional therapeutic agent, suitable therapeutically effective dosages for each agent may be lowered due to the additive action or synergy.

The treatments of the disclosure can be combined with other immunomodulatory treatments such as, *e.g.*, therapeutic vaccines (including but not limited to GVAX, DC-based vaccines, *etc.*), checkpoint inhibitors (including but not limited to agents that block CTLA4, PD1, LAG3, TIM3, *etc.*) or activators (including but not limited to agents that enhance 41BB, OX40, *etc.*).

Non-limiting examples of other therapeutic agents useful for combination with the immunotherapy of the disclosure include: (i) anti-angiogenic agents (*e.g.*, TNP-470, platelet factor 4, thrombospondin-1, tissue inhibitors of metalloproteases (TIMP1 and TIMP2), prolactin (16-Kd fragment), angiostatin (38-Kd fragment of plasminogen), endostatin, bFGF soluble receptor, transforming growth factor beta, interferon alpha, soluble KDR and FLT-1 receptors, placental proliferin-related protein, as well as those listed by Carmeliet and Jain (2000)); (ii) a VEGF antagonist or a VEGF receptor antagonist such as anti-VEGF antibodies, VEGF variants, soluble VEGF receptor fragments, aptamers capable of blocking VEGF or VEGFR, neutralizing anti-VEGFR antibodies, inhibitors of VEGFR tyrosine kinases and any combinations thereof; and (iii) chemotherapeutic compounds such as, *e.g.,* pyrimidine analogs (5-fluorouracil, floxuridine, capecitabine, gemcitabine and cytarabine), purine analogs, folate antagonists and related inhibitors (mercaptopurine, thioguanine, pentostatin and 2-chlorodeoxyadenosine (cladribine)); antiproliferative/antimitotic agents including natural products such as vinca alkaloids (vinblastine, vincristine, and vinorelbine), microtubule disruptors such as taxane (paclitaxel, docetaxel), vincristine, vinblastine, nocodazole, epothilones, and navelbine, epidipodophyllotoxins (etoposide and teniposide), DNA damaging agents (actinomycin, amsacrine, anthracyclines, bleomycin, busulfan, camptothecin, carboplatin, chlorambucil, cisplatin, cyclophosphamide, cytoxan, dactinomycin, daunorubicin, doxorubicin, epirubicin, hexamethylmelamine oxaliplatin, iphosphamide, melphalan, merchlorehtamine, mitomycin, mitoxantrone, nitrosourea, plicamycin, procarbazine, taxol, taxotere, teniposide, triethylenethiophosphoramide and etoposide (VP16)); antibiotics such as dactinomycin (actinomycin D), daunorubicin, doxorubicin (adriamycin), idarubicin, anthracyclines, mitoxantrone, bleomycin, plicamycin (mithramycin) and mitomycin; enzymes (L-asparaginase which systemically metabolizes L-asparagine and deprives cells which do not have the capacity to synthesize their own asparagine); antiplatelet agents; antiproliferative/antimitotic alkylating agents such as nitrogen mustards (mechlorethamine, cyclophosphamide and analogs, melphalan, chlorambucil), ethylenimines and methylmelamines (hexamethylmelamine and thiotepa), alkyl sulfonates-busulfan, nitrosoureas (carmustine (BCNU) and analogs, streptozocin), trazenes-dacarbazinine (DTIC); antiproliferative/antimitotic antimetabolites such as folic acid analogs (methotrexate); platinum coordination complexes (cisplatin, carboplatin), procarbazine, hydroxyurea, mitotane, aminoglutethimide; hormones, hormone analogs (estrogen, tamoxifen, goserelin, bicalutamide, nilutamide) and aromatase inhibitors (letrozole, anastrozole); anticoagulants (heparin, synthetic heparin salts and other inhibitors of thrombin); fibrinolytic agents (such as tissue plasminogen activator, streptokinase and urokinase), aspirin, dipyridamole, ticlopidine, clopidogrel, abciximab; antimigratory agents; antisecretory agents (brefeldin); immunosuppressives (cyclosporine, tacrolimus (FK-506), sirolimus (rapamycin), azathioprine, mycophenolate mofetil); anti-angiogenic compounds (*e.g.*, TNP-470, genistein, bevacizumab) and growth factor inhibitors (*e.g.*, fibroblast growth factor (FGF) inhibitors); angiotensin receptor blocker; nitric oxide donors; anti-sense oligonucleotides; antibodies (trastuzumab); cell cycle inhibitors and differentiation inducers (tretinoin); AKT inhibitors (such as MK-2206 2HCl, Perifosine (KRX-0401), GSK690693, Ipatasertib (GDC-0068), AZD5363, uprosertib, afuresertib, or triciribine); mTOR inhibitors, topoisomerase inhibitors (doxorubicin (adriamycin), amsacrine, camptothecin, daunorubicin, dactinomycin, eniposide, epirubicin, etoposide, idarubicin, mitoxantrone, topotecan, and irinotecan), corticosteroids (cortisone, dexamethasone, hydrocortisone, methylprednisolone, prednisone, and prednisolone); growth factor signal transduction kinase inhibitors; mitochondrial dysfunction inducers and caspase activators; and chromatin disruptors.

For examples of additional useful agents see also Physician's Desk Reference, 59.sup.th edition, (2005), Thomson P D R, Montvale N.J.; Gennaro et al., Eds. Remington's The Science and Practice of Pharmacy 20th edition, (2000), Lippincott Williams and Wilkins, Baltimore Md.; Braunwald et al., Eds. Harrison's Principles of Internal Medicine, 15.sup.th edition, (2001), McGraw Hill, NY; Berkow et al., Eds. The Merck Manual of Diagnosis and Therapy, (1992), Merck Research Laboratories, Rahway N.J.

The efficacy of the methods described herein may be assessed by any method known in the art and would be evident to a skilled medical professional. For example, the efficacy of the antibody-based immunotherapy may be assessed by survival of the subject or cancer burden in the subject or tissue or sample thereof. In some embodiments, the antibody-based immunotherapy is assessed based on the safety or toxicity of the therapy in the subject, for example by the overall health of the subject and/or the presence of adverse events or severe adverse events.

## VII. <u>Kits for Therapeutic Use</u>

The present disclosure also provides kits for use of the compositions described herein. For example, the present disclosure also provides kits for use of an antibody and a population of immune cells (*e.g.,* T lymphocytes or NK cells) that express a glucose importation polypeptide and/or a chimeric receptor polypeptide such as an ACTR polypeptide or a CAR polypeptide in enhancing cell-mediated cytotoxicity (either directly or mediated by an antibody), enhancing an antibody-based immunotherapy, and/or enhancing immune cell growth and/or proliferation in a low glucose environment. Such kits may include one or more containers comprising a first pharmaceutical composition that comprises a population of T lymphocytes and/or NK cells (immune cells) that express a glucose importation polypeptide and/or a chimeric receptor polypeptide such as those described herein, and a second pharmaceutical composition that comprises an antibody and a pharmaceutically acceptable carrier.

In some embodiments, the kit described herein comprises glucose importation polypeptide-expressing and/or chimeric receptor-expressing immune cells which are expanded *in vitro,* and an antibody specific to a cell surface antibody that is present on activated T cells, for example, an anti-CD5 antibody, an anti-CD38 antibody or an anti-CD7 antibody. The glucose importation polypeptide-expressing and/or chimeric receptor-expressing immune cells may express any of the chimeric receptor polypeptides known in the art or disclosed herein.

In some embodiments, the kit can additionally comprise instructions for use in any of the methods described herein. The included instructions may comprise a description of administration of the first and second pharmaceutical compositions to a subject to achieve the intended activity, *e.g.*, enhancing ADCC activity, and/or enhancing the efficacy of an antibody-based immunotherapy in a subject and/or enhancing the growth and/or proliferation of immune cells in a low-glucose environment (*e.g.*, a low glucose tumor microenvironment in the subject). The kit may further comprise a description of selecting a subject suitable for treatment based on identifying whether the subject is in need of the treatment. In some embodiments, the instructions comprise a description of administering the first and second pharmaceutical compositions to a subject who is in need of the treatment.

The instructions relating to the use of the first and second pharmaceutical compositions described herein generally include information as to dosage, dosing schedule, and route of administration for the intended treatment. The containers may be unit doses, bulk packages (*e.g.*, multi-dose packages) or sub-unit doses. Instructions supplied in the kits of the disclosure are typically written instructions on a label or package insert. The label or package insert indicates that the pharmaceutical compositions are used for treating, delaying the onset, and/or alleviating a disease or disorder in a subject.

The kits provided herein are in suitable packaging. Suitable packaging includes, but is not limited to, vials, bottles, jars, flexible packaging, and the like. Also contemplated are packages for use in combination with a specific device, such as an inhaler, nasal administration device, or an infusion device. A kit may have a sterile access port (for example, the container may be an intravenous solution bag or a vial having a stopper pierceable by a hypodermic injection needle). The container may also have a sterile access port. At least one active agent in the second pharmaceutical composition is an antibody as described herein. At least one active agent in the first pharmaceutical composition is a population of immune cells (*e.g.*, T lymphocytes or NK cells) that express a chimeric receptor polypeptide and/or a glucose importation polypeptide as described herein.

Kits optionally may provide additional components such as buffers and interpretive information. Normally, the kit comprises a container and a label or package insert(s) on or associated with the container. In some embodiment, the disclosure provides articles of manufacture comprising contents of the kits described above.

*General techniques*

The practice of the present disclosure will employ, unless otherwise indicated, conventional techniques of molecular biology (including recombinant techniques), microbiology, cell biology, biochemistry, and immunology, which are within the skill of the art. Such techniques are explained fully in the literature, such as Molecular Cloning: A Laboratory Manual, second edition (Sambrook, et al., 1989) Cold Spring Harbor Press; Oligonucleotide Synthesis (M. J. Gait, ed. 1984); Methods in Molecular Biology, Humana Press; Cell Biology: A Laboratory Notebook (J. E. Cellis, ed., 1989) Academic Press; Animal Cell Culture (R. I. Freshney, ed. 1987); Introuction to Cell and Tissue Culture (J. P. Mather and P. E. Roberts, 1998) Plenum Press; Cell and Tissue Culture: Laboratory Procedures (A. Doyle, J. B. Griffiths, and D. G. Newell, eds. 1993-8) J. Wiley and Sons; Methods in Enzymology (Academic Press, Inc.); Handbook of Experimental Immunology (D. M. Weir and C. C. Blackwell, eds.): Gene Transfer Vectors for Mammalian Cells (J. M. Miller and M. P. Calos, eds., 1987); Current Protocols in Molecular Biology (F. M. Ausubel, et al. eds. 1987); PCR: The Polymerase Chain Reaction, (Mullis, et al., eds. 1994); Current Protocols in Immunology (J. E. Coligan et al., eds., 1991); Short Protocols in Molecular Biology (Wiley and Sons, 1999); Immunobiology (C. A. Janeway and P. Travers, 1997); Antibodies (P. Finch, 1997); Antibodies: a practice approach (D. Catty., ed., IRL Press, 1988-1989); Monoclonal antibodies: a practical approach (P. Shepherd and C. Dean, eds., Oxford University Press, 2000); Using antibodies: a laboratory manual (E. Harlow and D. Lane (Cold Spring Harbor Laboratory Press, 1999); The Antibodies (M. Zanetti and J. D. Capra, eds. Harwood Academic Publishers, 1995); DNA Cloning: A practical Approach, Volumes I and II (D.N. Glover ed. 1985); Nucleic Acid Hybridization (B.D. Hames & S.J. Higgins eds.(1985»; Transcription and Translation (B.D. Hames & S.J. Higgins, eds. (1984»; Animal Cell Culture (R.I. Freshney, ed. (1986»; Immobilized Cells and Enzymes (IRL Press, (1986»; and B. Perbal, A practical Guide To Molecular Cloning (1984); F.M. Ausubel et al. (eds.).

Without further elaboration, it is believed that one skilled in the art can, based on the above description, utilize the present disclosure to its fullest extent. The following specific embodiments are, therefore, to be construed as merely illustrative, and not limitative of the remainder of the disclosure in any way whatsoever.

## EXAMPLES

### Example 1: Impact of expressing a glucose importation polypeptide on T cell function in lower glucose environments.

A glucose importation polypeptide transgene is co-expressed in the same T cell with an ACTR polypeptide. The transgene is, for example, GLUT1, GLUT1 S226D variant, GLUT3, GLUT8, GLUT8 L12A L13A variant, GLUT11, GLUT7, GLUT4, SGLT1, or SGLT2 (*e.g.*, SEQ ID NOs:81-90). The T cells are transduced with a virus encoding the ACTR polypeptide and the glucose importation polypeptide separated, for example, by a P2A ribosomal skip sequence. The T cells are mixed at a given effector-to-target (E:T) ratio with tumor target cells, such as IGROV-1 cells, and a tumor-targeting antibody such as an anti-FOLR1 antibody. Reactions are then incubated at 37 °C in a 5 % $CO_2$ incubator for a period of time (*e.g.*, 6 - 8 days) at different starting concentrations of glucose (*e.g.*, 0 - 20 mM). T cell function is then evaluated, for example, using cytokine production or T cell proliferation assays. Cytokine production (*e.g.*, IL-2 and/or IFN-gamma) is measured from the reaction supernatant. For proliferation experiments, co-cultures are harvested and stained with an anti-CD3 antibody and a live-dead cell stain. The number of live, CD3-positive cells is evaluated by flow cytometry as a measure of T cell proliferation. T cells expressing a glucose importation polypeptide in addition to the ACTR polypeptide show enhanced T cell function relative to T cells expressing ACTR alone including, for example, enhanced cytokine production or enhanced proliferation. This enhanced function may be more pronounced at lower glucose concentrations. These experiments demonstrate that expressing a glucose importation polypeptide in T cells has a positive impact on T cell activity.

### Example 2: Impact of expressing a glucose importation polypeptide gene on T cell function in environments with higher soluble inhibitor concentrations.

A glucose importation polypeptide transgene is co-expressed in the same T cell with an ACTR polypeptide. The transgene is, for example, GLUT1, GLUT1 S226D variant, GLUT3, GLUT8, GLUT8 L12A L13A variant, GLUT11, GLUT7, GLUT4, SGLT1, or SGLT2 (*e.g.*, SEQ ID NOs:81-90). The T cells are transduced with virus encoding the ACTR polypeptide and the glucose importation polypeptide separated, for example, by a P2A ribosomal skip sequence. Transduced T cells are mixed at a given effector-to-target (E:T) ratio with tumor target cells, such as IGROV-1 cells, and a tumor-targeting antibody such as an anti-FOLR1 antibody, in media containing different concentrations of soluble inhibitors that are present in the tumor microenvironment (*e.g.*, TGFbeta, $PGE_2$, and/or adenosine). Reactions are then incubated at 37 °C in a 5 % $CO_2$ incubator for a period of time (*e.g.*, 6 - 8 days). T cell function is then evaluated, for example, using cytokine production or T cell proliferation assays. Cytokine production (*e.g.*, IL-2 and/or IFN-gamma) is measured from the reaction supernatant. For proliferation experiments, co-cultures are harvested and stained with an anti-CD3 antibody and a live-dead cell stain. The number of live, CD3-positive cells is evaluated by flow cytometry as a measure of T cell proliferation. T cells expressing a glucose importation polypeptide in addition to the ACTR polypeptide show enhanced T cell function relative to T cells expressing ACTR alone including, for example, enhanced cytokine production or enhanced proliferation. This enhanced function may be achieved at higher soluble inhibitor concentrations. These experiments demonstrate that expressing a glucose importation polypeptide in T cells has a positive impact on T cell activity.

**Example 3: Impact of expressing a glucose importation polypeptide on T cell function in environments with greater immunosuppressive cell presence.**

A glucose importation polypeptide transgene is co-expressed in the same T cell with an ACTR polypeptide. The transgene is, for example, GLUT1, GLUT1 S226D variant, GLUT3, GLUT8, GLUT8 L12A L13A variant, GLUT11, GLUT7, GLUT4, SGLT1, or SGLT2 (*e.g.*, SEQ ID NOs:81-90). The T cells are transduced with virus encoding the ACTR polypeptide and the glucose importation polypeptide separated, for example, by a P2A ribosomal skip sequence. Transduced T cells are mixed at a given effector-to-target (E:T) ratio with tumor target cells, such as IGROV-1 cells, and a tumor-targeting antibody such as an anti-FOLR1 antibody, in the presence of immunosuppressive cells (*e.g.*, myeloid-derived suppressor cells and/or regulatory T cells). Reactions are then incubated at 37 °C in a 5 % $CO_2$ incubator for a period of time (*e.g.*, 3 - 10 days). T cell function is then evaluated, for example, using cytokine production or T cell proliferation assays. Cytokine production (*e.g.*, IL-2 and/or IFN-gamma) is measured from the reaction supernatant. For proliferation experiments, co-cultures are harvested and stained with an anti-CD3 antibody and a live-dead cell stain. The number of live, CD3-positive cells is evaluated by flow cytometry as a measure of T cell proliferation. T cells expressing a glucose importation polypeptide in addition to the ACTR polypeptide show enhanced T cell function relative to T cells expressing ACTR alone including, for example, enhanced cytokine production or enhanced proliferation. This enhanced function may be achieved in the presence of increased amounts (*e.g.*, greater number or percentage) of immunosuppressive cells. These experiments demonstrate that expressing a glucose importation polypeptide in T cells has a positive impact on T cell activity.

**Example 4: Impact of expressing a glucose importation polypeptide on T cell function on tumor models.**

A glucose importation polypeptide transgene is co-expressed in the same T cell with an ACTR polypeptide. The transgene is, for example, GLUT1, GLUT1 S226D variant, GLUT3, GLUT8, GLUT8 L12A L13A variant, GLUT11, GLUT7, GLUT4, SGLT1, or SGLT2 (*e.g.*, SEQ ID NOs:81-90). The T cells are transduced with virus encoding the ACTR polypeptide and the glucose importation polypeptide separated, for example, by a P2A ribosomal skip sequence. Transduced T cells are evaluated for anti-tumor activity in mouse tumor models. For these experiments, a tumor cell line, for example IGROV-1, is inoculated into NSG™ (NOD *scid* gamma, NOD.*Cg-Prkdc*$^{scid}$ IL2rg$^{tm1Wjl}$/SzJ, Strain 005557) mice. Tumor-bearing mice are subsequently dosed with a tumor-targeting antibody and T cells expressing ACTR alone or ACTR and a glucose importation polypeptide. Tumor growth is monitored throughout the course of the experiment. In combination with a tumor-targeting antibody, T cells expressing a glucose importation polypeptide in addition to an ACTR polypeptide show enhanced anti-tumor activity relative to T cells expressing an ACTR polypeptide alone. Additionally, in combination with a tumor-targeting antibody, T cells expressing a glucose importation polypeptide in addition to an ACTR polypeptide may show enhanced T cell activity including, for example, enhanced proliferation, enhanced T cell persistence, and/or enhanced cytokine production relative to T cells expressing the ACTR polypeptide alone. These experiments demonstrate that expressing a glucose importation polypeptide in ACTR-expressing T cells has a positive impact on T cell function *in vivo.*

**Example 5. Co-Expression of ACTR and GLUT1 in T Cells Enhanced GLUT1 Expression**

This example demonstrated that glucose transporter 1 (GLUT1) expression is increased in T cells that are transduced with a virus encoding an ACTR polypeptide and GLUT1. In these experiments, T cells were transduced with virus encoding an ACTR polypeptide alone (SEQ ID NO:57) or ACTR and GLUT1 (SEQ ID NO: 81) separated by a P2A ribosomal skip sequence. GLUT1 expression was evaluated by flow cytometry. T cells were stained with eFluor780 fixable viability dye (eBioscience), followed by staining with an anti-CD16 antibody to detect ACTR expression and anti-CD4 and anti-CD8 antibodies. Cells were washed and then fixed and permeabilized with Fixation/Permeabilization Solution (BD Biosciences). Cells were then stained with an anti-GLUT1 antibody, washed, and then analyzed by flow cytometry.

Histograms of the flow cytometry data are shown in Figure 2. Live cell populations were gated by CD16 expression to give rise to the non-transduced (CD16-) and ACTR (CD16+) populations. These cell populations were also gated as CD4+ or CD8+ cells. The observed GLUT1 expression was higher in CD8+ cells relative to CD4+ cells in the non-transduced cell populations in the ACTR alone T cells and ACTR + GLUT1 T cells. The observed GLUT1 expression was higher in the CD16+ populations of both CD4+ and CD8+ cells for T cells co-expressing ACTR and GLUT1 relative to T cells expressing ACTR alone. These experiments demonstrate that co-expression of ACTR and GLUT1 in T cells results in increased expression of GLUT1 in ACTR-positive T cells.

**Example 6. Co-Expression of ACTR and GLUT1 Enhanced Glucose Uptake in T Cells**

This example demonstrated that glucose uptake is increased in T cells that are transduced with a virus encoding an ACTR polypeptide and GLUT1. In these experiments, T cells were transduced with virus encoding an ACTR polypeptide alone (SEQ ID NO:57) or ACTR and GLUT1 (SEQ ID NO:81) separated by a P2A ribosomal skip sequence.

For pre-activation experiments, T cells were rested overnight in RPMI 1640 media supplemented with 10 % fetal bovine serum in a $CO_2$ (5 %) incubator at 37 degrees C. Cells were harvested and resuspended in PBS with calcium and magnesium. Glucose uptake was measured by evaluating the ability of cells to uptake 2-deoxy-glucose (2DG) using the Glucose Uptake-Glo assay (Promega) according to the manufacturer's protocol. For each experiment, T cells (50,000) were incubated with 2DG (1 mM) for 20 minutes prior to sample processing; all measurements were carried out in triplicate.

For day 4 activation experiments, T cells were rested overnight in RPMI 1640 media supplemented with 10 % fetal bovine serum in a $CO_2$ (5 %) incubator at 37 degrees C. Cells were harvested and resuspended in 50 % RPMI 1640 and 50 % glucose-free RPMI 1640 to give a final concentration of 5 mM glucose; media was supplemented with 10 % fetal bovine serum. T cells were mixed at a 3.2:1 E:T ratio with fixed JHH7 or fixed HepG2 cells, both of which express GPC3, and an anti-GPC3 antibody (0.5 $\mu$g/mL) or with fixed IGROV-1 cells, which express FOLR$\alpha$, an anti-FOLR$\alpha$ antibody (1 $\mu$g/mL). Reactions were incubated in a $CO_2$ (5 %) incubator at 37 degrees C for 4 days. Cells were harvested and resuspended in PBS with calcium and magnesium. Glucose uptake was measured by evaluating the ability of cells to uptake 2-deoxy-glucose (2DG) using the Glucose Uptake-Glo assay (Promega) according to the manufacturer's protocol. For each experiment, T cells (50,000) were incubated with 2DG (1 mM) for 20 minutes prior to sample processing; all measurements were carried out in triplicate.

The fold change in 2DG uptake for T cells co-expressing ACTR and GLUT1 relative T cells expressing ACTR alone is plotted for pre-activation and day 4 activation experiments (Figure 3) for multiple T cell samples from multiple donors across multiple target-antibody pairs. Each symbol represents the mean of 3 measurements. In aggregate, these data demonstrate that T cells co-expressing ACTR and GLUT1 uptake more glucose than T cells expressing ACTR alone prior to activation and after 4 days of activation.

**Example 7. Co-Expression of ACTR and GLUT1 Enhanced ACTR-T Cell Activity**

This example demonstrates that expressing glucose transporter 1 (GLUT1) in T cells in combination with ACTR enhances the activity of the T cell relative to ACTR alone in the presence of suppressive regulatory T cells. Inducible regulatory T cells (Tregs) were isolated and expanded from PBMCs. Briefly, CD4+ CD127-/dim cells were isolated from PBMCs using a Regulatory T Cell Isolation Kit II (Miltenyi) according to the manufacturer's protocol. Isolated cells were expanded by stimulating with Human Treg Expander Dynabeads (Gibco) every 4 - 6 days and culturing in RPMI 1640 media supplemented with 10 % fetal bovine serum in the presence of human IL-2 (1000 U/mL), human TGF-beta (10 ng/mL), and rapamycin (100 nM). Cells were maintained at approximately 0.5 x $10^6$ cells per mL throughout the expansion. Cell phenotype was monitored by flow cytometry using anti-CD4, anti-CD25, anti-CD127, and anti-FoxP3 antibodies. Activated inducible Tregs were defined as a cell population with >10 $\mu$m diameter and a phenotype of >95 % CD4+/CD25$_{high}$/FoxP3+/CD127$_{dim}$. Activated inducible Tregs were used in subsequent experiments.

In these experiments, T cells were transduced with virus encoding an ACTR polypeptide (SEQ ID NO:57) alone or an ACTR polypeptide and GLUT1 (SEQ ID NO:81) separated by a P2A ribosomal skip sequence. Transduced T cells were incubated at a 1:1 E:T ratio with live IGROV-1 target cells, which express FOLR$\alpha$, an anti-FOLR$\alpha$ antibody (1 $\mu$g/mL) in RPMI 1640 media supplemented with 10 % fetal bovine serum. Reactions were carried out in the absence or presence of donor-matched inducible Tregs at varying ratios relative to T cells (T cell:Treg = 1:0, 4:1, 2:1, or 1:1). Reactions were incubated in a $CO_2$ (5 %) incubator at 37 degrees C for 7 days. Supernatants were removed from each reaction after 4 days and IFN-gamma levels were measured using a Human IFN gamma Kit (Cisbio).

The amount of IFN-gamma is plotted as a function of condition for ACTR T cells (SEQ ID NO:57) made from two different donors (Figure 4, panel A). These data demonstrate a dose-dependent inhibition of IFN-gamma in the presence of increasing amounts of inducible Tregs. The percentage of maximum IFN-gamma, determined by the amount of IFN-gamma produced relative to a matched reaction in the absence of inducible Tregs, is plotted as a function of TNF superfamily polypeptide co-expressed with ACTR in T cells (Figure 4, panel B). Experiments in Donor 1 were carried out with a 4:1 T cell:Treg ratio; experiments in Donor 2 were carried out with a 1:1 T cell:Treg ratio. IFN-gamma production from T cells expressing ACTR alone (parent) is suppressed in the presence of Tregs to a level that is 30 - 40 % that of cells in the absence of Tregs in both donors. Similar results are observed with T cells co-expressing ACTR and GLUT1 in both in Donor 1. T cells co-expressing ACTR and GLUT1 are more resistant to Treg suppression than T cells expressing ACTR alone as evidenced by higher relative IFN-gamma production in Donor 2.

These experiments demonstrate that co-expressing GLUT1 in T cells that also express ACTR can enhance T cell activity by making them more resistant to suppression by Tregs, which are known to be present in solid tumor microenvironments.

### Example 8: Impact of reduced glucose concentrations on T cell function.

A gamma-retroviral vector encoding an exemplary GPC3-targeting CAR construct (SEQ ID NO:104) was generated via recombinant technology and used to infect primary human T-cells for generating cells that express the GPC3-targeting CAR polypeptide on cell surface. A six-day flow-based proliferation assay was then used to test the functionality of the GPC3-targeting CAR expressing cells. Specifically, 200,000 untransduced mock T-cells or T-cells expressing the GPC3-targeting CAR construct were incubated together at a ratio of 4:1 (effector cells/CAR-expressing T cells to target cells) with either 50,000 GPC3+ hepatocellular carcinoma JHH7 or Hep3B tumor cells. The co-culture was incubated at 37 °C in a 5 % $CO_2$ incubator for 6 days in the presence of different concentrations of glucose. At the end of 6 days, co-cultures were harvested and stained with an anti-CD3 antibody. The number of CD3-positive cells was evaluated by flow cytometry as a measure of T cell proliferation. At lower glucose concentrations, less CAR-T proliferation was observed (Figure 5). These experiments demonstrate that low glucose environments may have a negative impact on CAR-T cell proliferation.

### Example 9: Impact of expressing a glucose transporter gene on T cell co-expressing a GPC3-targeting CAR polypeptide

A gamma-retroviral vector encoding an exemplary GPC3-targeting CAR polypeptide (SEQ ID NO:104) was generated via recombinant technology and used to infect primary human T-cells to generate cells expressing a GPC3-targeting CAR polypeptide on their cell surface. Additionally, gamma-retroviral vectors encoding the exemplary GPC3-targeting CAR polypeptide and a glucose importation polypeptide (GLUT1, GLUT3, or a GLUT 1 S226D variant) were generated via recombinant technology and used to infect primary human T-cells to generate cells that express a GPC3-targeting polypeptide and a glucose importation polypeptide. In the constructs encoding both the CAR polypeptide and the glucose importation polypeptide, the coding sequences of the two polypeptides were separated by a P2A ribosomal skip sequence. The combinations of the GPC3-targeting CAR and the glucose transporter include: SEQ ID NO:104 + SEQ ID NO:81 (GLUT1), SEQ ID NO:1 + SEQ ID NO:5 (GLUT3), and SEQ ID NO:104 + SEQ ID NO: 2 (GLUT1 S226D). A six-day flow-based proliferation assay was then used to test the functionality of the GPC3-targeting CAR expressing cells. Specifically, 200,000 untransduced mock T-cells, T-cells expressing a GPC3-targeting CAR polypeptide, or T-cells expressing a GPC3-targeting CAR polypeptide and a GLUT1 peptide were incubated together at a ratio of 4:1 (effector cells/CAR-expressing T cells to target cells) with 50,000 GPC3+ hepatocellular carcinoma JHH7 tumor cells. The co-culture was incubated at 37 °C in a 5 % $CO_2$ incubator for 6 days in the presence of 1.25 mM glucose (tumor-relevant) and 10 mM glucose (approximate peripheral blood levels). At the end of 6 days, co-cultures were harvested and stained with an anti-CD3 antibody. The number of CD3-positive cells was evaluated by flow cytometry as a measure of T cell proliferation. T cells expressing the glucose importation polypeptide in addition to the CAR polypeptide demonstrated enhanced T cell proliferation relative to T cells expressing the CAR construct alone (Figures 6-8). This enhanced proliferation also occurred at tumor-relevant low glucose concentrations. These experiments demonstrate that expressing the glucose transporter GLUT1 in T cells in T cells has a positive impact on CAR-T cell proliferation activity.

### Example 10: Impact of expressing a glucose transporter gene on CAR-T cell function in lower glucose environments.

A glucose importation transgene is co-expressed in the same T cell with a chimeric antigen receptor (CAR) polypeptide. The transgene is, for example, GLUT1, GLUT1 S226D variant, GLUT3, GLUT8, GLUT8 L12A L13A variant, GLUT11, GLUT7, GLUT4, SGLT1, or SGLT2 (e.g., SEQ ID NOs:81-90). The T cells are transduced with a virus encoding the CAR polypeptide and the glucose importation polypeptide separated, for example, by a P2A ribosomal skip sequence. The T cells are mixed at a given effector-to-target (E:T) ratio with tumor target cells, such as HepG2 cells. Reactions are then incubated at 37 °C in a 5 % $CO_2$ incubator for a period of time (e.g., 6 - 8 days) at different starting concentrations of glucose (e.g., 0 - 20 mM). T cell function is then evaluated, for example, using cytokine production or T cell proliferation assays. Cytokine production (e.g., IL-2 and/or IFN-gamma) is measured from the reaction supernatant. For proliferation experiments, co-cultures are harvested and stained with an anti-CD3 antibody and a live-dead cell stain. The number of live, CD3-positive cells is evaluated by flow cytometry as a measure of T cell proliferation. T cells expressing a glucose importation polypeptide in addition to the CAR polypeptide show enhanced T cell function relative to T cells expressing CAR alone including, for example, enhanced cytokine production or enhanced proliferation. This enhanced function may be more pronounced at lower glucose concentrations. These experiments demonstrate that expressing a glucose transporter in T cells has a positive impact on T cell activity.

**Example 11: Impact of expressing a glucose transporter gene on CAR-T cell function in environments with higher soluble inhibitor concentrations.**

A glucose importation transgene is co-expressed in the same T cell with a chimeric antigen receptor (CAR) polypeptide. The transgene is, for example, GLUT1, GLUT1 S226D variant, GLUT3, GLUT8, GLUT8 L12A L13A variant, GLUT11, GLUT7, GLUT4, SGLT1, or SGLT2 (*e.g.*, SEQ ID NOs: 81-90). The T cells are transduced with virus encoding the CAR polypeptide and the glucose importation polypeptide separated, for example, by a P2A ribosomal skip sequence. Transduced T cells are mixed at a given effector-to-target (E:T) ratio with tumor target cells, such as HepG2 cells, in media containing different concentrations of soluble inhibitors that are present in the tumor microenvironment (*e.g.*, TGFbeta, $PGE_2$, and/or adenosine). Reactions are then incubated at 37 °C in a 5 % $CO_2$ incubator for a period of time (*e.g.*, 6 - 8 days). T cell function is then evaluated, for example, using cytokine production or T cell proliferation assays. Cytokine production (*e.g.*, IL-2 and/or IFN-gamma) is measured from the reaction supernatant. For proliferation experiments, co-cultures are harvested and stained with an anti-CD3 antibody and a live-dead cell stain. The number of live, CD3-positive cells is evaluated by flow cytometry as a measure of T cell proliferation. T cells expressing a glucose importation polypeptide in addition to the CAR polypeptide show enhanced T cell function relative to T cells expressing CAR alone including, for example, enhanced cytokine production or enhanced proliferation. This enhanced function may be achieved at higher soluble inhibitor concentrations. These experiments demonstrate that expressing a glucose transporter in T cells has a positive impact on T cell activity.

**Example 12: Impact of expressing a glucose transporter gene on CAR-T cell function in environments with greater immunosuppressive cell presence.**

A glucose importation transgene is co-expressed in the same T cell with a chimeric antigen receptor (CAR) polypeptide. The transgene is, for example, GLUT1, GLUT1 S226D variant, GLUT3, GLUT8, GLUT8 L12A L13A variant, GLUT11, GLUT7, GLUT4, SGLT1, or SGLT2 (*e.g.*, SEQ ID NOs: 81-90). The T cells are transduced with virus encoding the CAR polypeptide and the glucose importation polypeptide separated, for example, by a P2A ribosomal skip sequence. Transduced T cells are mixed at a given effector-to-target (E:T) ratio with tumor target cells, such as HepG2 cells, in the presence of immunosuppressive cells (*e.g.*, myeloid-derived suppressor cells and/or regulatory T cells). Reactions are then incubated at 37 °C in a 5 % $CO_2$ incubator for a period of time (*e.g.*, 3 - 10 days). T cell function is then evaluated, for example, using cytokine production or T cell proliferation assays. Cytokine production (*e.g.*, IL-2 and/or IFN-gamma) is measured from the reaction supernatant. For proliferation experiments, co-cultures are harvested and stained with an anti-CD3 antibody and a live-dead cell stain. The number of live, CD3-positive cells is evaluated by flow cytometry as a measure of T cell proliferation. T cells expressing a glucose importation polypeptide in addition to the CAR polypeptide show enhanced T cell function relative to T cells expressing CAR alone including, for example, enhanced cytokine production or enhanced proliferation. This enhanced function may be achieved in the presence of increased amounts (e.g., greater number or percentage) of immunosuppressive cells. These experiments demonstrate that expressing a glucose transporter in T cells has a positive impact on T cell activity.

**Example 13: Impact of expressing a glucose transporter gene on CAR-T cell function on tumor models.**

A glucose importation transgene is co-expressed in the same T cell with a chimeric antigen receptor (CAR) polypeptide. The transgene is, for example, GLUT1, GLUT1 S226D variant, GLUT3, GLUT8, GLUT8 L12A L13A variant, GLUT11, GLUT7, GLUT4, SGLT1, or SGLT2 (*e.g.,* SEQ ID NOs: 81-90). The T cells are transduced with virus encoding the CAR polypeptide and the glucose importation polypeptide separated, for example, by a P2A ribosomal skip sequence. Transduced T cells are evaluated for anti-tumor activity in mouse tumor models. For these experiments, a tumor cell line, for example HerG2, is inoculated into NSG™ (NOD *scid* gamma, NOD.*Cg-Prkdc^scid* IL2rg^tm1Wjl/SzJ, Strain 005557) mice. Tumor-bearing mice are subsequently dosed with T cells expressing CAR alone or CAR and a glucose importation polypeptide. Tumor growth is monitored throughout the course of the experiment. T cells expressing a glucose importation polypeptide in addition to a CAR polypeptide show enhanced anti-tumor activity relative to T cells expressing a CAR polypeptide alone. Additionally, T cells expressing a glucose importation polypeptide in addition to a CAR polypeptide may show enhanced T cell activity including, for example, enhanced proliferation, enhanced T cell persistence, and/or enhanced cytokine production relative to T cells expressing the CAR polypeptide alone. These experiments demonstrate that expressing a glucose importation polypeptide in CAR-expressing T cells has a positive impact on T cell function *in vivo.*

**Example 14: Impact of expressing a glucose transporter gene on CAR-T cell function in a mouse tumor model using a GPC3-targeting CAR-T expression construct.**

A glucose importation transgene was co-expressed in the same T cell with a GPC3-targeting CAR-T polypeptide. A gamma-retroviral vector encoding an exemplary GPC3-targeting CAR-T polypeptide (SEQ ID NO: 104) was generated via recombinant technology and used to infect primary human T-cells to generate cells that expressed a GPC3-targeting CAR-T on their cell surface. T cells were also transduced with virus encoding the CAR-T polypeptide and the glucose importation polypeptide (GLUT 1, SEQ ID NO: 3), the coding sequences of which were separated by a P2A ribosomal skip sequence (SEQ ID NO:22). The CAR transduced and CAR/GLUT1 transduced T cells were evaluated for anti-tumor activity in a mouse tumor model. For these experiments, the hepatocellular carcinoma tumor cell line, JHH7, was inoculated into NSG™ (NOD $scid$ gamma, NOD.$Cg$-$Prkdc^{scid}$ IL2rg$^{tm1Wjl}$/SzJ, Strain 005557) mice. JHH7 human hepatocellular carcinoma (HCC) xenografts were established in female NSG by subcutaneous injection with 5 x 10$^6$ cells in the right flank. Treatment with GPC3 CAR-expressing T cells was initiated when tumor volumes reached approximately 50 mm$^3$ (day 8 post inoculation). Mice were randomized into treatment groups of 5 mice each based on tumor volume, and treated with T cells expressing the GPC3-targeted CAR polypeptide and T cells co-expressing the CAR polypeptide and GLUT1 at a dose of 5 x 10$^6$ CAR+ T cells on days 8 and 15 post inoculation. The total T cell dose varied based on the CAR transduction efficiency of each construct; total T cell doses were 1.02 x 10$^7$ and 1.72 x 10$^7$ weekly for 2 weeks for the T cells expressing the GPC3-targeted CAR (48.8% CAR+) and the T cells co-expressing the GPC3-targeted CAR and GLUT1 (29.0% CAR+), respectively. Tumor volume and body weights were measured two-to-three times weekly for the duration of the experiment. CAR-T cells co-expressing the glucose transporter GLUT1 demonstrated enhanced anti-tumor efficacy relative to the T cells only expressing the GPC3-targeted CAR construct (Figure 9). These experiments demonstrate that co-expressing the glucose transporter GLUT1 in CAR-T cells has a positive impact on CAR-T cell anti-tumor efficacy in a mouse xenograft model of hepatocellular carcinoma.

**Example 15: Impact of expressing a glucose transporter gene on CAR-T cell function in lower glucose environments.**

A glucose importation transgene was co-expressed in the same T cell with a GPC3-targeting CAR-T polypeptide. A gamma-retroviral vector encoding an exemplary GPC3-targeting CAR-T polypeptide (SEQ ID NO: 104) was generated via recombinant technology and used to infect primary human T-cells to generate cells that expressed a GPC3-targeting CAR-T on their cell surface. T cells were also transduced with virus encoding the CAR-T polypeptide and the glucose importation polypeptide (GLUT1, SEQ ID NO: 81), the coding sequences of which were separated by a P2A ribosomal skip sequence. Co-cultures of T cells were mixed with solid tumor target cells, harvested, and stained with an anti-CD3 antibody and a live-dead cell stain. The number of live, CD3-positive cells was evaluated by flow cytometry as a measure of T cell proliferation (Figure 10). T cells expressing a glucose importation polypeptide in addition to the CAR polypeptide demonstrated enhanced T cell proliferation, which was more pronounced at lower glucose concentrations.

**Example 16: Impact of expressing a glucose transporter gene on CAR-T cell function in a mouse tumor model using a GPC3-targeting CAR-T expression construct.**

A gamma-retroviral vector encoding an exemplary GPC3-targeting CAR-T polypeptide (SEQ ID NO:104) was generated via recombinant technology and used to infect primary human T-cells to generate cells that expressed a GPC3-targeting CAR-T on their cell surface. T cells were also transduced with virus encoding the CAR-T polypeptide and the glucose importation polypeptide (GLUT1, SEQ ID NO: 81), the coding sequences of which were separated by a P2A ribosomal skip sequence. The CAR transduced and CAR/GLUT1 transduced T cells were evaluated for anti-tumor activity in a mouse tumor model. For these experiments, the hepatocellular carcinoma tumor cell line, JHH7, was inoculated into NSG™ (NOD $scid$ gamma, NOD.$Cg$-$Prkdc^{scid}$ IL2rg$^{tm1Wjl}$/SzJ, Strain 005557) mice. JHH7 human hepatocellular carcinoma (HCC) xenografts were established in female NSG by subcutaneous injection with 5 x 10$^6$ cells in the right flank. Treatment with GPC3 CAR-expressing T cells was initiated when tumor volumes reached approximately 50 mm$^3$ (day 8 post inoculation). Mice were randomized into treatment groups of 5 mice each based on tumor volume, and treated with T cells expressing the GPC3-targeted CAR polypeptide and T cells co-expressing the CAR polypeptide and GLUT1 at a dose of 5 x 10$^6$ CAR+ T cells on days 8 and 15 post inoculation. The total T cell dose varied based on the CAR transduction efficiency of each construct; total T cell doses were 1.02 x 10$^7$ and 1.41 x 10$^7$ weekly for 2 weeks for the T cells expressing the GPC3-targeted CAR (48.8% CAR+) and the T cells co-expressing the GPC3-targeted CAR and GLUT1 (35.5% CAR+), respectively. Tumor volume and body weights were measured two-to-three times weekly for the duration of the experiment (Figure 11). Untreated tumors were excised at endpoint, loaded into 0.45$\mu$m filter inset tubes, and subjected to 500 g centrifugal force to expel interstitial fluid (Wiig 2003 Am J Physiol Heart Circ Physiol 284:H416). Blood and tumor interstitial fluid glucose levels were measured using a diabetic blood glucose meter (Figure 13). CAR-T cells co-expressing the glucose transporter GLUT1 demonstrated enhanced anti-tumor efficacy relative to the T cells only expressing the GPC3-targeted CAR construct. These experiments demonstrate that co-expressing the glucose transporter GLUT1 in CAR-T cells has a positive impact on CAR-T cell anti-tumor efficacy in a mouse xenograft model of hepatocellular carcinoma.

## Example 17: Impact of expressing a glucose transporter gene on CAR-T cell function in a mouse tumor model using a GPC3-targeting CAR-T expression construct

A gamma-retroviral vector encoding an exemplary GPC3-targeting CAR-T polypeptide (SEQ ID NO: 1) was generated via recombinant technology and used to infect primary human T-cells to generate cells that expressed a GPC3-targeting CAR-T on their cell surface. T cells were also transduced with virus encoding the CAR-T polypeptide and the glucose importation polypeptide (GLUT1, SEQ ID NO:81), the coding sequences of which were separated by a P2A ribosomal skip sequence. The CAR transduced and CAR/GLUT1 transduced T cells were evaluated for anti-tumor activity in a mouse tumor model. For these experiments, the hepatocellular carcinoma tumor cell line, Hep3B, was inoculated into NSG™ (NOD *scid* gamma, NOD.*Cg-Prkdc*$^{scid}$ IL2rg$^{tm1Wjl}$/SzJ, Strain 005557) mice. Hep3B human hepatocellular carcinoma (HCC) xenografts were established in female NSG by subcutaneous injection with $5 \times 10^6$ cells in the right flank. Treatment with GPC3 CAR-expressing T cells was initiated when tumor volumes reached approximately 100 mm$^3$ (day 20 post inoculation). Mice were randomized into treatment groups of 5 mice each based on tumor volume, and treated with T cells expressing the GPC3-targeted CAR polypeptide and T cells co-expressing the CAR polypeptide and GLUT1 at a dose of $1 \times 10^6$ CAR+ T cells on days 20 and 27 post inoculation. The total T cell dose varied based on the CAR transduction efficiency of each construct; total T cell doses were $2.05 \times 10^6$ and $2.82 \times 10^6$ weekly for 2 weeks for the T cells expressing the GPC3-targeted CAR (48.8% CAR+) and the T cells co-expressing the GPC3-targeted CAR and GLUT1 (35.5% CAR+), respectively. Tumor volume and body weights were measured two-to-three times weekly for the duration of the experiment (Figure 12). Untreated tumors were excised at endpoint, loaded into $0.45\mu$m filter inset tubes, and subjected to 500 g centrifugal force to expel interstitial fluid (Wiig 2003 Am J Physiol Heart Circ Physiol 284:H416). Blood and tumor interstitial fluid glucose levels were measured using a diabetic blood glucose meter (Figure 13). CAR-T cells co-expressing the glucose transporter GLUT1 demonstrated enhanced anti-tumor efficacy relative to the T cells only expressing the GPC3-targeted CAR construct. These experiments demonstrate that co-expressing the glucose transporter GLUT1 in CAR-T cells has a positive impact on CAR-T cell anti-tumor efficacy in a mouse xenograft model of hepatocellular carcinoma.

## Example 18. Co-Expression of Anti-GPC3 CAR and GLUT1 Enhanced GLUT1 Expression

This example demonstrated that glucose transporter 1 (GLUT1) expression is increased in T cells that are transduced with a virus encoding an anti-GPC3 CAR polypeptide and GLUT1. In these experiments, T cells were transduced with virus encoding an anti-GPC3 CAR polypeptide alone (SEQ ID NO:104) or anti-GPC3 CAR and GLUT1 (SEQ ID NO:81) separated by a P2A ribosomal skip sequence. GLUT1 expression was evaluated by flow cytometry. T cells were stained with eFluor780 fixable viability dye (eBioscience), followed by staining with fluorescently-labeled GPC3 extracellular domain to detect CAR expression and anti-CD4 and anti-CD8 antibodies. Cells were washed and then fixed and permeabilized with Fixation/Permeabilization Solution (BD Biosciences). Cells were then stained with an anti-GLUT1 antibody, washed, and then analyzed by flow cytometry.

Histograms of the flow cytometry data are shown in Figure 14. Live cell populations were gated by staining with GPC3 extracellular domain (GPC3 CAR expression) to give rise to the non-transduced (CAR-) and CAR (CAR+) populations. These cell populations were also gated as CD4+ or CD8+ cells. The observed GLUT1 expression was higher in CD8+ cells relative to CD4+ cells in the non-transduced cell populations in the anti-GPC3 CAR alone T cells and anti-GPC3 CAR + GLUT1 T cells. The observed GLUT1 expression was higher in the CAR+ populations of both CD4+ and CD8+ cells for T cells co-expressing anti-GPC3 CAR and GLUT1 relative to T cells expressing CAR alone. These experiments demonstrate that co-expression of anti-GPC3 CAR and GLUT1 in T cells results in increased expression of GLUT1 in CAR-positive T cells.

## Example 19. Co-Expression of Anti-GPC3 CAR and GLUT1 Enhanced Glucose Uptake

This example demonstrated that glucose uptake is increased in T cells that are transduced with a virus encoding an anti-GPC3 CAR and GLUT1. In these experiments, T cells were transduced with virus encoding an anti-GPC3 CAR alone (SEQ ID NO: 104) or an anti-GPC3 CAR and GLUT1 (SEQ ID NO: 81) separated by a P2A ribosomal skip sequence.

For pre-activation experiments, T cells were rested overnight in RPMI 1640 media supplemented with 10 % fetal bovine serum in a $CO_2$ (5 %) incubator at 37 degrees C. Cells were harvested and resuspended in PBS with calcium and magnesium. Glucose uptake was measured by evaluating the ability of cells to uptake 2-deoxy-glucose (2DG) using the Glucose Uptake-Glo assay (Promega) according to the manufacturer's protocol. For each experiment, T cells (50,000) were incubated with 2DG (1 mM) for 20 minutes prior to sample processing; all measurements were carried out in triplicate.

For day 4 activation experiments, T cells were rested overnight in RPMI 1640 media supplemented with 10 % fetal bovine serum in a $CO_2$ (5 %) incubator at 37 degrees C. Cells were harvested and resuspended in 50 % RPMI 1640 and 50 % glucose-free RPMI 1640 to give a final concentration of 5 mM glucose; media was supplemented with 10 % fetal bovine serum. T cells were mixed at a 2:1 E:T ratio with fixed JHH7 cells or fixed HepG2 cells, both of which express GPC3. Reactions were incubated in a $CO_2$ (5 %) incubator at 37 degrees C for 4 days. Cells were harvested and resuspended in PBS with calcium and magnesium. Glucose uptake was measured by evaluating the ability of cells to uptake 2-deoxy-glucose (2DG) using the Glucose Uptake-Glo assay (Promega) according to the manufacturer's protocol. For each experiment, T cells (50,000) were incubated with 2DG (1 mM) for 20 minutes prior to sample processing; all measurements were carried out in triplicate.

The fold change in 2DG uptake for T cells co-expressing anti-GPC3 CAR and GLUT1 relative T cells expressing anti-GPC3 CAR alone is plotted for pre-activation and day 4 activation experiments (Figure 15) for multiple T cell samples from multiple donors. Symbols depict the mean of 3 measurements for each experiment. In aggregate, these data demonstrate that T cells co-expressing anti-GPC3 CAR and GLUT1 uptake more glucose than T cells expressing anti-GPC3 CAR alone after 4 days of activation, similar to modestly increased glucose uptake prior to activation.

## Example 20. T Cells Co-Expressing Anti-GPC3 CAR and GLUT1 Showed High Survival Rate in Xenograft Mice

This example demonstrated that T cells co-expressing anti-GPC3 CAR and GLUT1 expand and persist in mouse xenograft models. For these experiments, blood samples were taken from Hep3B tumor-bearing NSG mice treated with T cells expressing anti-GPC3 CAR (SEQ ID NO:104) and T cells co-expressing anti-GPC3 CAR and GLUT1 (SEQ ID NO:81) (See Examples 10 for details of the xenograft study). Whole blood samples (20 μL) were collected by orbital bleed under isoflurane anesthesia on days 15, 25, 40 and 60 and frozen with BamBanker cryoprotectant until processed for flow cytometry. Red blood cells were lysed, and samples were stained with live/dead stain, anti-human CD3, and fluorescently-labeled recombinant GPC3 protein and analyzed by flow cytometry. Results are expressed as number of live CAR+/CD3+ cells per μL of blood (Figure 16). Each time point represents the mean of groups that contained 3 or more mice. A small number (<1 cell per μL of blood) of CAR-expressing T cells are detected in mice treated with T cells expressing the anti-GPC3 CAR alone at 15 days; the number of mice in this group fell below 3 for subsequent time points. The number of CAR-expressing T cells in mice treated with T cells co-expressing the anti-GPC3 CAR and GLUT1 was much higher than those treated with T cells expressing anti-GPC3 CAR alone at day 15, and remained detectable throughout the course of the experiment at all time points evaluated. These data demonstrate that T cells co-expressing anti-GPC3 CAR and GLUT1 show superior cell expansion and persistence relative to T cells expressing anti-GPC3 CAR alone in tumor-bearing NSG mice.

OTHER EMBODIMENTS

All of the features disclosed in this specification may be combined in any combination. Each feature disclosed in this specification may be replaced by an alternative feature serving the same, equivalent, or similar purpose. Thus, unless expressly stated otherwise, each feature disclosed is only an example of a generic series of equivalent or similar features.

From the above description, one of skill in the art can easily ascertain the essential characteristics of the present disclosure, and without departing from the spirit and scope thereof, can make various changes and modifications of the disclosure to adapt it to various usages and conditions. Thus, other embodiments are also within the claims.

EQUIVALENTS

While several inventive embodiments have been described and illustrated herein, those of ordinary skill in the art will readily envision a variety of other means and/or structures for performing the function and/or obtaining the results and/or one or more of the advantages described herein, and each of such variations and/or modifications is deemed to be within the scope of the inventive embodiments described herein. More generally, those skilled in the art will readily appreciate that all parameters, dimensions, materials, and configurations described herein are meant to be exemplary and that the actual parameters, dimensions, materials, and/or configurations will depend upon the specific application or applications for which the inventive teachings is/are used. Those skilled in the art will recognize, or be able to ascertain using no more than routine experimentation, many equivalents to the specific inventive embodiments described herein. It is, therefore, to be understood that the foregoing embodiments are presented by way of example only and that, within the scope of the appended claims and equivalents thereto, inventive embodiments may be practiced otherwise than as specifically described and claimed. Inventive embodiments of the present disclosure are directed to each individual feature, system, article, material, kit, and/or method described herein. In addition, any combination of two or more such features, systems, articles, materials, kits, and/or methods, if such features, systems, articles, materials, kits, and/or methods are not mutually inconsistent, is included within the inventive scope of the present disclosure.

All definitions, as defined and used herein, should be understood to control over dictionary definitions, and/or ordinary meanings of the defined terms.

The indefinite articles "a" and "an," as used herein in the specification and in the claims, unless clearly indicated to the contrary, should be understood to mean "at least one."

The phrase "and/or," as used herein in the specification and in the claims, should be understood to mean "either or both" of the elements so conjoined, *i.e.,* elements that are conjunctively present in some cases and disjunctively present in other cases. Multiple elements listed with "and/or" should be construed in the same fashion, *i.e.,* "one or more" of the elements so conjoined. Other elements may optionally be present other than the elements specifically identified by the "and/or" clause, whether related or unrelated to those elements specifically identified. Thus, as a non-limiting example, a reference to "A and/or B", when used in conjunction with open-ended language such as "comprising" can refer, in one embodiment, to A only (optionally including elements other than B); in another embodiment, to B only (optionally including elements other than A); in yet another embodiment, to both A and B (optionally including other elements); *etc.*

As used herein in the specification and in the claims, "or" should be understood to have the same meaning as "and/or" as defined above. For example, when separating items in a list, "or" or "and/or" shall be interpreted as being inclusive, *i.e.,* the inclusion of at least one, but also including more than one, of a number or list of elements, and, optionally, additional unlisted items. Only terms clearly indicated to the contrary, such as "only one of" or "exactly one of," or, when used in the claims, "consisting of," will refer to the inclusion of exactly one element of a number or list of elements. In general, the term "or" as used herein shall only be interpreted as indicating exclusive alternatives (*i.e.,* "one or the other but not both") when preceded by terms of exclusivity, such as "either," "one of," "only one of," or "exactly one of." "Consisting essentially of," when used in the claims, shall have its ordinary meaning as used in the field of patent law.

As used herein in the specification and in the claims, the phrase "at least one," in reference to a list of one or more elements, should be understood to mean at least one element selected from any one or more of the elements in the list of elements, but not necessarily including at least one of each and every element specifically listed within the list of elements and not excluding any combinations of elements in the list of elements. This definition also allows that elements may optionally be present other than the elements specifically identified within the list of elements to which the phrase "at least one" refers, whether related or unrelated to those elements specifically identified. Thus, as a non-limiting example, "at least one of A and B" (or, equivalently, "at least one of A or B," or, equivalently "at least one of A and/or B") can refer, in one embodiment, to at least one, optionally including more than one, A, with no B present (and optionally including elements other than B); in another embodiment, to at least one, optionally including more than one, B, with no A present (and optionally including elements other than A); in yet another embodiment, to at least one, optionally including more than one, A, and at least one, optionally including more than one, B (and optionally including other elements); etc.

It should also be understood that, unless clearly indicated to the contrary, in any methods claimed herein that include more than one step or act, the order of the steps or acts of the method is not necessarily limited to the order in which the steps or acts of the method are recited.

**Claims**

1.  A genetically engineered immune cell, wherein the immune cell has an improved glucose uptake activity as relative to a wild-type immune cell of the same type, wherein the immune cell is genetically engineered to express or overly express:

    (i) a glucose importation polypeptide, and
    (ii) a chimeric receptor polypeptide; wherein the chimeric receptor polypeptide comprises:

        (a) an extracellular target binding domain;
        (b) a transmembrane domain; and
        (c) a cytoplasmic signaling domain;

    wherein the nucleic acid encoding the glucose importation polypeptide is located extrachromosomally or is integrated into a chromosome of the immune cell at a site that is different from the native loci of the endogenous gene,
    wherein the glucose importation polypeptide is a glucose transporter (GLUT) or a sodium-glucose cotransporter (SGLT), preferably wherein the glucose importation polypeptide is selected from the group consisting of: GLUT1, GLUT3, GLUT1 S226D, SGLT1, SGLT2, GLUT8, GLUT8 L12A L13A, GLUT11, GLUT7, and GLUT4.

**2.** The immune cell of claim 1, wherein the chimeric receptor polypeptide is an antibody-coupled T cell receptor (ACTR) polypeptide, in which (a) is an extracellular Fc binding domain, optionally

wherein the Fc binding domain is an extracellular ligand-binding domain of an Fc-receptor; preferably wherein the Fc binding domain is an extracellular ligand-binding domain of CD16A, CD32A, or CD64A; and more preferably wherein the Fc binding domain is an extracellular ligand-binding domain of F158 CD16A or V158 CD16A.

**3.** The immune cell of claim 1, wherein the chimeric receptor polypeptide is a chimeric antigen receptor (CAR) polypeptide, in which (a) is an extracellular antigen binding domain; preferably wherein the extracellular antigen binding domain of (a) is a single chain antibody fragment that binds to a tumor antigen, a pathogenic antigen, or an immune cell specific to an autoantigen.

**4.** The immune cell of claim 3, wherein

(i) the tumor antigen is associated with a hematologic tumor, preferably wherein the tumor antigen is selected from the group consisting of CD19, CD20, CD22, Kappa-chain, CD30, CD123, CD33, LeY, CD138, CD5, BCMA, CD7, CD40, and IL-1RAP;

(ii) the tumor antigen is associated with a solid tumor, preferably wherein the tumor antigen is selected from the group consisting of GD2, GPC3, FOLR, HER2, EphA2, EFGRVIII, IL13RA2, VEGFR2, ROR1, NKG2D, EpCAM, CEA, Mesothelin, MUC1, CLDN18.2, CD171, CD133, PSCA, cMET, EGFR, PSMA, FAP, CD70, MUC16, L1-CAM, and CAIX; or

(iii) the pathogenic antigen is a bacterial antigen, a viral antigen, or a fungal antigen.

**5.** The immune cell of any one of claims 1-4, wherein the chimeric receptor polypeptide comprises one or more of the following features:

(i) the chimeric receptor polypeptide further comprises at least one co-stimulatory signaling domain;

(ii) the chimeric receptor polypeptide is free of co-stimulatory signaling domains;

(iii) the cytoplasmic signaling domain comprises an immunoreceptor tyrosine-based activation motif (ITAM);

(iv) the cytoplasmic signaling domain (c) is located at the C-terminus of the chimeric receptor polypeptide;

(v) the chimeric receptor polypeptide further comprises a hinge domain, which is located at the C-terminus of (a) and the N-terminus of (b), or the chimeric receptor polypeptide is free of any hinge domain; and

(vi) the chimeric receptor polypeptide further comprises a signal peptide at its N-terminus.

**6.** The immune cell of any one of claims 1-5, wherein

(i) the transmembrane domain of (b) is of a single-pass membrane protein, which preferably is of a membrane protein selected from the group consisting of CD8α, CD8β, 4-1BB, CD28, CD34, CD4, FcεRIγ, CD16A, OX40, CD3ζ, CD3ε, CD3γ, CD3δ, TCRα, CD32, CD64, VEGFR2, FAS, and FGFR2B; or the transmembrane domain of (b) is a non-naturally occurring hydrophobic protein segment; and/or

(ii) the cytoplasmic signaling domain of (c) is a cytoplasmic domain of CD3ζ or FcεR1γ.

**7.** The immune cell of any one of claims 5 or 6, wherein the at least one co-stimulatory signaling domain is of a co-stimulatory molecule selected from the group consisting of 4-1BB, CD28, CD28$_{LL \to GG}$ variant, OX40, ICOS, CD27, GITR, HVEM, TIM1, LFA1, and CD2, preferably wherein the at least one co-stimulatory signaling domain is a CD28 co-stimulatory signaling domain or a 4-1BB co-stimulatory signaling domain.

**8.** The immune cell of any one of claims 5-7, wherein the hinge domain is 1 to 60 amino acids in length, preferably wherein the hinge domain is of CD28, CD16A, CD8α, or IgG; or wherein the hinge domain is a non-naturally occurring peptide, preferably wherein the hinge domain is an extended recombinant polypeptide (XTEN) or a $(Gly_4Ser)_n$ polypeptide, in which n is an integer of 3-12, inclusive.

**9.** The immune cell of any one of claims 3-8, wherein the chimeric receptor polypeptide is a CAR polypeptide, which comprises

(i) a CD28 co-stimulatory domain; in combination with a CD28 transmembrane domain, a CD28 hinge domain, or a combination thereof, or

(ii) a 4-1BB co-stimulatory domain in combination with a CD8 transmembrane domain, a CD8 hinge domain, or a combination thereof; and/or

(iii) wherein the CAR polypeptide comprises the amino acid sequence of SEQ ID NOs: 104 or 105.

10. The immune cell of any one of claims 1-9, wherein

(i) the immune cell is a natural killer cell, macrophage, neutrophil, eosinophil, or T cell, preferably wherein the immune cell is a T cell in which the expression of an endogenous T cell receptor, an endogenous major histocompatibility complex, an endogenous beta-2-microglobulin, or a combination thereof has been inhibited or eliminated; and/or
(ii) the immune cell is derived from peripheral blood mononuclear cells (PBMC), hematopoietic stem cells (HSCs), or inducible pluripotent stem cells (iPSCs).

11. The immune cell of any one of claims 1-10, wherein the immune cell comprises a nucleic acid or nucleic acid set, which collectively comprises:

(A) a first nucleotide sequence encoding the glucose importation polypeptide; and optionally
(B) a second nucleotide sequence encoding the chimeric receptor polypeptide, preferably wherein the nucleic acid or the nucleic acid set is an RNA molecule or a set of RNA molecules.

12. The immune cell of claim 11, wherein the immune cell comprises the nucleic acid, which comprises both the first nucleotide sequence and the second nucleotide sequence; optionally wherein the nucleic acid further comprises a third nucleotide sequence located between the first nucleotide sequence and the second nucleotide sequence, wherein the third nucleotide sequence encodes a ribosomal skipping site, an internal ribosome entry site (IRES), or a second promoter, preferably wherein the third nucleotide sequence encodes a ribosomal skipping site, which is a P2A peptide.

13. The immune cell of any one of claims 11 or 12, wherein the nucleic acid or the nucleic acid set is comprised within a vector or a set of vectors, preferably wherein the vector or set of vectors is an expression vector or a set of expression vectors, more preferably wherein the vector or set of vectors comprises one or more viral vectors, most preferably wherein the one or more viral vectors is a lentiviral vector or retroviral vector.

14. A pharmaceutical composition, comprising an immune cell of any one of claims 1-13, and a pharmaceutically acceptable carrier.

15. A population of immune cells set forth in any one of claims 1-13 for use in inhibiting cells expressing a target antigen in a subject.

16. The population of immune cells for use of claim 15, wherein at least some of the immune cells expressing the target antigen are located in a low-glucose environment.

17. The population of immune cells for use of any one of claims 15 or 16, wherein the immune cells meet one or more of the following:

(i) the immune cells are autologous;
(ii) the immune cells are allogeneic;
(iii) the immune cells are activated, expanded, or both *ex vivo,*

optionally, wherein the immune cells comprise T cells, which are activated in the presence of one or more of anti-CD3 antibody, anti-CD28 antibody, IL-2, phytohemoagglutinin, and an engineered artificial stimulatory cell or particle; or wherein the immune cells comprise natural killer cells, which are activated in the presence of one or more of 4-1BB ligand, anti-4-1BB antibody, IL-15, anti-IL-15 receptor antibody, IL-2, IL-12, IL-21 and K562 cells, and an engineered artificial stimulatory cell or particle.

18. The population of immune cells for use of any one of claims 15-17, wherein the subject is a human patient suffering from a cancer and the target antigen is a tumor antigen; preferably wherein the cancer is selected from the group consisting of carcinoma, lymphoma, sarcoma, blastoma, and leukemia, optionally wherein

(i) the cancer is selected from the group consisting of a cancer of B-cell origin, breast cancer, gastric cancer, neuroblastoma, osteosarcoma, lung cancer, skin cancer, prostate cancer, colon cancer, renal cell carcinoma, ovarian cancer, rhabdomyosarcoma, leukemia, mesothelioma, pancreatic cancer, head and neck cancer, retinoblastoma, glioma, glioblastoma, liver cancer, and thyroid cancer; or
(ii) the cancer of B-cell origin is selected from the group consisting of B-lineage acute lymphoblastic leukemia, B-cell chronic lymphocytic leukemia, and B-cell non-Hodgkin's lymphoma.

19. A nucleic acid or nucleic acid set, which collectively comprises:

(A) a first nucleotide sequence encoding a chimeric receptor polypeptide set forth in any one of claims 1-13; and
(B) a second nucleotide sequence encoding a glucose importation polypeptide set forth in claim 1,

preferably wherein the nucleic acid or the nucleic acid set is an RNA molecule or a set of RNA molecules, optionally wherein the nucleic acid comprises both the first nucleotide sequence and the second nucleotide sequence, and wherein the nucleic acid further comprises a third nucleotide sequence located between the first nucleotide sequence and the second nucleotide sequence, the third nucleotide sequence encoding a ribosomal skipping site, an internal ribosome entry site (IRES), or a second promoter, preferably wherein the ribosomal skipping site is a P2A peptide.

20. The nucleic acid or nucleic acid set of claim 19, wherein the nucleic acid or the nucleic acid set is comprised within a vector or a set of vectors, preferably wherein the vector or set of vectors is an expression vector or a set of expression vectors, more preferably wherein the vector or set of vectors comprises one or more viral vectors, most preferably wherein the one or more viral vectors is a retroviral vector, such as a lentiviral vector or a gamma retroviral vector.

**Patentansprüche**

1. Gentechnisch veränderte Immunzelle, wobei die Immunzelle eine verbesserte Glukoseaufnahme im Verhältnis zu einem Wildtyp der Immunzelle desselben Typs aufweist, wobei die Immunzelle gentechnisch so verändert ist, dass sie Folgendes exprimiert oder überexprimiert:

(i) ein Glukoseimportpolypeptid und
(ii) ein chimäres Rezeptorpolypeptid; wobei das chimäre Rezeptorpolypeptid Folgendes umfasst:

(a) eine extrazelluläre Zielbindungsdomäne;
(b) eine Transmembrandomäne; und
(c) eine zytoplasmatische Signaldomäne;

wobei sich die Nukleinsäure, die für das Glukoseimportpolypeptid kodiert, außerhalb von Chromosomen liegt oder in ein Chromosom der Immunzelle an einer Stelle aufgenommen ist, die sich vom Ursprungsort des endogenen Gens unterscheidet,
wobei das Glukoseimportpolypeptid ein Glukosetransporter (GLUT) oder ein Natrium-Glukose-Cotransporter (SGLT) ist, wobei vorzugsweise das Glukoseimportpolypeptid aus der Gruppe ausgewählt ist, bestehend aus: GLUT1, GLUT3, GLUT1 S226D, SGLT1, SGLT2, GLUT8, GLUT8 L12AL13A, GLUT11, GLUT7 und GLUT4.

2. Immunzelle nach Anspruch 1, wobei das chimäre Rezeptorpolypeptid ein antikörpergekoppeltes T-Zell-Rezeptor-Polypeptid (ACTR-Polypeptid) ist, bei dem (a) eine extrazelluläre Fc-bindende Domäne ist, wobei optional die Fc-bindende Domäne eine extrazelluläre ligandenbindende Domäne eines Fc-Rezeptors ist; wobei vorzugsweise die Fc-bindende Domäne eine extrazelluläre ligandenbindende Domäne von CD16A, CD32A oder CD64A ist; und wobei bevorzugter die Fc-bindende Domäne eine extrazelluläre ligandenbindende Domäne von F158 CD16A oder VI58 CD16A ist.

3. Immunzelle nach Anspruch 1, wobei das chimäre Rezeptorpolypeptid ein chimäres Antigenrezeptorpolypeptid (CAR-Polypeptid) ist, bei dem (a) eine extrazelluläre antigenbindende Domäne ist; wobei die extrazelluläre antigenbindende Domäne von (a) vorzugsweise ein Fragment einer Antikörper-Einzelkette ist, das an ein Tumorantigen, ein pathogenes Antigen oder eine Immunzelle bindet, die für ein Autoantigen spezifisch ist.

4. Immunzelle nach Anspruch 3, wobei

(i) das Tumorantigen mit einem hämatologischen Tumor assoziiert ist, wobei das Tumorantigen vorzugsweise aus der Gruppe ausgewählt ist, bestehend aus CD19, CD20, CD22, Kappa-Kette, CD30, CD123, CD33, LeY, CD138, CD5, BCMA, CD7, CD40 und IL-1RAP;

(ii) das Tumorantigen mit einem soliden Tumor assoziiert ist, wobei das Tumorantigen vorzugsweise aus der Gruppe ausgewählt ist, bestehend aus GD2, GPC3, FOLR, HER2, EphA2, EFGRVIII, IL13RA2, YEGFR2, ROR1, NKG2D, EpCAM, CEA, Mesothelin, MUC1, CLDN18.2, CD171, CD133, PSCA, cMET, EGFR, PSMA, FAP, CD70, MUC16, L1CAM und CAIX; oder

(iii) das pathogene Antigen ein Bakterienantigen, ein Virusantigen oder ein Pilzantigen ist.

5. Immunzelle nach einem der Ansprüche 1-4, wobei das chimäre Rezeptorpolypeptid eine oder mehrere der folgenden Eigenschaften aufweist:

(i) das chimäre Rezeptorpolypeptid umfasst ferner mindestens eine kostimulatorische Signaldomäne;

(ii) das chimäre Rezeptorpolypeptid umfasst keine kostimulatorischen Signaldomänen;

(iii) die zytoplasmatische Signaldomäne umfasst ein Immunrezeptor-Tyrosin-basiertes Aktivierungsmotiv (ITAM);

(iv) die zytoplasmatische Signaldomäne (c) befindet sich am C-Terminus des chimären Rezeptorpolypeptids;

(v) das chimäre Rezeptorpolypeptid umfasst ferner eine Gelenkdomäne, die sich am C-Terminus von (a) und am N-Terminus von (b) befindet, oder das chimäre Rezeptorpolypeptid umfasst keine Gelenkdomäne; und

(vi) das chimäre Rezeptorpolypeptid umfasst ferner an seinem N-Terminus ein Signalpeptid.

6. Immunzelle nach einem der Ansprüche 1-5, wobei

(i) die Transmembrandomäne von (b) von einem Singlepass-Membranprotein stammt, bei dem es sich bevorzugt um ein Membranprotein handelt, das aus der Gruppe ausgewählt ist, bestehend aus CD8$\alpha$, CD8$\beta$, 4-1BB, CD28, CD34, CD4, Fc$\epsilon$R1$\gamma$, CD16A, OX40, CD3$\zeta$, CD3$\epsilon$, CD3$\gamma$, CD3$\delta$, TCR$\alpha$, CD32, CD64, VEGFR2, FAS und FGFR2B; oder die Transmembrandomäne von (b) ein nicht natürlich vorkommendes hydrophobes Proteinsegment ist; und/oder

(ii) die zytoplasmatische Signaldomäne von (c) eine zytoplasmatische Domäne von CD3$\zeta$ oder Fc$\epsilon$R1$\gamma$ ist.

7. Immunzelle nach einem der Ansprüche 5 oder 6, wobei die mindestens eine kostimulatorische Signaldomäne von einem kostimulatorischen Molekül stammt, das aus der Gruppe ausgewählt ist, bestehend aus 4-1BB, CD28, Variante CD28$_{LL}$→$_{GG}$, OX40, ICOS, CD27, GITR, HVEM, TIM1, LFA1 und CD2, wobei bevorzugt die mindestens eine kostimulatorische Signaldomäne eine kostimulatorische Signaldomäne von CD28 oder eine kostimulatorische Signaldomäne von 4-1BB ist.

8. Immunzelle nach einem der Ansprüche 5-7, wobei die Gelenkdomäne eine Länge von 1 bis 60 Aminosäuren aufweist, wobei die Gelenkdomäne bevorzugt von CD28, CD16A, CD8$\alpha$ oder IgG stammt; oder wobei die Gelenkdomäne ein nicht natürlich vorkommendes Peptid ist, wobei die Gelenkdomäne vorzugsweise ein langkettiges rekombinantes Polypeptid (XTEN) oder ein (Gly$_4$Ser)$_n$-Polypeptid ist, bei dem n eine ganze Zahl von 3-12, jeweils einschließlich, ist.

9. Immunzelle nach einem der Ansprüche 3-8, wobei das chimäre Rezeptorpolypeptid ein CAR-Polypeptid ist, das Folgendes umfasst:

(i) eine kostimulatorische Domäne von CD28; in Verbindung mit einer Transmembrandomäne von CD28, einer Gelenkdomäne von CD28 oder einer Kombination daraus, oder

(ii) eine kostimulatorische Domäne von 4-1BB in Verbindung mit einer Transmembrandomäne von CD28, einer Gelenkdomäne von CD28 oder einer Kombination daraus, und/oder

(iii) wobei das CAR-Polypeptid die Aminosäuresequenz von SEQ-ID-Nr. 104 oder 105 umfasst.

10. Immunzelle nach einem der Ansprüche 1-9, wobei

(i) die Immunzelle eine natürliche Killerzelle, Makrophage, Neutrophile, Eosinophile oder T-Zelle ist, wobei vorzugsweise die Immunzelle eine T-Zelle ist, in der die Expression eines endogenen T-Zellrezeptors, eines endogenen Haupthistokompatibilitätskomplexes, eines endogenen Beta-2-Mikroglobulins oder eine Kombination davon gehemmt oder unterbunden wurde; und/oder

(ii) die Immunzelle von peripheren mononukleären Blutzellen (PBMC), hämatopoetischen Stammzellen (HSC) oder induzierbaren pluripotenten Stammzellen (iPSC) abgeleitet ist.

11. Immunzelle nach einem der Ansprüche 1-10, wobei die Immunzelle eine Nukleinsäure oder einen Nukleinsäuresatz umfasst, die bzw. der zusammenfassend Folgendes umfasst:

(A) eine erste Nukleotidsequenz, die für das Glukoseimportpolypeptid kodiert; und optional
(B) eine zweite Nukleotidsequenz, die für das chimäre Rezeptorpolypeptid kodiert, wobei vorzugsweise die Nukleinsäure oder der Nukleinsäuresatz ein RNA-Molekül oder ein Satz aus RNA-Molekülen ist.

12. Immunzelle nach Anspruch 11, wobei die Immunzelle die Nukleinsäure umfasst, die sowohl die erste Nukleotidsequenz als auch die zweite Nukleotidsequenz umfasst; wobei optional die Nukleinsäure ferner eine dritte Nukleotidsequenz umfasst, die zwischen der ersten Nukleotidsequenz und der zweiten Nukleotidsequenz liegt, wobei die dritte Nukleotidsequenz für eine Ribosomenüberlesestelle, eine interne Ribosomeneintrittsstelle (IRES) oder einen zweiten Promotor kodiert, wobei vorzugsweise die dritte Nukleotidsequenz für eine Ribosomenüberlesestelle kodiert, bei der es sich um ein P2A-Peptid handelt.

13. Immunzelle nach einem der Ansprüche 11 oder 12, wobei ein Vektor oder Vektorsatz die Nukleinsäure oder den Nukleinsäuresatz umfasst, wobei vorzugsweise der Vektor oder Vektorsatz ein Expressionsvektor oder ein Satz aus Expressionsvektoren ist, wobei bevorzugter der Vektor oder Vektorsatz einen oder mehrere virale Vektoren umfasst, wobei am bevorzugtesten der eine oder die mehreren viralen Vektoren ein lentiviraler Vektor oder retroviraler Vektor ist.

14. Pharmazeutische Zusammensetzung, die eine Immunzelle nach einem der Ansprüche 1 bis 13 und einen pharmazeutisch akzeptablen Träger umfasst.

15. Population von Immunzellen nach einem der Ansprüche 1-13 zur Verwendung beim Hemmen von Zellen, die ein Zielantigen in einem Patienten exprimieren.

16. Population von Immunzellen zur Verwendung nach Anspruch 15, wobei sich zumindest einige der Immunzellen, die das Zielantigen exprimieren, in einer Umgebung mit niedrigem Glukosespiegel befinden.

17. Population von Immunzellen zur Verwendung nach einem der Ansprüche 15-16, wobei die Immunzellen ein oder mehrere der folgenden Merkmale erfüllen:

(i) die Immunzellen sind autolog;
(ii) die Immunzellen sind allogen;
(iii) die Immunzellen werden *ex vivo* aktiviert, expandiert oder beides,

wobei die Immunzellen optional T-Zellen umfassen, die in Gegenwart von einem oder mehreren aus Anti-CD3-Antikörper, Anti-CD28-Antikörper, IL-2, Phytohämagglutinin und einer technisch hergestellten künstlichen stimulatorischen Zelle oder einem entsprechenden Partikel aktiviert sind, oder wobei die Immunzellen natürliche Killerzellen umfassen, die in Gegenwart von einem oder mehreren aus 4-1BB-Ligand, Anti-4-1BB-Antikörper, IL-15, Anti-IL-15-Rezeptorantikörper, IL-2, IL-12, IL-21 und K562-Zellen, und einer technisch hergestellten künstlichen stimulatorischen Zelle oder einem entsprechenden Partikel aktiviert sind.

18. Population von Immunzellen zur Verwendung nach einem der Ansprüche 15-17, wobei der Patient ein menschlicher Patient ist, der an einem Krebs erkrankt ist und das Zielantigen ein Tumorantigen ist; wobei der Krebs vorzugsweise aus der Gruppe ausgewählt ist, bestehend aus aus Karzinom, Lymphom, Sarkom, Blastom und Leukämie, wobei optional

(i) der Krebs aus der Gruppe ausgewählt ist, bestehend aus einem Krebs aus B-Zellen, Brustkrebs, Magenkrebs, Neuroblastom, Osteosarkom, Lungenkrebs, Hautkrebs, Prostatakrebs, Darmkrebs, Nierenzellkarzinom, Eierstockkrebs, Rhabdomyosarkom, Leukämie, Mesotheliom, Bauchspeicheldrüsenkrebs, Kopf-Hals-Karzinom, Retinoblastom, Gliom, Glioblastom, Leberkrebs und Schilddrüsenkrebs; oder
(ii) der Krebs aus B-Zellen aus der Gruppe ausgewählt ist, bestehend aus akuter lymphoblastischer Leukämie der B-Zellreihe, chronischer lymphatischer Leukämie aus B-Zellen und B-Zell-Non-Hodgkin-Lymphom.

19. Nukleinsäure oder Nukleinsäuresatz, die bzw. der zusammenfassend Folgendes umfasst:

(A) eine erste Nukleotidsequenz, die für ein chimäres Rezeptorpolypeptid nach einem der Ansprüche 1-13 kodiert, und

(B) eine zweite Nukleotidsequenz, die für ein Glukoseimportpolypeptid nach Anspruch 1 kodiert,

wobei vorzugsweise die Nukleinsäure oder der Nukleinsäuresatz ein RNA-Molekül oder ein Satz aus RNA-Molekülen ist, wobei optional die Nukleinsäure sowohl die erste Nukleotidsequenz als auch die zweite Nukleotidsequenz umfasst, und wobei die Nukleinsäure ferner eine dritte Nukleotidsequenz umfasst, die zwischen der ersten Nukleotidsequenz und der zweiten Nukleotidsequenz liegt, wobei die dritte Nukleotidsequenz für eine Ribosomenüberlesestelle, eine interne Ribosomeneintrittsstelle (IRES) oder einen zweiten Promotor kodiert, wobei vorzugsweise die Ribosomenüberlesestelle ein P2A-Peptid ist.

20. Nukleinsäure oder Nukleinsäuresatz nach Anspruch 19, wobei ein Vektor oder Vektorsatz die Nukleinsäure oder den Nukleinsäuresatz umfasst, wobei vorzugsweise der Vektor oder Vektorsatz ein Expressionsvektor oder ein Satz aus Expressionsvektoren ist, wobei bevorzugter der Vektor oder Vektorsatz einen oder mehrere virale Vektoren umfasst, wobei am bevorzugtesten der eine oder die mehreren viralen Vektoren ein retroviraler Vektor ist, beispielsweise ein lentiviraler Vektor oder ein gammaretroviraler Vektor.

## Revendications

1. Cellule immunitaire modifiée génétiquement, dans laquelle la cellule immunitaire a une activité de prélèvement de glucose améliorée par rapport à une cellule immunitaire sauvage du même type, la cellule immunitaire étant génétiquement modifiée pour exprimer ou exprimer excessivement :

(i) un polypeptide d'importation de glucose et
(ii) un polypeptide de récepteur chimérique, le polypeptide de récepteur chimérique comprenant :

(a) un domaine de liaison à une cible extracellulaire ;
(b) un domaine transmembranaire ; et
(c) un domaine de signalisation cytoplasmique ;

dans laquelle l'acide nucléique codant pour le polypeptide d'importation de glucose est situé de manière extrachromosomique ou est intégré dans un chromosome de la cellule immunitaire à un site qui est différent des loci natifs du gène endogène,
dans laquelle le polypeptide d'importation de glucose est un transporteur de glucose (GLUT) ou un cotransporteur de glucose-sodium (SGLT), de préférence le polypeptide d'importation de glucose étant choisi dans le groupe constitué par : GLUT1, GLUT3, GLUT1 S226D, SGLT1, SGLT2, GLUT8, GLUT8 L12A L13A, GLUT11, GLUT7 et GLUT4.

2. Cellule immunitaire selon la revendication 1, dans laquelle le polypeptide de récepteur chimérique est un polypeptide de récepteur de lymphocyte T couplé à un anticorps (ACTR), dans lequel (a) est un domaine de liaison de Fc extracellulaire, éventuellement
le domaine de liaison de Fc étant un domaine de liaison de ligand extracellulaire d'un récepteur de Fc ; de préférence, le domaine de liaison de Fc étant un domaine de liaison extracellulaire de CD16A, CD32A ou CD64A ; et davantage de préférence le domaine de liaison de Fc étant un domaine de liaison de ligand extracellulaire de F158 CD16A ou V158 CD16A.

3. Cellule immunitaire selon la revendication 1, dans laquelle le polypeptide de récepteur chimérique est un polypeptide de récepteur d'antigène chimérique (CAR), dans lequel (a) est un domaine de liaison à un antigène extracellulaire ; de préférence le domaine de liaison à un antigène extracellulaire (a) étant un fragment d'anticorps monocaténaire qui se lie à un antigène de tumeur, un antigène pathogène ou une cellule immunitaire spécifique d'un autoantigène.

4. Cellule immunitaire selon la revendication 3, dans laquelle

(i) l'antigène de tumeur est associé à une tumeur hématologique, de préférence l'antigène de tumeur étant choisi dans le groupe constitué par CD19, CD20, CD22, la chaîne kappa, CD30, CD123, CD33, LeY, CD138, CD5, BCMA, CD7, CD40 et IL-1RAP ;
(ii) l'antigène de tumeur est associé avec une tumeur solide, de préférence l'antigène de tumeur étant choisi dans le groupe constitué par GD2, GPC3, FOLR, HER2, EphA2, EFGRVIII, IL13RA2, VEGFR2, ROR1, NKG2D, EpCAM, CEA, la mésothéline, MUC1, CLDN18.2, CD171, CD133, PSCA, cMET, EGFR, PSMA, FAP, CD70, MUC16, L1-CAM et CAIX ; ou

(iii) l'antigène pathogène est un antigène bactérien, un antigène viral ou un antigène fongique.

5. Cellule immunitaire selon l'une quelconque des revendications 1 à 4, dans laquelle le polypeptide de récepteur chimérique comprend une ou plusieurs des caractéristiques suivantes :

(i) le polypeptide de récepteur chimérique comprend en outre au moins un domaine de signalisation co-stimulateur ;
(ii) le polypeptide de récepteur chimérique est sans domaines de signalisation co-stimulateurs ;
(iii) le domaine de signalisation cytoplasmique comprend un motif d'activation à base de tyrosine d'immunorécepteur (ITAM) ;
(iv) le domaine de polypeptide de récepteur chimérique est sans domaine charnière ; et
(vi) le polypeptide de récepteur chimérique comprend en outre un peptide signal à son extrémité N-terminale.

6. Cellule immunitaire selon l'une quelconque des revendications 1 à 5, dans laquelle

(i) le domaine transmembranaire de (b) est d'une protéine membranaire à simple passage, qui est de préférence d'une protéine membranaire choisie dans le groupe constitué par CD8$\alpha$, CD8$\beta$, 4-1BB, CD28, CD34, CD4, Fc$\epsilon$R1$\gamma$, CD16A, OX40, CD3$\zeta$, CD3$\epsilon$, CD3$\gamma$, CD3$\delta$, TCR$\alpha$, CD32, CD64, VEGFR2, FAS et FGFR2B ; ou le domaine transmembranaire de (b) est un segment de protéine hydrophobe non naturel ; et/ou
(ii) le domaine de signalisation cytoplasmique de (c ) est un domaine cytoplasmique de CD3$\zeta$ ou de Fc$\epsilon$R1$\gamma$.

7. Cellule immunitaire selon l'une quelconque des revendications 5 ou 6, dans laquelle l'au moins un domaine de signalisation co-stimulateur est d'une molécule co-stimulatrice choisie dans le groupe constitué par 4-1BB, CD28, le variant CD28$_{LL->GG}$, OX40, ICOS, CD27, GITR, HVEM, TIM1, LFA1 et CD2, de préférence l'au moins un domaine de signalisation co-stimulateur étant un domaine de signalisation co-stimulateur de CD28 ou un domaine de signalisation co-stimulateur de 4-1BB.

8. Cellule immunitaire selon l'une quelconque des revendications 5 à 7, dans laquelle le domaine charnière est de 1 à 60 acides aminés de long, de préférence le domaine charnière étant de CD28, CD16A, CD8$\alpha$ ou IgG ; ou le domaine charnière étant un peptide non naturel, de préférence le domaine charnière étant un polypeptide recombinant étendu (XTEN) ou un polypeptide (Gly$_4$Ser)$_n$, dans lequel n est un nombre entier de 3 à 12, inclusif.

9. Cellule immunitaire selon l'une quelconque des revendications 3 à 8, dans laquelle le polypeptide d'antigène chimérique est un polypeptide CAR, qui comprend

(i) un domaine co-stimulateur de CD28 ; en combinaison avec un domaine transmembranaire de CD28, un domaine charnière de CD28 ou une de leurs combinaisons ou
(ii) un domaine co-stimulateur de 4-1BB en combinaison avec un domaine transmembranaire de CD8, un domaine charnière de CD8 ou l'une de leurs combinaisons ; et/ou
(iii) le polypeptide CAR comprenant la séquence d'acides aminés des SEQ ID n° 104 ou 105.

10. Cellule immunitaire selon l'une quelconque des revendications 1 à 9, dans laquelle

(i) la cellule immunitaire est une cellule tueuse naturelle, un macrophage, un neutrophile, un éosinophile ou un lymphocyte T, la cellule immunitaire étant un lymphocyte T dans lequel l'expression d'un récepteur de lymphocyte T endogène, d'un complexe d'histocompatibilité majeur endogène, d'une microglobuline béta-2 endogène ou d'une de leurs combinaisons a été inhibée ou éliminée ; et/ou
(ii) la cellule immunitaire est dérivée des cellules mononucléaires du sang périphérique (PBMC), des cellules souches hématopoïétiques (HSC) ou de cellules souches pluripotentes inductibles (iPSC).

11. Cellule immunitaire selon l'une quelconque des revendications 1 à 10, dans laquelle la cellule immunitaire comprend un acide nucléique ou un ensemble d'acides nucléiques, qui comprend collectivement :

(A) une première séquence nucléotidique codant pour le polypeptide d'importation de glucose ; et éventuellement
(B) une seconde séquence nucléotidique codant pour le polypeptide de récepteur chimérique, de préférence l'acide nucléique ou l'ensemble d'acides nucléiques étant une molécule d'ARN ou un ensemble de molécules d'ARN.

12. Cellule immunitaire selon la revendication 11, dans laquelle la cellule immunitaire comprend l'acide nucléique, qui comprend les deux première séquence nucléotidique et seconde séquence nucléotidique ; éventuellement l'acide nucléique comprenant en outre une troisième séquence nucléotidique située entre la première séquence nucléotidique et la deuxième séquence nucléotidique, la troisième séquence nucléotidique codant pour un site de saut ribosomique, un site d'entrée de ribosome interne (IRES) ou un second promoteur, de préférence la troisième séquence nucléotidique codant pour un site de saut ribosomique, qui est un peptide P2A.

13. Cellule immunitaire selon l'une quelconque des revendications 11 ou 12, dans laquelle l'acide nucléique ou l'ensemble d'acides nucléiques est compris dans un vecteur ou un ensemble de vecteurs, le vecteur ou l'ensemble de vecteurs étant un vecteur d'expression ou un ensemble de vecteurs d'expression, davantage de préférence le vecteur ou l'ensemble de vecteurs comprenant un ou plusieurs vecteurs viraux, de préférence entre toutes les un ou plusieurs vecteurs viraux étant un vecteur lentiviral ou un vecteur rétroviral.

14. Composition pharmaceutique, comprenant une cellule immunitaire selon l'une quelconque des revendications 1 à 13 et un véhicule pharmaceutiquement acceptable.

15. Population de cellules immunitaires présentée dans l'une quelconque des revendications 1 à 13 destinée à être utilisée dans l'inhibition de cellules exprimant un antigène cible chez un sujet.

16. Population de cellules immunitaires destinée à être utilisée selon la revendication 15, dans laquelle au moins certaines des cellules immunitaires exprimant l'antigène cible sont situées dans un environnement à bas glucose.

17. Population de cellules immunitaires destinée à être utilisé selon l'une quelconque des revendications 15 ou 16, dans laquelle les cellules immunitaires répondent à un ou plusieurs de ce qui suit :

(i) les cellules immunitaires sont autologues ;
(ii) les cellules immunitaires sont allogènes ;
(iii) les cellules immunitaires sont activées, étendues ou les deux *ex vivo,*

dans laquelle, éventuellement, les cellules immunitaires comprennent des lymphocytes T, qui sont activées en présence d'un ou de plusieurs d'anticorps anti-CD3, d'anticorps anti-CD28, d'IL-2, de phytohémoagglutinine et d'une cellule ou particule stimulatrice artificielle modifiée ; ou dans laquelle les cellules immunitaires comprennent des cellules tueuses naturelles, qui sont activées en présence d'u ou de plusieurs d'un ligand de 4-1BB , d'un anticorps anti-4-1BB, d'IL-15, d'un anticorps de récepteur anti IL-15, d'IL-2, d'IL-12, d'IL-21 et de cellules K562 et d'une cellule ou particule stimulatrice artificielle modifiée.

18. Population de cellules immunitaires destinée à être utilisée selon l'une quelconque des revendications 15 à 17, dans laquelle le sujet est un patient humain souffrant d'un cancer et l'antigène cible est un antigène de tumeur ; de préférence dans laquelle le cancer est choisi dans le groupe constitué par un carcinome, un lymphome, un sarcome, un blastome et la leucémie, dans laquelle éventuellement

(i) le cancer est choisi dans le groupe constitué par un cancer d'origine de lymphocytes B, le cancer du sein, le cancer gastrique, le neuroblastome, l'ostéosarcome, le cancer du poumon, le cancer de la peau, le cancer de la prostate, le cancer du côlon, le carcinome de cellule rénale, le cancer des ovaires, le rhabdomyosarcome, la leucémie, le mésothéliome, le cancer pancréatique, le cancer de la tête et du cou, le rétinoblastome, le gliome, le glioblastome, le cancer du foie et le cancer de la thyroïde ; ou
(ii) le cancer d'origine de lymphocytes B est choisi dans le groupe constitué par la leucémie lymphoblastique aiguë de lignée B, la leucémie lymphocytaire chronique de lymphocytes B et le lymphome non de Hodgkin de lymphocytes B.

19. Acide nucléique ou ensemble d'acides nucléiques, qui comprend collectivement :

(A) une première séquence nucléotidique codant pour un polypeptide de récepteur chimérique présenté dans l'une quelconque des revendications 1 à 13 ; et
(B) une seconde séquence nucléotidique codant pour un polypeptide d'importation de glucose présenté dans la revendication 1,

de préférence l'acide nucléique ou l'ensemble d'acides nucléiques étant une molécule d'ARN ou un ensemble de molécules d'ARN, éventuellement l'acide nucléique comprenant les deux première séquence nucléotidique et seconde séquence nucléotidique et l'acide nucléique comprenant en outre une troisième séquence nucléotidique située entre la première séquence nucléotidique et la deuxième séquence nucléotidique, la troisième séquence nucléotidique codant pour un site de saut ribosomique, un site d'entrée de ribosome interne (IRES) ou un second promoteur, de préférence le site de saut ribosomique étant un peptide P2A.

20. Acide nucléique ou ensemble d'acides nucléiques selon la revendication 19, dans lequel l'acide nucléique ou l'ensemble d'acides nucléiques étant compris dans un vecteur ou un ensemble de vecteurs, de préférence le vecteur ou l'ensemble de vecteurs étant un vecteur d'expression ou un ensemble de vecteurs d'expression, davantage de préférence le vecteur ou l'ensemble de vecteurs comprenant un ou plusieurs vecteurs viraux, de préférence entre tous les un ou plusieurs vecteurs viraux étant un vecteur rétroviral, tel qu'un vecteur lentiviral ou un vecteur rétroviral gamma.

**Figure 1A**

Glucose

Extracellular

Intracellular

Immune-cell-expressed
glucose transporter

**Figure 1B**

Immune-cell-expressed
glucose transporter

Non-immune-cell-expressed
glucose transporter

**Figure 1C**

glucose transporter

Increased activity
glucose transporter

## Figure 1D

■ glucose transporter
at cell surface

☐ intracellular glucose
transporter

Glucose transporter trafficking ⟹

## Figure 1E

■ glucose transporter
at cell surface

☐ intracellular glucose
transporter

▨ glucose transporter with altered trafficking

Glucose transporter trafficking ⟹

Altered glucose transporter trafficking ⟹

# Figure 2

# Figure 3

# Figure 4A

# Figure 4B

Figure 5

# Figure 6A

**CAR + GLUT1, 1.25 mM**

# Figure 6B

**CAR + GLUT1, 10 mM**

## Figure 7A

## Figure 7B

CAR + GLUT3, 1.25 mM glucose

CAR + GLUT3, 10 mM glucose

# Figure 8A

**CAR + GLUT1 S226D, 1.25 mM**

# Figure 8B

**CAR + GLUT1 S226D, 10 mM**

EP 3 817 763 B1

EP 3 817 763 B1

**Figure 9**

# Figure 10

**Figure 11**

**Figure 12**

**Figure 13**

EP 3 817 763 B1

# Figure 14

## Figure 15

# Figure 16

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2015058018 A1
- US 2018015999 W
- US 7052906 B1
- WO 2000032776 A2
- US 8673860 B
- WO 2016040441 A1
- WO 2017161333 A
- US 20140106449 A
- WO 9007936 A
- WO 9403622 A
- WO 9325698 A
- WO 9325234 A
- WO 9311230 A
- WO 9310218 A
- WO 9102805 A
- US 5219740 A
- US 4777127 A
- GB 2200651 A
- EP 0345242 A

- WO 9412649 A
- WO 9303769 A
- WO 9319191 A
- WO 9428938 A
- WO 9511984 A
- WO 9500655 A
- US 5399346 A, Anderson
- US 4650764 A, Temin
- US 4980289 A, Temin
- US 5124263 A, Temin
- WO 9507358 A
- WO 1991002805 A2
- WO 1998009271 A1
- US 6194191 B
- WO 2013040557 A
- WO 9958572 A
- US 7435596 B
- US 8026097 B

### Non-patent literature cited in the description

- **ESHHAR et al.** *Proc. Natl. Acad. Sci. U. S. A.*, 1993, vol. 90 (2), 720-724
- **GEIGER et al.** *J Immunol.*, 1999, vol. 162 (10), 5931-5939
- **BRENTJENS et al.** *Nat. Med.*, 2003, vol. 9 (3), 279-286
- **COOPER et al.** *Blood.*, 2003, vol. 101 (4), 1637-1644
- **IMAI et al.** *Leukemia*, 2004, vol. 18, 676-684
- **PULE et al.** *Nat. Med.*, 2008, vol. 14 (11), 1264-1270
- **PORTER et al.** *N Engl J Med*, 2011, vol. 25 (365), 725-733
- **BRENTJENS et al.** *Blood*, 2011, vol. 118 (18), 4817-4828
- **TILL et al.** *Blood*, 2012, vol. 119 (17), 3940-3950
- **KOCHENDERFER et al.** *Blood*, 2012, vol. 119 (12), 2709-2720
- **BRENTJENS et al.** *Sci Transl Med*, 2013, vol. 5 (177), 177ra138
- **IRVING et al.** report engineering Chimeric Antigen Receptor T-cells for solid tumors. *Frontiers in Immunology*, 2017, vol. 8 (3)

- **KAWALEKAR et al.** show that the choice of signaling domain can metabolically reprogram T cells to alter mitochondrial biogenesis and persistence. *Immunity*, 2016, vol. 44 (2), 380-390
- **PALMER et al.** report that glucose metabolism regulates T cell activation, differentiation and functions. *Frontiers in Immunology*, 2015, vol. 6 (22)
- **CRETENET et al.** report that cell surface Glut1 levels distinguish human CD4 and CD8 T lymphocyte subsets with distinct effector functions. *Scientific Reports*, 2016, vol. 6 (1)
- **KISHTON et al.** report metabolic regulation of T cell longevity and function in tumor immunotherapy. *Cell Metabolism*, 2017, vol. 26 (1), 94-109
- **KUDO et al.** *Cancer Research*, 2014, vol. 74, 93-103
- **KARLIN ; ALTSCHUL.** *Proc. Natl. Acad. Sci. USA*, 1990, vol. 87, 2264-68
- **KARLIN ; ALTSCHUL.** *Proc. Natl. Acad. Sci. USA*, 1993, vol. 90, 5873-77
- **ALTSCHUL et al.** *J. Mol. Biol.*, 1990, vol. 215, 403-10
- **ALTSCHUL et al.** *Nucleic Acids Res.*, 1997, vol. 25 (17), 3389-3402

- Molecular Cloning: A Laboratory Manual. Cold Spring Harbor Laboratory Press, Cold Spring Harbor, 1989
- Current Protocols in Molecular Biology. John Wiley & Sons, Inc.
- **KIM et al.** *J. Mol. Evol.*, 2001, vol. 53, 1-9
- **YING et al.** *Nature Medicine*, 2019, vol. 25 (6), 947-953
- **CAROLINE J**. Suicide Gene Therapy: Methods and Reviews. Springer
- Cancer Research UK Centre for Cancer Therapeutics at the Institute of Cancer Research. Humana Press, 2004
- **MANN et al.** *Cell*, 1983, vol. 33, 153
- **MARKOWITZ et al.** *J. Virol.*, 1988, vol. 62, 1120
- **DOUGHERTY** ; **KUO et al.** *Blood*, 1993, vol. 82, 845
- **RABINOVICH et al.** *Human Gene Therapy*, vol. 17, 1027-1035
- **KIM et al.** *PLoS One*, 2011, vol. 6 (4), e18556
- **NEAL et al.** *J. Immunol. Res. Ther.*, 2017, vol. 2 (1), 68-79
- **TURTLE et al.** *Cancer J.*, 2010, vol. 16 (4), 374-381
- **REMINGTON**. The Science and Practice of Pharmacy. Lippincott Williams and Wilkins, 2000
- **LOZZIO et al.** *Blood*, 1975, vol. 45 (3), 321-334
- **KLEIN et al.** *Int. J. Cancer*, 1976, vol. 18, 421-431
- **FEHNIGER et al.** *Int Rev Immunol*, 2001, vol. 20 (3-4), 503-534
- **HARADA H et al.** *Exp Hematol*, 2004, vol. 32 (7), 614-621
- **HARADA et al.** *Jpn. J. Cancer Res*, 2002, vol. 93, 313-319
- Physician's Desk Reference. Thomson P D R, 2005
- Remington's The Science and Practice of Pharmacy. Lippincott Williams and Wilkins, 2000
- Harrison's Principles of Internal Medicine. McGraw Hill, 2001
- The Merck Manual of Diagnosis and Therapy. Merck Research Laboratories, 1992
- **SAMBROOK et al.** Molecular Cloning: A Laboratory Manual. Cold Spring Harbor Press, 1989
- Oligonucleotide Synthesis. Methods in Molecular Biology. Humana Press, 1984
- Cell Biology: A Laboratory Notebook. Academic Press, 1989
- Introuction to Cell and Tissue Culture. **J. P. MATHER** ; **P. E. ROBERTS**. Animal Cell Culture. Plenum Press, 1987
- Cell and Tissue Culture: Laboratory Procedures. J. Wiley and Sons, August 1993
- Handbook of Experimental Immunology. Methods in Enzymology. Academic Press, Inc
- Gene Transfer Vectors for Mammalian Cells. 1987
- Current Protocols in Molecular Biology. 1987
- PCR: The Polymerase Chain Reaction. 1994
- Current Protocols in Immunology. 1991
- Short Protocols in Molecular Biology. Wiley and Sons, 1999
- **C. A. JANEWAY** ; **P. TRAVERS**. *Immunobiology*, 1997
- **P. FINCH**. *Antibodies*, 1997
- Antibodies: a practice approach. IRL Press, 1988
- Monoclonal antibodies: a practical approach. Oxford University Press, 2000
- **E. HARLOW** ; **D. LANE**. Using antibodies: a laboratory manual. Cold Spring Harbor Laboratory Press, 1999
- The Antibodies. Harwood Academic Publishers, 1995
- DNA Cloning: A practical Approach. 1985, vol. I and II
- Nucleic Acid Hybridization. 1985
- Transcription and Translation. 1984
- Animal Cell Culture. 1986
- Immobilized Cells and Enzymes. IRL Press, 1986
- **B. PERBAL et al.** A practical Guide To Molecular Cloning. 1984
- **WIIG**. *Am J Physiol Heart Circ Physiol*, 2003, vol. 284, H416